# EUROPEAN PATENT APPLICATION

(11) **EP 4 512 806 A1**
(43) Date of publication of application: **26.02.2025**
(21) Application number: 23306421.1
(22) Date of filing: 25.08.2023
(51) Int. Cl.: C07D 487/04, A61K 31/53, A61P 29/00, A61P 35/00

(54) **MOLECULAR GLUE DEGRADERS AND USES THEREOF**

(71) Applicant: Mablink Bioscience, 69009 Lyon (FR); UNIVERSITE CLAUDE BERNARD - LYON 1, 69100 Villeurbanne (FR); Hospices Civils de Lyon, 69002 Lyon (FR); Ecole Sup Chimie Phys Electroniq Lyon (CPE Lyon), 69100 Villeurbanne (FR); Centre Léon Bérard, 69008 Lyon (FR); Centre National de la Recherche Scientifique, 75016 Paris (FR); Institut National de la Santé et de la Recherche Médicale, 75013 Paris (FR); Institut National des Sciences Appliquees de Lyon (Insa Lyon), 69100 Villeurbanne (FR)
(72) Inventor: JOSEPH, Benoît, 69100 Villeurbanne (FR); FOURNET, Guy, 69006 Lyon (FR); DUMONTET, Charles, 69200 Vénissieux (FR); VIRICEL, Warren, 69280 Sainte Consorce (FR); CONILH, Louise, 69006 Lyon (FR)
(74) Representative: Plasseraud IP

(57) **Abstract**

The present disclosure relates to pyrazolo[1,5-a][1,3,5]triazine derivatives and uses thereof. In particular the present disclosure relates to compounds of formula (I) which are 2-substituted 8-halogeno-N4-(4-(pyridin-2-yl) benzyl) pyrazolo[1,5-a][1,3,5] triazine-2,4-diamine, pharmaceutical compositions comprising these compounds, the preparation of these compounds and uses thereof, in particular as a molecular glue degrader.

## Description

### Technical field

The present disclosure relates to pyrazolo[1,5-a][1,3,5]triazine derivatives and uses thereof. In particular the present disclosure relates to compounds of formula (I) which are 2-substituted 8-halogeno-N4-(4-(pyridin-2-yl) benzyl) pyrazolo[1,5-a][1,3,5] triazine-2,4-diamine, pharmaceutical compositions comprising these compounds, the preparation of these compounds and uses thereof, in particular as a molecular glue degrader.

### Background

Protein-protein interactions are of great interest in the therapeutic field. Small-molecules drugs that can modulate protein-protein interactions have been developed in the last years and have shown interesting pharmaceutical properties. Among these small-molecule drugs, molecular glue degraders are a class of small-molecules that promotes proximity-induced target protein degradation (TPD) in which protein of interest are linked to natural digestive enzymes (typically E3 ubiquitin ligases of the ubiquitin-proteasome system) and are destroyed. Molecular glue degraders are advantageous because they act via transient binding rather than competitive occupancy and dissociate after promoting polyubiquitination of the protein of interest. As a result, a single molecular glue degrader compound can therefore destroy many copies of a targeted protein, thus inactivating disease-relevant proteins. A recent review explaining this approach can be found in Lai and Crews, Nat Rev Drug Discov 2017, 16(2), 1474-1784.

Several molecular glue degraders based on different molecular scaffolds have been developed these last years *(*Chem. Soc. Rev. 2022, 51, 5498-5517). However, because of the growing interest in the field of protein-protein interaction, there is always a need to provide small-molecules that can act as a molecular glue degrader, that are potent enough to be used in therapy while having interesting physicochemical and pharmacological properties.

### Summary

The inventors surprisingly found that 2-substituted 8-halogeno-N4-(4-(pyridin-2-yl) benzyl) pyrazolo[1,5-a][1,3,5] triazine-2,4-diamine derivatives shows high potency and valuable pharmaceutical properties, and are therefore indicated in therapy. In particular, they can act as a molecular glue degrader that can degrade cyclin-dependent kinases, particularly cyclin K. They also show improved low nM IC₅₀ cytotoxic potencies on different cancer cell lines, and distinct physicochemical (hydrophobicity, passive permeability) and pharmacological (efflux pump substrate susceptibility, rodent toxicity profile) properties.

Consequently, in a first aspect, the present disclosure relates to a compound formula (I): 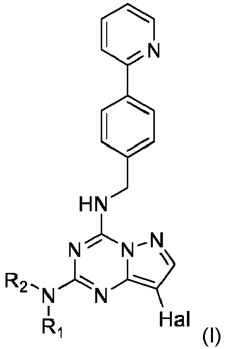 wherein
Hal is a halogen atom, preferably Br;
R1 is selected from the group consisting of H and C₁-C₆ alkyl;
R2 is selected from the group consisting of C₁-C₁₂ alkyl, C₁-C₁₂ heteroalkyl, aryl having 6 to 10 ring atoms, C₃-C₈ cycloalkyl, heterocycloalkyl having 3 to 10 ring atoms, heteroaryl having 5 to 10 ring atoms, and any combination thereof, said alkyl, heteroalkyl, aryl, cycloalkyl, heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl;
or, R1 and R2, together with the N atom to which they are bonded, form a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl; and pharmaceutically acceptable salts and solvates thereof.

In a second aspect, the disclosure relates to a pharmaceutical composition comprising a compound of the present disclosure and a pharmaceutically acceptable carrier.

In a third aspect, the disclosure relates to a compound of the present disclosure for use as a drug.

In a fourth aspect, the disclosure relates to a compound of the present disclosure for use in treating cancer.

### Legends of the Figures

Figures 1A and 1B represent the in vitro cytotoxicity assays of the halogenated compound 2384 and its isopropyl counterpart 2420 according to example 3.
Figures 2A and 2B represent the in vitro cytotoxicity assays of the halogenated compound 2388 and its isopropyl counterpart 2428 according to example 3.
Figure 3 represents the in vitro cytotoxicity assays of compound 2384 of the disclosure *versus* the known molecular glue degrader compound CR8 according to example 3.
Figure 4 represents the result of the kinase screening assay of compound 2384 according to example 5. Kinases inhibited at more than 90% are presented in the map as circles whose size is representative of percentage of inhibition.
Figure 5 represents the dose-response inhibition on top 25 selected kinases of compound 2384 according to example 5.
Figure 6 represents the in vitro cytotoxicity assays of compound 2384 of the disclosure with or without pevonedistat, a known inhibitor of the NEDDylation pathway that drives degradation of the CycK protein, according to example 6.
Figure 7 shows CycK quantification experiments in cancer cells treated with compound 2384 of the disclosure and known molecular glue degrader CR8, according to example 7.

### Detailed description

### Definitions

As used herein, the terms "Cₓ-C_{y} alkyl", by itself or as part of another substituent, refer to a linear or branched alkyl functional group having x to y carbon atoms, preferably 1 to 12 carbon atoms, more preferably 1 to 6 carbon atoms. Suitable alkyl groups include methyl, ethyl, *n*-propyl, *i*-propyl, *n*-butyl, *i-*butyl, *s*-butyl and *t*-butyl, pentyl and its isomers (e.g. *n*-pentyl, *iso*-pentyl), and hexyl and its isomers (e.g. *n*-hexyl, *iso*-hexyl).

As used herein, the terms "C₃-C₁₂ cycloalkyl" refer to a saturated or unsaturated cyclic group having 3 to 12 carbon atoms, preferably 3 to 6. The cycloalkyl can have a single ring or multiple rings fused together. The cycloalkyl can also include spirocyclic rings. Suitable cycloalkyl groups include cyclopropyl, cyclobutyl, cyclopentyl and cyclohexyl.

As used herein, the term "halogen" refers to a fluoro (-F), chloro (-CI), bromo (-Br), or iodo (-I) group.

As used herein, the terms "C₁-Cₓ heteroalkyl", refer to a straight or branched hydrocarbon chain consisting of 1 to x carbon atoms, preferably 1 to 12 carbon atoms, more preferably 1 to 6 carbon atoms, and at least one heteroatom, preferably from one to three heteroatoms selected from the group consisting of O, N, Si and S, and wherein the nitrogen and sulfur atoms may optionally be oxidized (for example: a sulfoxide or a sulfone) and the nitrogen heteroatom may optionally be quaternized. The heteroatom(s) O, N and S may be placed at any interior position of the heteroalkyl group or at the position at which the alkyl group is attached to the remainder of the molecule.

As used herein, the terms "C₁-Cₓ alkylene", used alone or as part of another substituent, refer to a divalent saturated, straight-chained or branched hydrocarbon group having 1 to x carbon atom, preferably 1 to 12 carbon atoms, and more preferably 1 to 6.

As used herein, the terms "C₁-Cₓ heteroalkylene", refer to a divalent heteroalkyl as defined above. For heteroalkylene groups, heteroatoms can also occupy either or both of the chain termini.

As used herein, the terms "aryl having 6 to 10 ring atoms" refer to a polyunsaturated, aromatic hydrocarbyl group having a single ring or multiple aromatic rings fused together, containing 6 to 10 ring atoms, wherein at least one ring is aromatic. The aromatic ring may optionally include one to two additional rings (cycloalkyl, heterocyclyl or heteroaryl as defined herein) fused thereto. Suitable aryl groups include phenyl, naphtyl and phenyl ring fused to a heterocyclyl, like benzopyranyl, benzodioxolyl, benzodioxanyl and the like.

As used herein, the terms "heteroaryl having 5 to 10 ring atoms" refer to a polyunsaturated, aromatic ring system having a single ring or multiple aromatic rings fused together or linked covalently, containing 5 to 10 atoms, wherein at least one ring is aromatic and at least one ring atom is a heteroatom selected from N, O and S. The nitrogen and sulfur heteroatoms may optionally be oxidized and the nitrogen heteroatoms may optionally be quaternized. Such rings may be fused to an aryl, cycloalkyl or heterocyclyl ring. Non-limiting examples of such heteroaryl, include: furanyl, thiophenyl, pyrrolyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, indolyl, isoindolyl, benzofuranyl, isobenzofuranyl, benzothiophenyl, isobenzothiophenyl, indazolyl, benzimidazolyl, benzoxazolyl, purinyl, benzothiadiazolyl, quinolinyl, isoquinolinyl, cinnolinyl, quinazolinyl and quinoxalinyl.

As used herein, the terms "heterocyclyl having 3 to 10 ring atoms" refer to a saturated or unsaturated cyclic group having 3 to 10 ring atoms, preferably 5 to 10 ring atoms, wherein at least one ring atom is a heteroatom selected from N, O and S. The nitrogen and sulfur heteroatoms may optionally be oxidized and the nitrogen heteroatoms may optionally be quaternized. The heterocycle can include fused or bridged rings as well as spirocyclic rings. Examples of heterocycle include, but are not limited to, tetrahydropyridyl, piperidinyl, morpholinyl, tetrahydrofuranyl, tetrahydrothienyl, piperazinyl, 1-azepanyl, imidazolinyl, 1 ,4-dioxanyl and the like.

As used herein, the terms "organyl group" refer to any organic substituent group, regardless of functional type, having one free valence at a carbon atom.

As used herein, the terms "optionally substituted" can refer to groups that can be substituted with one or more of the substituents independently selected from: C₁₋C₂₀ alkyl, C₁₋C₂₀ heteroalkyl, aryl having 6 to 10 ring atoms, C₃-C₈ cycloalkyl, heterocycloalkyl having 3 to 10 ring atoms, heteroaryl having 5 to 10 ring atoms, -X, -R', -O⁻, -OR', =O, -SR', -S-, -NR'₂, -NR'₃, =NR', -CX₃, -CN, -OCN, -SCN, -NCS, -NO, -NO₂, =N₂, -NRC(=O)R', -C(=O)R', -C(=O)NR'₂, -SO₃⁻, -SOsH, -S(=O)₂R', -OS(=O)₂OR', - S(=O)₂NR', -S(=O)R', -C(=O)R', -C(=S)R', -CO₂R', -CO₂, -C(=S)OR', C(=O)SR', C(=S)SR', C(=O)NR'₂, C(=S)NR'₂, C(=NR')NR'₂, and any combination thereof, where each X is independently a halogen: -F, - CI, -Br, or -I; and each R' is independently -H, -C₁₋C₂₀ alkyl, -C₆-C₁₀ aryl, C₃-C₈ cycloalkyl, heterocycloalkyl having 3 to 10 ring atoms, or heteroaryl having 5 to 10 ring atoms.

In the present disclosure, the dotted bond represents the attachment point of a moiety to the rest of the molecule.

Various embodiments of the disclosure are described herein. It will be recognized that features specified in each embodiment may be combined with other specified features to provide further embodiments.

The present disclosure encompasses the compounds of the present disclosure, their tautomers, enantiomers, diastereomers, racemates or mixtures thereof, and their hydrates, esters, solvates or pharmaceutically acceptable salts.

The terms "pharmaceutically acceptable salts" refers to salts that retain the biological effectiveness and properties of the compounds of this disclosure and, which typically are not biologically or otherwise undesirable. In many cases, the compounds of the disclosure are capable of forming acid and/or base salts by virtue of the presence of amino and/or carboxyl groups or groups similar thereto. Pharmaceutically acceptable acid addition salts can be formed with organic acids and/or inorganic acids. Pharmaceutically acceptable base addition salts can be formed with organic bases and/or inorganic bases.

In many cases, the compounds of the disclosure are capable of forming esters by virtue of the presence of carboxyl groups. Esters include C₁-C₆ alkyl esters.

Any formula given herein is also intended to represent unlabeled as well as isotopically forms of the compounds, like deuterium labeled compounds or ¹⁴C-labeled compounds.

### Compounds of formula I

The present disclosure first relates to a compound of formula (I) 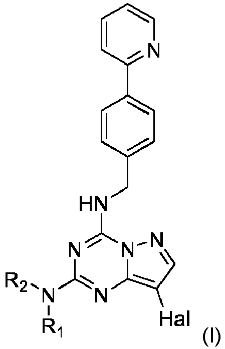 wherein
Hal is a halogen atom, preferably Br;
R1 is selected from the group consisting of H and C₁-C₆ alkyl, said alkyl being optionally substituted;
R2 is selected from the group consisting of C₁₋C₂₀ alkyl, C₁₋C₂₀ heteroalkyl, aryl having 6 to 10 ring atoms, C₃-C₈ cycloalkyl, heterocycloalkyl having 3 to 10 ring atoms, heteroaryl having 5 to 10 ring atoms, and any combination thereof, said alkyl, heteroalkyl, aryl, cycloalkyl, heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, and -NR'R"; R', and R" being independently selected from H and C₁-C₆ alkyl;
or, R1 and R2, together with the N atom to which they are bonded, form a heterocycloalkyl having 3 to 10 ring atoms, or a heteroaryl having 5 to 10 ring atoms, said heterocycloalkyl and heteroaryl being optionally substituted, preferably by one or more substituent selected from - OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), -(C₃-C₈ cycloalkyl)-(C₁-C₁₂ heteroalkyl), (heterocycloalkyl having 3 to 10 ring atoms)-(C₁-C₁₂ heteroalkyl), (heteroaryl having 5 to 10 ring atoms)-(C₁-C₁₂ heteroalkyl), (aryl having 6 to 10 ring atoms)-(C₁-C₁₂ heteroalkyl), and -NR'R"; R', and R" being independently selected from H and C₁-C₆ alkyl;
and pharmaceutically acceptable salts and solvates thereof.

According to an embodiment, Hal is a halogen atom, preferably Br;
R1 is selected from the group consisting of H and C₁-C₆ alkyl;
R2 is selected from the group consisting of C₁-C₁₂ alkyl, C₁-C₁₂ heteroalkyl, aryl having 6 to 10 ring atoms, C₃-C₈ cycloalkyl, heterocycloalkyl having 3 to 10 ring atoms, heteroaryl having 5 to 10 ring atoms, and any combination thereof, said alkyl, heteroalkyl, aryl, cycloalkyl, heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl;
or, R1 and R2, together with the N atom to which they are bonded, form a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl; and pharmaceutically acceptable salts and solvates thereof.

Hal is an halogen atom, like F, CI, Br or I. For example, Hal can be F or Br. Preferably Hal is Br.

According to an embodiment, R2 is selected from the group consisting of C₁-C₁₂ heteroalkyl, aryl having 6 to 10 ring atoms, C₃-C₈ cycloalkyl, heterocycloalkyl having 3 to 10 ring atoms, -(C₁-C₁₂ alkyl)-(C₃-C₈ cycloalkyl), -(C₁-C₁₂ alkyl)-(heterocycloalkyl having 3 to 10 ring atoms), said alkyl, heteroalkyl, aryl, cycloalkyl, and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl.

According to an embodiment, R2 comprises at least one nitrogen atom.

According to an embodiment, R1 and R2, together with the N atom to which they are bonded, form a heterocycloalkyl having 3 to 10 ring atoms and comprising at least 2 N atoms, said heterocycloalkyl being optionally substituted, by one or more substituent preferably selected from C₁-C₁₂ heteroalkyl, heterocyclyl having 5 to 10 ring atoms, and -NR'R"; R', and R" being independently selected from H and C₁-C₆ alkyl.

According to an embodiment, Hal is Br, R1 is H, and R2 is a C₃-C₈ cycloalkyl substituted by - NR'R"; R', and R" being independently selected from H and C₁-C₆ alkyl.

According to an embodiment, the compound is of formula (II): 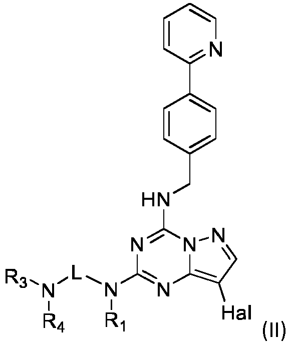 wherein
L is a linker selected from a bond, C₁₋C₂₀ alkylene, C₁₋C₂₀ heteroalkylene, arylene having 6 to 10 ring atoms, C₃-C₈ cycloalkylene, heterocycloalkylene having 3 to 10 ring atoms, heteroarylene having 5 to 10 ring atoms, and any combination thereof, said alkylene, heteroalkylene, arylene, cycloalkylene, heteroarylene and heterocycloalkylene being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl,
R3 is selected from the group consisting of H and C₁-C₆ alkyl, and
R4 is selected from the group consisting of H and C₁-C₆ alkyl.

According to an embodiment, the compound is of formula (III) 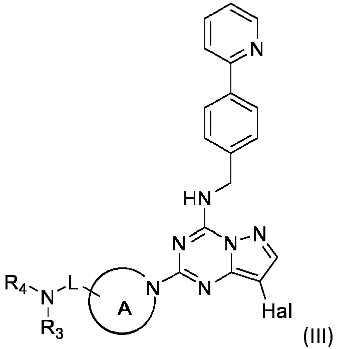 wherein
Ring A is a heterocycloalkyl having 3 to 10 ring atoms, or a heteroaryl having 5 to 10 ring atoms, said heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl;
L is a linker selected from a bond, C₁-C₁₂ alkylene, C₁-C₁₂ heteroalkylene, arylene having 6 to 10 ring atoms, C₃-C₈ cycloalkylene, heterocycloalkylene having 3 to 10 ring atoms, heteroarylene having 5 to 10 ring atoms, and any combination thereof, said alkylene, heteroalkylene, arylene, cycloalkylene, heteroarylene and heterocycloalkylene being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl;
R3 is selected from the group consisting of H and C₁-C₆ alkyl, and
R4 is selected from the group consisting of H and C₁-C₆ alkyl.

According to an embodiment, the compound is of formula (IV) 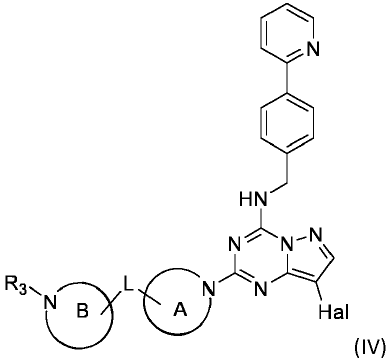 wherein
Ring A is a heterocycloalkyl having 3 to 10 ring atoms, or a heteroaryl having 5 to 10 ring atoms, said heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl
Ring B is a heterocycloalkyl having 3 to 10 ring atoms, or a heteroaryl having 5 to 10 ring atoms, said heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl
L is a linker selected from a bond, C₁-C₁₂ alkylene, C₁-C₁₂ heteroalkylene, arylene having 6 to 10 ring atoms, C₃-C₈ cycloalkylene, heterocycloalkylene having 3 to 10 ring atoms, heteroarylene having 5 to 10 ring atoms, and any combination thereof, said alkylene, heteroalkylene, arylene, cycloalkylene, heteroarylene and heterocycloalkylene being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl; and
R3 is selected from the group consisting of H and C₁-C₆ alkyl.

According to an embodiment, the compound is of formula (V) 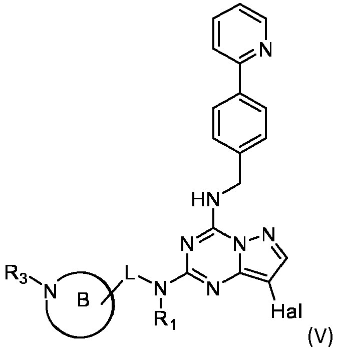 wherein
Ring B is a heterocycloalkyl having 3 to 10 ring atoms, or a heteroaryl having 5 to 10 ring atoms, said heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl;
L is a linker selected from a bond, C₁-C₁₂ alkylene, C₁-C₁₂ heteroalkylene, arylene having 6 to 10 ring atoms, C₃-C₈ cycloalkylene, heterocycloalkylene having 3 to 10 ring atoms, heteroarylene having 5 to 10 ring atoms, and any combination thereof, said alkylene, heteroalkylene, arylene, cycloalkylene, heteroarylene and heterocycloalkylene being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl; and
R3 is selected from the group consisting of H and C₁-C₆ alkyl.

According to an embodiment, R2 is selected from the group consisting of C₁-C₁₂ heteroalkyl, aryl having 6 to 10 ring atoms, C₃-C₈ cycloalkyl, heterocycloalkyl having 3 to 10 ring atoms, -(C₁-C₁₂ alkyl)-(C₃-C₈ cycloalkyl), -(C₁-C₁₂ alkyl)-(heterocycloalkyl having 3 to 10 ring atoms), said alkyl, heteroalkyl, aryl, cycloalkyl, and heterocycloalkyl being optionally substituted, preferably by one or more substituent selected from the group consisting of -OH, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl.

According to an embodiment, the compound of formula (I) is a compound selected from

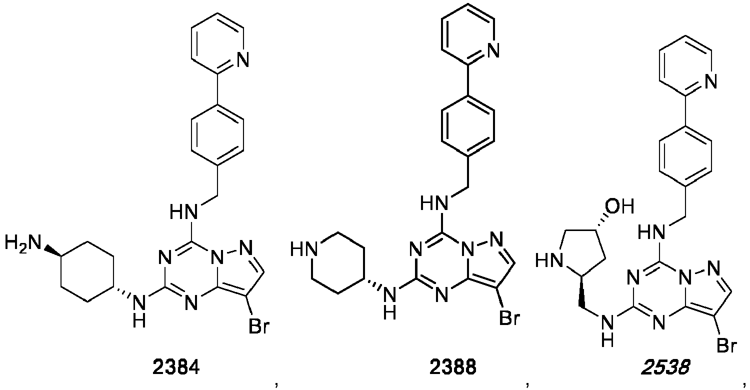

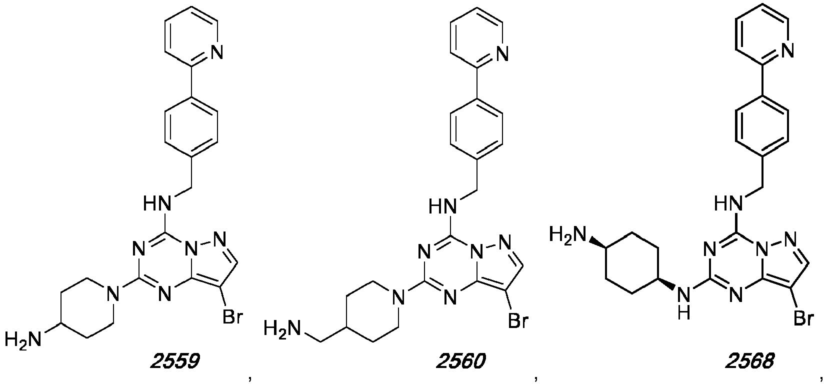

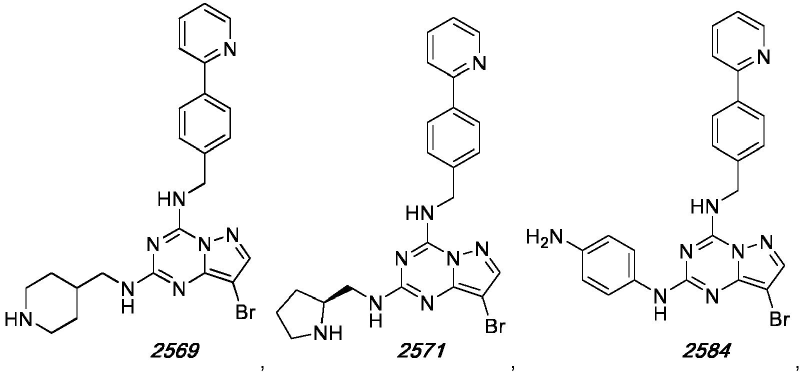

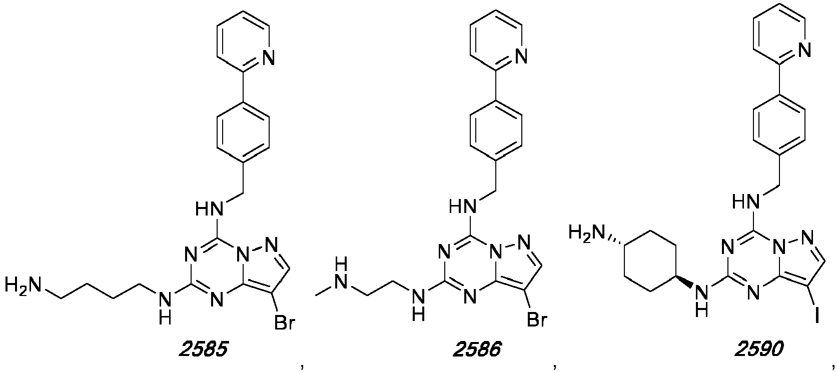

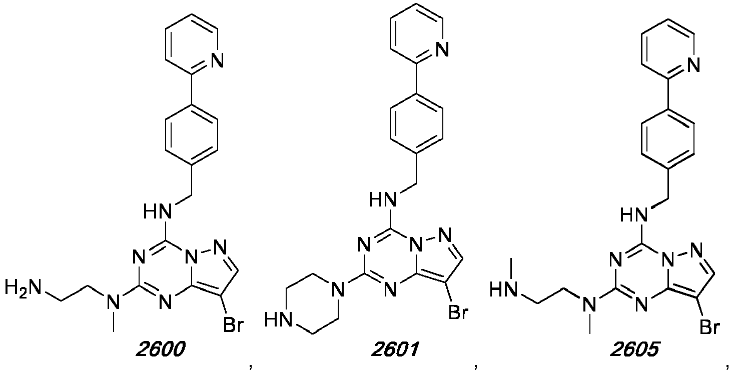

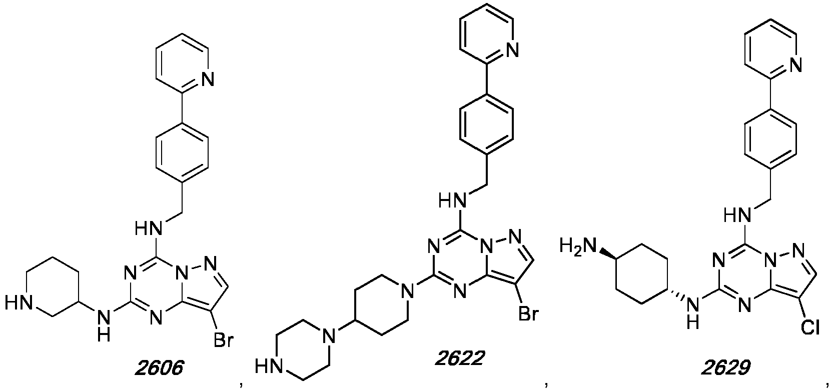

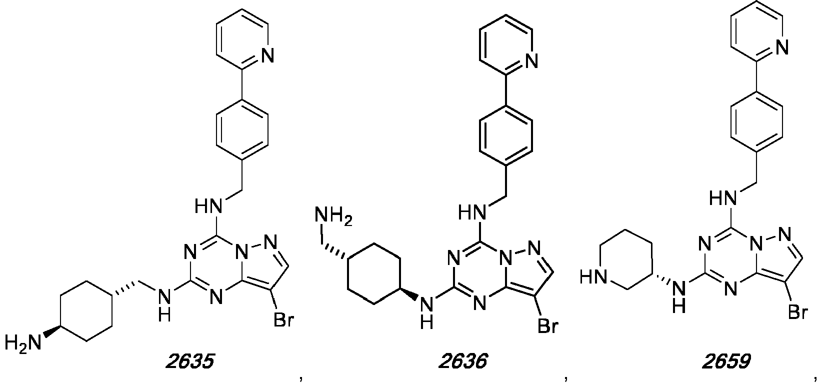

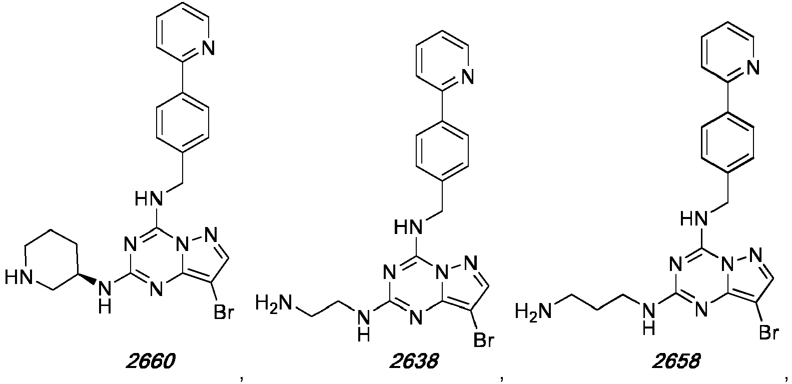

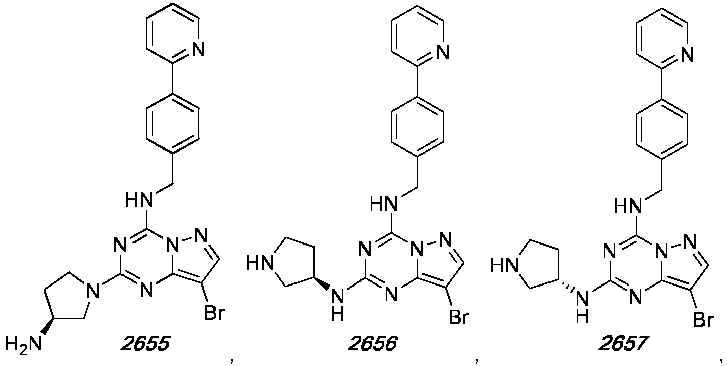

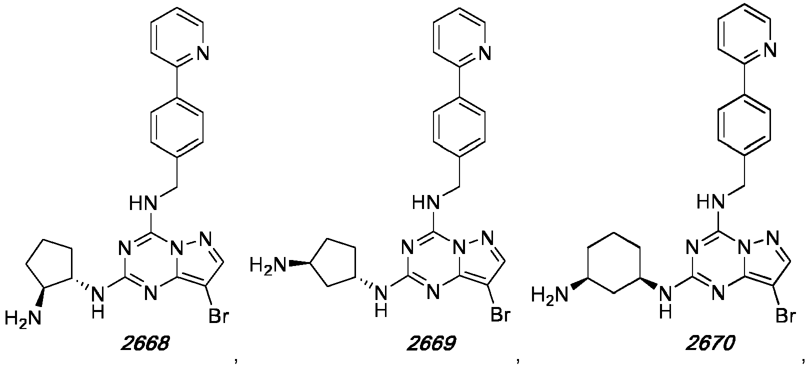

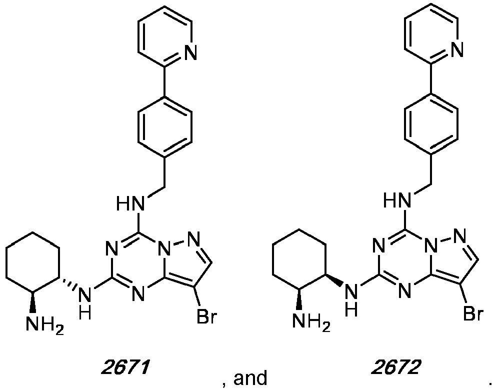

According to an embodiment, the compound of formula (I) is

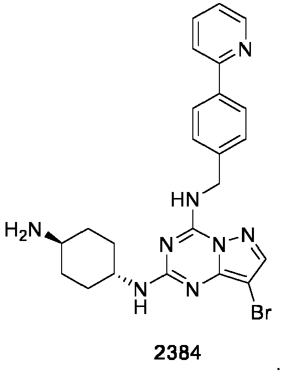

The present disclosure also relates to a method for synthesizing a compound of the disclosure.

The compounds of the disclosure can be prepared following procedures that are known in the art of organic synthesis (chemical reactions, extraction, evaporation, precipitation, chromatography, filtration, trituration, crystallization, and the like), and analytical procedures, which are known to persons of ordinary skill in the art of analytical chemistry. The details of such reactions and techniques can be found in a number of treatises, including Richard Larock, Comprehensive Organic Transformations, A Guide to Functional Group Preparations, 2nd Ed (2010), and the multi-volume serie edited by Michael B . Smith and others, Compendium of Organic Synthetic Methods (1974 et seq.). Starting materials and reagents may be obtained from commercial sources or may be prepared using literature methods.

The compound of formula (I) can be synthesized as follows:

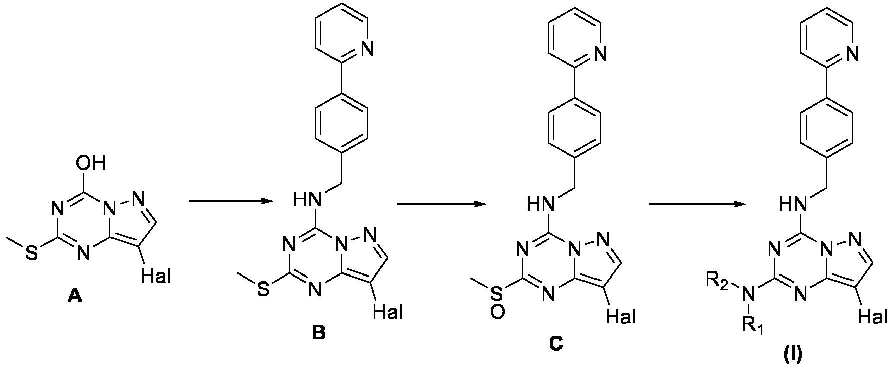

Compound **A** can be prepared as described in WO2018195397. Compound **A** can be converted to compound **B** using first phosphorous oxychloride and then 4-(Pyridin-2-yl)phenyl)methanamine. Compound **B** is then oxidized to the corresponding sulfone **C,** for example using meta-chloroperoxybenzoic acid. Finally, the compound of formula (I) can be prepared by nucleophilic aromatic substitution using the amine HNR₁R₂. This step can be done by heating the reaction mixture to a temperature comprised between 100 and 180°C, like at 140°C.

The same procedure can be used for compounds of formula (II), (III), (IV) and (V) using the corresponding amines.

### Pharmaceutical composition

The disclosure also relates to a pharmaceutical composition comprising a compound of the disclosure and at least one pharmaceutically acceptable carrier. In particular, the present disclosure relates to a pharmaceutical composition comprising a compound of formula (I) and at least one pharmaceutically acceptable carrier.

As used herein, "pharmaceutically acceptable carrier" includes any and all solvents, dispersion media, coatings, antibacterial and antifungal agents, isotonic and absorption delaying agents, and the like that are physiologically compatible.

The pharmaceutical compositions of the disclosure can be formulated for a topical, oral, intranasal, intraocular, intravenous, intramuscular or subcutaneous administration and the like.

For example, the pharmaceutical compositions can take the form of tablets, pills, capsules, semisolids, powders, sustained release formulations, solutions, suspensions, emulsions, syrups, elixirs, aerosols, or any other appropriate compositions; and comprise at least one compound according to this disclosure.

Because of their ease in administration, tablets and capsules represent the most advantageous oral dosage unit form, in which case solid pharmaceutical carriers are obviously employed. If desired, tablets may be sugar coated or enteric coated by standard techniques. The tablets or pills can be coated to provide a dosage form affording the advantage of prolonged action. For example, the tablet or pills can comprise an inner dosage and an outer dosage component, the latter being in the form of an envelope over the former. The two components can be separated by an enteric layer, which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The compounds of the disclosure may also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

According to an embodiment, the pharmaceutical compositions contain vehicles which are pharmaceutically acceptable for oral formulation.

In other specific embodiments, the carrier could be suitable for intravenous, intramuscular, subcutaneous, parenteral, spinal or epidermal administration (e.g., by injection or infusion). In one embodiment, the carrier should be suitable for subcutaneous route or intratumoral injection. Depending on the route of administration, the active compound may be coated in a material to protect the compound from the action of acids and other natural conditions that may inactivate the compound. Formulations for injection may further include one or more excipients, preservatives, solubilizers, buffering agents, etc.

The form of the pharmaceutical compositions, the route of administration, the dosage and the regimen naturally depend upon the condition to be treated, the severity of the illness, the age, weight, and sex of the patient, etc.

Preferably, the pharmaceutical compositions contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

To prepare pharmaceutical compositions, an effective amount of the compound according to the disclosure may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders or lyophilisates for the extemporaneous preparation of sterile injectable solutions or dispersions. In all cases, the form must be sterile and must be fluid to the extent that easy syringeability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

Compounds of the disclosure can be formulated into a composition in a neutral or salt form. Pharmaceutically acceptable salts include the acid addition salts (formed with free amino groups) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 mL of isotonic NaCl solution and either added to 1000 mL of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The doses used for the administration can be adapted as a function of various parameters, and in particular as a function of the mode of administration used, of the relevant pathology, or alternatively of the desired duration of treatment. It will be appreciated that appropriate dosages of the compounds, and compositions comprising the compounds, can vary from patient to patient. Determining the optimal dosage will generally involve the balancing of the level of therapeutic benefit against any risk or deleterious side effects of the treatments described herein. The selected dosage level will depend on a variety of factors including, but not limited to, the activity of the particular compound, the route of administration, the time of administration, the rate of excretion of the compound, the duration of the treatment, other drugs, compounds, and/or materials used in combination, and the age, sex, weight, condition, general health, and prior medical history of the patient. The amount of compound and route of administration will ultimately be at the discretion of the physician, although generally the dosage will be to achieve local concentrations at the site of action which achieve the desired effect without causing substantial harmful or deleterious side-effects. For example, the dose used for the administration can be of about 0.0001-5000 mg of the compound of the disclosure for a subject of about 50-70 kg.

### Method of use

The compounds of the disclosure exhibit valuable pharmaceutical properties as indicated in the in vitro tests and in vivo tests provided in the examples and are therefore indicated for therapy, in particular for treating cancer. The disclosure also relates to a compound of the disclosure for use as a drug. In particular, the disclosure relates to a compound of formula (I), (II), (III), (IV) or (V) for use as a drug.

In particular, the compounds of the disclosure, like compounds of formula (I), (II), (III), (IV) or (V), are useful in the prevention or treatment of cancer or an inflammatory disease.

The disclosure relates to a method for treating cancer or an inflammatory disease said method comprising administering to a subject in need thereof, preferably a human, a therapeutically efficient amount of
(i) a compound of the disclosure, or
(ii) a pharmaceutical composition comprising such compound as described herein.

As used herein, the term "treating" includes reversing, alleviating, inhibiting the progression of, preventing or reducing the likelihood of the disease, disorder, or condition to which such term applies, or one or more symptoms or manifestations of such disease, disorder or condition. Preventing refers to causing a disease, disorder, condition, or symptom or manifestation of such, or worsening of the severity of such, not to occur. Accordingly, the presently disclosed compounds can be administered prophylactically to prevent or reduce the incidence or recurrence of the disease, disorder, or condition.

As used herein, the terms "therapeutically efficient amount" of a compound refer to an amount of the compound that will elicit the biological or medical response of a subject, for example, ameliorate the symptoms, alleviate conditions, slow or delay disease progression, or prevent a disease.

The disclosure also relates to the use of a compound of the disclosure, preferably a compound of formula (I), for the manufacture of a medicament for the treatment of cancer.

The disclosure also relates to a compound of the disclosure for use in a method for treating cancer. As used herein, the term "cancer" has its general meaning in the art and includes an abnormal state or condition characterized by rapidly proliferating cell growth. The term is meant to include all types of cancerous growths or oncogenic processes, metastatic tissues or malignantly transformed cells, tissues or organs, irrespective of histopathologic type or stage of invasiveness. The term cancer includes malignancies of the various organ systems, such as affecting skin, lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the oesophages.

The disclosure also relates to a compound of the disclosure for use in a method for treating inflammatory disease. The inflammatory disease can be caused, induced, initiated and/or enhanced by bacteria, viruses, prions, parasites, fungi, and/or caused by irritative, traumatic, metabolic, allergic, autoimmune, or idiopathic agents.

The inflammatory disease can be selected from the group comprising or consisting of inflammatory diseases of the central nervous system (CNS), inflammatory rheumatic diseases, inflammatory diseases of blood vessels, inflammatory diseases of the middle ear, inflammatory bowel diseases, inflammatory diseases of the skin, inflammatory disease uveitis, and inflammatory diseases of the larynx.

### Examples

### Example 1: synthesis of 2-substituted 8-halogeno-N4-(4-(pyridin-2-yl) benzyl) pyrazolo[1,5-a][1,3,5] triazine-2,4-diamine family of compounds

### Materials and general organic synthesis methods

All solvents, reagents and active pharmaceutical ingredients were obtained from reputable commercial sources (Sigma-Aldrich, Fluorochem, TCI Chemicals, Acros Organics, Alfa Aesar, Enamine, Thermo Fisher, Carbosynth, WuXi AppTec, MedChemExpress) and used without further purification unless stated otherwise. Anhydrous solvents were purchased from Sigma-Aldrich. Unless stated otherwise, all chemical reactions were carried out at room temperature under an inert argon atmosphere. Compounds were obtained with HPLC purity >95% at 254nm, unless stated otherwise.

Liquid nuclear magnetic resonance spectra were recorded on a Bruker Fourier 300HD or Bruker AVANCE III HD400 spectrometer, using residual solvent peak for calibration. Exchangeable N*H*s were not described. Mass spectroscopy analysis has been performed by the Centre Commun de Spectrométrie de Masse (CCSM) of the UMR5246 CNRS institute of the University Claude Bernard Lyon 1.

Normal phase flash chromatography was performed either on Teledyne Isco CombiFlash^{®} Rf200 devices or Combiflash^{®} NextGen 300 devices, using Macherey-Nagel Chromabond^{®} flash cartridges (40-63µm). Reverse phase chromatography was performed using Biotage^{®} Sfär C18 Duo 100Å 30µm cartridges or using an Agilent 1100 preparative binary HPLC purification system.

Chemical reactions and compound characterization were respectively monitored and analyzed by thin-layer chromatography using pre-coated 40-63µm silica gel (Macherey-Nagel), HPLC-UV (Agilent 1100 systems) or UHPLC-UV/MS (Thermo UltiMate 3000 UHPLC system equipped with a Bruker Impact II^{™} Q-ToF mass spectrometer or Agilent 1260 HPLC system equipped with a Bruker MicrOTOF-QII mass spectrometer).

HPLC Method 1: Agilent 1100 HPLC system equipped with DAD detection. Mobile phase A was water + 0.1% TFA and mobile phase B was acetonitrile. Column was an Agilent Zorbax SB-Aq 4.6x150mm 5µm (room temperature). Linear gradient was 0%B to 50%B in 30 min, followed by a 5 min hold at 50%B. Flow rate was 1.0 mL/min.

HPLC Method 2: Agilent 1100 HPLC system equipped with DAD detection. Mobile phase A was water + 0.1% TFA and mobile phase B was acetonitrile. Column was an Agilent Poroshell 120 EC-C18 3.0x50mm 2.7µm (room temperature). Linear gradient was 5%B to 80%B in 9 min, followed by a 1 min hold at 80%B. Flow rate was 0.8 mL/min.

HPLC Method 3: Agilent 1100 HPLC system equipped with DAD detection. Mobile phase A was water + 0.1% TFA and mobile phase B was acetonitrile. Column was an Agilent Poroshell 120 EC-C18 3.0x50mm 2.7µm (room temperature). Linear gradient was 5%B to 80%B in 20 min, followed by a 2 min hold at 80%B. Flow rate was 0.8 mL/min.

HPLC Method 4: Thermo UltiMate 3000 UHPLC system + Bruker Impact II^{™} Q-ToF mass spectrometer. Mobile phase A was water + 0.1% formic acid and mobile phase B was acetonitrile + 0.1% formic acid. Column was an Agilent PLRP-S 1000Å 2.1x150mm 8µm (80°C). Linear gradient was 10%B to 50%B in 25 min. Flow rate was 0.4 mL/min. UV detection was monitored at 280 nm. The Q-ToF mass spectrometer was used in the m/z range 500-3500 (ESI⁺). Data were deconvoluted using the MaxEnt algorithm included in the Bruker Compass^{®} software.

HPLC Method 5 (preparative method): Teledyne Isco CombiFlash^{®} Rf200 binary MPLC system equipped with DAD detection. Mobile phase A was water + 0.1% TFA and mobile phase B was acetonitrile. Reusable cartridges were Biotage^{®} Sfär C18 Duo 100Å 30µm (30g). Linear gradient was 10%B to 50%B in 35 min, followed by a 5 min hold at 50%B. Flow rate was 25 mL/min.

HPLC Method 6 (preparative method): Agilent 1100 preparative binary HPLC system equipped with dual-loop auto-injector, DAD detection and fraction collector. Mobile phase A was water + 0.1% TFA and mobile phase B was acetonitrile. Column was a Waters SunFire C18 OBD Prep Column, 100Å, 5 µm, 19mm x 250mm (room temperature). Linear gradient was 10%B to 60%B in 40 min, followed by a 5 min hold at 60%B. Flow rate was 25 mL/min.

### 1.1) Synthesis of compound 2384

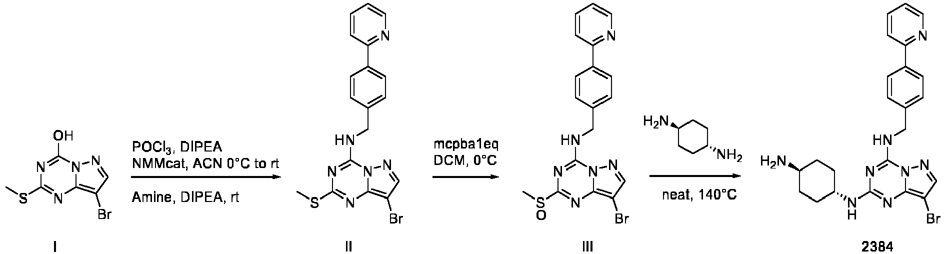

### Synthesis of compound I

Compound 8-Bromo-2-(methylthio)pyrazolo[1,5-*a*]-1,3,5-triazin-4(3*H*)-one [CAS 54346-35-9] (*compound I*) was prepared as described in WO2018195397 with slight modifications. To 2-(methylthio)pyrazolo[1,*5-a*]-1,3,5-triazin-4(3*H*)-one (3.0 g, 16.46 mmol) in DMF (30 mL) was added NBS (2.51 g, 14.10 mmol) within 5 min in small portions, maintaining the temperature between 8-10°C. The mixture was kept for 30 min at 0°C and concentrated in vacuo. The resulting solid was dispersed in EtOH (20 mL), filtered and washed with EtOH (5 mL x2). 3.09 g (84% yield) of bromo derivative as a white solid were obtained after drying. ¹H NMR (300 MHz, DMSO) δ 13.07 (s, 1H), 8.12 (s, 1H), 2.57 (s, 3H). HPLC Method 2 retention time = 4.64 min.

### Synthesis of compound II

Prepared from compound *I* and 4-(Pyridin-2-yl)phenyl)methanamine (the free base was obtained by DCM extraction of pH 9-10 basified aqueous solution of commercial hydrochloride salt CAS1498333-87-1). To a clear solution of 8-Bromo-2-(methylthio)pyrazolo[1,5-a]-1,3,5-triazin-4(3H)-one (*compound I*) (0.685 g, 2.62 mmol), DIPEA (0.914 mL, 2 eq) and N-methyl morpholine (0.001 mL, 0.005 eq) in ACN (4.6 mL) was added POCl₃ (0.318 mL, 1.3 eq) at 0°C. The mixture was then warmed to room temperature and stirred for 1h. To the suspension obtained was added at 0°C (4-(Pyridin-2yl)phenyl)methanamine (0.725 g, 1.5 eq) followed by DIPEA (0.457 mL, 1eq). The mixture clarified briefly before re-precipitation. After overnight stirring at room temperature the solid was filtrated and washed with ACN (1 mL x2) giving expected 8-bromo-2-(methylthio)-N-(4-(pyridin-2-yl)benzyl)pyrazolo[1,5-*a*][1,3,5]triazin-4-amine (0.898 g, 80% yield, HPLC purity 94%, 254 nm) as a beige solid). MS m/z (ESI+): Calc for C₁₈H₁₅BrN₆S [M+H]+ = 427.03 ; Exp [M+H]+ = 427.0. HPLC Method 2, retention time = 5.91 min. ¹H NMR (300 MHz, DMSO) δ 9.66 (t, *J* = 6.2 Hz, 1H), 8.65 (ddd, *J* = 4.8, 1.7, 0.9 Hz, 1H), 8.25 (s, 1H), 8.05 (d, *J* = 8.4 Hz, 2H), 7.95 (d, *J* = 8.0 Hz, 1H), 7.91 - 7.84 (m, 1H), 7.49 (d, *J* = 8.4 Hz, 2H), 7.35 (ddd, *J* = 7.2, 4.8, 1.3 Hz, 1H), 4.74 (d, *J* = 6.2 Hz, 2H), 2.49 (s, 3H). MS m/z (ESI+): Calc for C₁₈H₁₅BrN₆S [M+H]+ = 427.0 ; Exp [M+H]+ = 427.0.

### Synthesis of compound III

To 8-bromo-2-(methylthio)-N-(4-(pyridin-2-yl)benzyl)pyrazolo[1,5-*a*][1,3,5]triazin-4-amine (*compound II*) (0.896 g, 2.10 mmol) in DCM (42 mL) was added MCPBA 70% (0.517 mg, 1.0 eq) portionwise at 0°C within 5 min. The mixture was stirred at 0°C for 1 h, then diluted with DCM (250 mL) and washed with aqueous saturated NaHCOs (50 mL x2) then brine (30 mL). The organic layer was dried on Na₂SO₄, filtered and concentrated in vacuo to give the expected sulfoxide compound *III* (0.920 g, 99% yield, HPLC purity: 91%, 254 nm) as a near white solid. MS m/z (ESI+): Calc for C₁₈H₁₅BrN₆OS [M+H]+ = 443.03 ; Exp [M+H]+ = 443.0. HPLC Method 2, retention time = 4.10 min. ¹H NMR (300 MHz, DMSO) δ 10.10 (s, 1H), 8.64 (ddd, *J* = 4.8, 1.8, 1.0 Hz, 1H), 8.45 (s, 1H), 8.10 - 7.99 (m, 2H), 7.94 (dt, *J* = 8.1, 1.2 Hz, 1H), 7.86 (td, *J* = 7.7, 1.8 Hz, 1H), 7.56 - 7.46 (m, 2H), 7.34 (ddd, *J* = 7.3, 4.8, 1.3 Hz, 1H), 4.79 (s, 2H), 2.83 (s, 3H).

### Synthesis of compound 2384

A mixture of 8-bromo-2-(methylsulfinyl)-N-(4-(pyridin-2-yl)benzyl)pyrazolo[1,5-*a*][1,3,5]triazin-4-amine (*compound III*) (0.262 g, 0.591 mmol) and trans-1,4-diaminocyclohexane (0.540 g, 8 eq) were heated for 30 min at 140°C. After cooling to room temperature was partitioned between DCM (20 mL) and aqueous saturated NaHCOs (10 mL) then extract with DCM (20 mL x2) and washed with brine (10 mL). The organic layer was dried on Na₂SO₄, filtered and concentrated in vacuo. The crude was purified by chromatography on silicagel (15g) eluting with 1% to 20% MeOH in DCM to give compound 2384 (0.150 g, 51% yield) as a white foam. MS m/z (ESI+): Calc for C₂₃H₂₅BrN₈ [M+H]+ = 493.15 ; Exp = 493.2. HPLC Method 2, retention time = 3.84 min. ¹H NMR (300 MHz, DMSO) δ 8.65 (ddd, *J* = 4.8, 1.8, 1.0 Hz, 1H), 8.04 (d, *J* = 8.3 Hz, 2H), 7.94 - 7.91 (m, 2H), 7.86 (ddd, *J* = 8.0, 7.2, 1.8 Hz, 1H), 7.54 - 7.41 (m, 2H), 7.33 (ddd, *J* = 7.2, 4.8, 1.3 Hz, 1H), 4.71 - 4.61 (m, 2H), 3.79 - 3.54 (m, 1H), 2.63 - 2.41 (m, 1H), 1.93 - 1.66 (m, 4H), 1.36 - 1.04 (m, 4H).

### 1.2) Synthesis of compound 2388

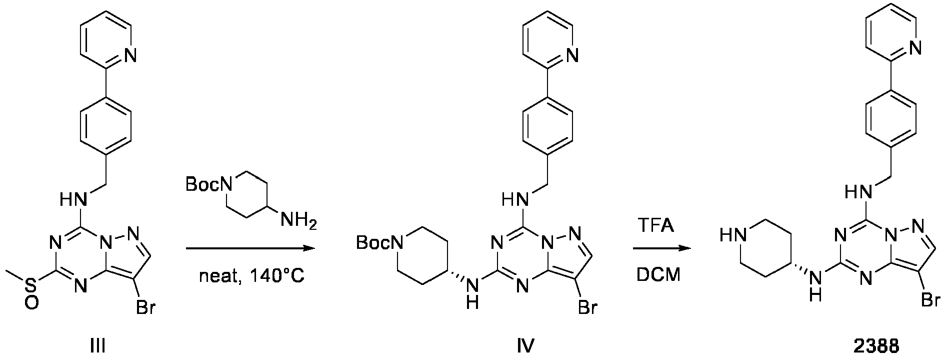

### Synthesis of intermediate compound IV

The described above sulfoxide 8-bromo-2-(methylsulfinyl)-N-(4-(pyridin-2-yl)benzyl)pyrazolo[1,5-a][1,3,5]triazin-4-amine (*compound III*) (0.300 g, 0.68 mmol) and *tert*-Butyl 4-aminopiperidine-1-carboxylate [CAS 87120-72-7] (3 eq) were finely mixed and warmed for 35 min at 145°C. The crude mixture was partitioned between DCM (20 mL) and aqueous saturated NaHCO₃ (10 mL) then extract with DCM (20 mL x2) and washed with brine (10 mL). The organic layer was dried on Na₂SO₄, filtered and concentrated in vacuo. The crude was purified by chromatography on silicagel (15g) eluting with DCM/MeOH: gradient from 100/0 to 95/5 to afford *compound IV* (0.288 g, 73% yield, HPLC purity: 99% at 254 nm) as a beige solid (foam). MS m/z (ESI+): Calc for C₂₇H₃₂BrN₈O₂ [M+H]+ = 579.18 ; Exp = 579.2. HPLC Method 2, retention time = 5.86 min

### Synthesis of compound 2388

The Boc-protected *compound IV* tert-butyl 4-((8-bromo-4-((4-(pyridin-2-yl)benzyl)amino)pyrazolo[1,5-a][1,3,5]triazin-2-yl)amino)piperidine-1-carboxylate (257 mg, 0.44 mmol) in DCM (5 mL) was reacted with TFA (1 mL) at room temperature for 30 min. The mixture was concentrated in vacuo then taken up with water and basified with 1M NaOH (pH9-10). The mixture was partitioned between DCM (20 mL) and aqueous saturated NaHCO₃ (10 mL) then extract with DCM (20 mL x2) and washed with brine (10 mL). The organic layer was dried on Na₂SO₄, filtered and concentrated in vacuo to give 8-bromo-N2-(piperidin-4-yl)-N4-(4-(pyridin-2-yl)benzyl)pyrazolo[1,5-a][1,3,5]triazine-2,4-diamine (*compound* 2388) (0.189 g, 88% yield) as a white foam. MS m/z (ESI+): Calc for C₂₂H₂₄BrN₈ [M+H]+ = 479.1 ; Exp = 479.1. HPLC Method 2, retention time = 3.70 min. ¹H NMR (300 MHz, CDCl3) δ 8.69 (ddd, J = 4.9, 1.8, 1.0 Hz, 1H), 7.99 (d, J = 8.3 Hz, 2H), 7.79 - 7.69 (m, 2H), 7.46 (d, J = 8.2 Hz, 2H), 7.23 (ddd, J = 6.8, 4.8, 1.8 Hz, 1H), 6.91 - 6.55 (m, 1H), 5.28 - 4.91 (m, 1H), 4.79 (broad s, 2H), 4.20 - 3.80 (m, 1H), 3.21 - 2.99 (m, 2H), 2.90 - 2.56 (m, 2H), 2.20 - 1.83 (m, 2H), 1.48 - 1.27 (m, 2H). ¹H NMR (300 MHz, DMSO) δ 8.64 (ddd, *J* = 4.8, 1.8, 1.0 Hz, 1H), 8.04 (d, *J* = 7.0 Hz, 2H), 7.96 - 7.89 (m, 2H), 7.86 (td, *J* = 7.6, 1.8 Hz, 1H), 7.53 - 7.42 (m, 2H), 7.33 (ddd, *J* = 7.3, 4.8, 1.3 Hz, 1H), 4.77 - 4.57 (m, 2H), 3.95 - 3.61 (m, 1H), 2.93 (s, 2H), 2.62 - 2.5 (m, 2H), 1.90 - 1.56 (m, 2H), 1.46 - 1.12 (m, 2H).

### 1.3 Synthesis of compound 2568

### Synthesis of intermediate compound tert-butyl (cis-4-((8-bromo-4-((4-(pyridin-2-yl)benzyl)amino)pyrazolo[1,5-a][1,3,5]triazin-2-yl)amino)cyclohexyl)carbamate

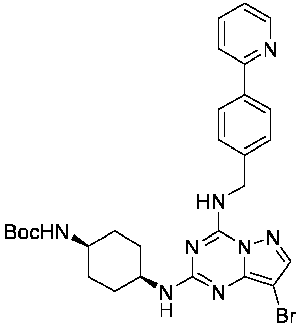

Prepared from N-Boc-cis-1,4-Cyclohexanediamine [CAS n°177906-48-8] and the described above sulfoxide 8-bromo-2-(methylsulfinyl)-N-(4-(pyridin-2-yl)benzyl)pyrazolo[1,5-*a*][1,3,5]triazin-4-amine (*compound III*) (0.100 g, 0.226 mmol) using the same protocol as described above for compound *IV.* Boc protected compound was obtained as a beige solid (foam) (0.113 g, 84% yield). MS m/z (ESI+): Calc for CzaHssBrNaOz [M+H]+ = 593.20 ; Exp = 593.2. HPLC Method 2, retention time = 5.99 min. ¹H NMR (300 MHz, DMSO) δ 8.64 (ddd, *J* = 4.8, 1.8, 1.0 Hz, 1H), 8.04 (d, *J* = 8.0 Hz, 2H), 7.96 - 7.90 (m, 2H), 7.86 (td, *J* = 7.7, 1.8 Hz, 1H), 7.48 (d, *J* = 8.3 Hz, 2H), 7.33 (ddd, *J* = 7.3, 4.8, 1.3 Hz, 1H), 6.61 (s, 1H, NHBoc), 4.78 - 4.60 (m, 2H), 3.89 - 3.68 (m, 1H), 3.39 (broad s, 1H), 1.82 - 1.41 (m, 8H), 1.37 (s, 9H).

### Synthesis of compound 2568

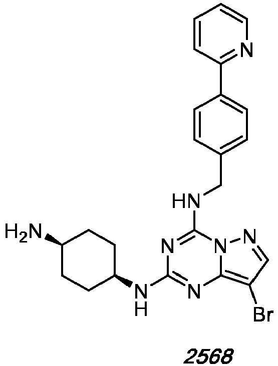

Prepared by TFA deprotection of previous Boc amino compound (0.085 g, 0.143 mmol) as described above in the synthesis procedure of compound 2388. Compound 2568 was obtained as a white foam (0.069 g, 98% yield). MS m/z (ESI+): Calc for C₂₃H₂₅BrN₈ [M+H]+ = 493.15 ; Exp = 493.1. HPLC Method 2, retention time = 3.85 min. ¹H NMR (300 MHz, DMSO) δ 8.64 (ddd, *J* = 4.8, 1.8, 1.0 Hz, 1H), 8.04 (d, *J* = 7.9 Hz, 2H), 7.96 - 7.89 (m, 2H), 7.86 (td, *J* = 7.6, 1.8 Hz, 1H), 7.48 (d, *J* = 8.0 Hz, 2H), 7.33 (ddd, *J* = 7.2, 4.8, 1.3 Hz, 1H), 8.06 - 8.01 (m, 2H), 2.85 (broad s, 1H), 1.82 - 1.38 (m, 8H)

### 1.4 Synthesis of compound 2584

### Synthesis of Boc protected intermediate compound tert-butyl (4-((8-bromo-4-((4-(pyridin-2-yl)benzyl)amino)pyrazolo[1,5-a][1,3,5]triazin-2-yl)amino)phenyl)carbamate

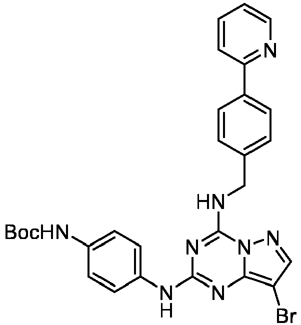

Prepared from *tert*-Butyl (4-aminophenyl)carbamate [CAS n°71026-66-9] and the described above sulfoxide 8-bromo-2-(methylsulfinyl)-N-(4-(pyridin-2-yl)benzyl)pyrazolo[1,5-*a*][1,3,5]triazin-4-amine (0.096 g, 0.217 mmol) using the same protocol described for intermediate *IV*. Boc protected compound was obtained as a beige solid (foam) (0.087 g, 68% yield). MS m/z (ESI+): Calc for C₂₈H₂₈BrN₈O₂ [M+H]+ = 587.15 ; Exp = 587.2. HPLC Method 2, retention time = 6.84 min. ¹H NMR (300 MHz, DMSO) δ 9.52 (broad s, 1H, exch NH), 9.29 (broad s, 1H, exch NH), 9.19 (broad s, 1H, exch NH), 8.65 (ddd, *J* = 4.8, 1.8, 0.9 Hz, 1H), 8.10 -8.03 (m, 3H), 7.95 (dt, *J* = 8.0, 1.2 Hz, 1H), 7.87 (ddd, *J* = 8.1, 7.3, 1.9 Hz, 1H), 7.51 (d, *J* = 8.2 Hz, 2H), 7.41 - 7.20 (m, 3H), 7.74 - 7.44 (m, 2H), 4.75 (d, *J* = 6.1 Hz, 2H).

### Synthesis of compound 2584

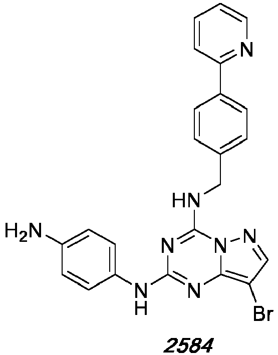

Prepared by TFA deprotection of previous Boc amino compound (0.060 g, 0.102 mmol) as described above in the synthesis procedure of compound 2388. Compound *2584* was obtained as a white foam (0.046 g, 92% yield). MS m/z (ESI+): Calc for C₂₃H₁₉BrN₈ [M+H]+ = 487.10 ; Exp = 487.1. HPLC Method 2, retention time = 4.20 min. ¹H NMR (300 MHz, DMSO) δ 9.19 (broad s, 2H, exch NH), 8.64 (ddd, *J* = 4.8, 1.8, 1.0 Hz, 1H), 8.05 (d, *J* = 8.3 Hz, 2H), 8.01 (s, 1H), 7.96 - 7.90 (m, 1H), 7.86 (td, *J* = 7.6, 1.8 Hz, 1H), 7.48 (d, *J* = 7.9 Hz, 2H), 7.33 (ddd, *J* = 7.3, 4.8, 1.3 Hz, 1H), 7.03 - 7.64 (m, 1H), 6.49 (d, *J* = 8.4 Hz, 2H), 4.79 (s, 2H, exch NH), 4.71 (broad s, 2H).

### 1.5 Synthesis of compound 2559

### Synthesis of Boc protected intermediate compound tert-butyl (1-(8-bromo-4-((4-(pyridin-2-yl)benzyl)amino)pyrazolo[1,5-a][1,3,5]triazin-2-yl)piperidin-4-yl)carbamate

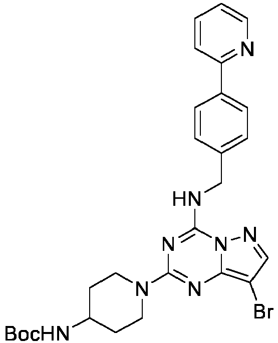

Prepared from 4-(*tert*-Butoxycarbonylamino)piperidine [CAS n°73874-95-0] and the described above sulfoxide 8-bromo-2-(methylsulfinyl)-N-(4-(pyridin-2-yl)benzyl)pyrazolo[1,5-a][1,3,5]triazin-4-amine (0.092 g, 0.208 mmol) using the same protocol as described for intermediate *IV*. Boc protected compound was obtained as a foam (0.105 g, 87% yield). MS m/z (ESI+): Calc for C₂₇H₃₁BrN₈O₂ [M+H]+ = 579.18 ; Exp = 579.2. HPLC Method 2, retention time = 6.69 min. ¹H NMR (300 MHz, DMSO) δ 9.18 (t, *J* = 6.2 Hz, 1H), 8.64 (ddd, *J* = 4.8, 1.8, 1.0 Hz, 1H), 8.04 (d, *J* = 8.3 Hz, 2H), 7.97 (s, 1H), 7.92 (dt, *J* = 8.1, 1.2 Hz, 1H), 7.86 (ddd, *J* = 8.1, 7.2, 1.8 Hz, 1H), 7.50 (d, *J* = 8.3 Hz, 2H), 7.33 (ddd, *J* = 7.2, 4.8, 1.3 Hz, 1H), 6.82 (d, *J* = 8.0 Hz, 1H), 4.66 (d, *J* = 6.1 Hz, 2H), 4.59 - 4.49 (m, 2H), 3.60 - 3.38 (m, 1H), 3.06 -2.92 (m, 2H), 1.81 -1.70 (m, 2H), 1.37 (s, 9H), 1.32 - 1.15 (m, 2H).

### Synthesis of compound 2559

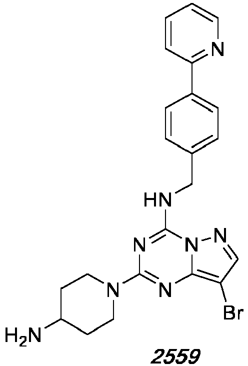

Prepared by TFA deprotection of previous Boc amino compound (0.100 g, 0.173 mmol) as described above in the synthesis procedure of compound *2388*. Compound 2559 was obtained as a white foam (0.072 g, 87% yield). MS m/z (ESI+): Calc for C₂₂H₂₃BrN₈ [M+H]+ = 479.13; Exp = 479.1. HPLC Method 2, retention time = 3.91 min. ¹H NMR (300 MHz, DMSO) δ 8.64 (ddd, *J* = 4.8, 1.8, 0.9 Hz, 1H), 8.03 (d, *J* = 8.3 Hz, 2H), 7.96 (s, 1H), 7.92 (dt, *J* = 8.1, 1.2 Hz, 1H), 7.86 (ddd, *J* = 8.0, 7.2, 1.8 Hz, 1H), 7.50 (d, *J* = 8.4 Hz, 2H), 7.33 (ddd, *J* = 7.3, 4.8, 1.3 Hz, 1H), 4.66 (s, 2H), 4.55 - 4.41 (m, 2H), 3.08 - 2.92 (m, 2H), 2.85 - 2.68 (m, 1H), 1.80 - 1.62 (m, 2H), 1.19 - 1.01 (m, 2H). Exchangeable NH, NH2 were not visible.

### 1.6 Synthesis of compound 2622

### Synthesis of Boc protected intermediate compound tert-butyl 4-(1-(8-bromo-4-((4-(pyridin-2-yl)benzyl)amino)pyrazolo[1,5-a][1,3,5]triazin-2-yl)piperidin-4-yl)piperazine-1-carboxylate

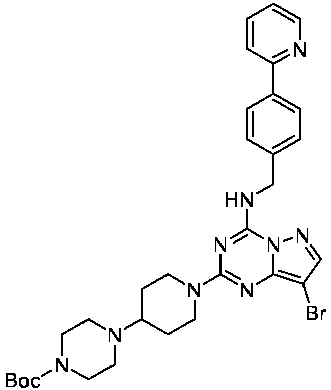

Prepared from 1,1-Dimethylethyl 4-(4-piperidinyl)-1-piperazinecarboxylate [CAS n°205059-24-1] and the described above sulfoxide 8-bromo-2-(methylsulfinyl)-N-(4-(pyridin-2-yl)benzyl)pyrazolo[1,5-a][1,3,5]triazin-4-amine (0.090 g, 0.203 mmol) using the same protocol as described for intermediate *IV*. Boc protected compound was obtained as a foam (0.094 g, 71% yield). MS m/z (ESI+): Calc for C₃₁H₃₈BrN₉O₂ [M+H]+ = 648.24 ; Exp = 648.2. HPLC Method 2, retention time = 5.29 min. ¹H NMR (300 MHz, DMSO) δ 9.19 (t, *J* = 6.1 Hz, 1H), 8.64 (ddd, *J* = 4.8, 1.8, 1.0 Hz, 1H), 8.04 (d, *J* = 8.3 Hz, 2H), 7.98 (s, 1H), 7.92 (dt, *J* = 8.1, 1.2 Hz, 1H), 7.85 (ddd, *J* = 8.0, 7.3, 1.8 Hz, 1H), 7.48 (d, *J* = 8.3 Hz, 2H), 7.32 (ddd, *J* = 7.3, 4.8, 1.3 Hz, 1H), 4.71 - 4.56 (m, 4H), 3.29 - 3.14 (m, 4H), 2.88 - 2.76 (m, 1H), 2.45 - 2.28 (m, 4H), 1.81 - 1.63 (m, 2H), 1.38 (s, 9H), 1.26 - 1.09 (m, 2H).

### Synthesis of compound 2622

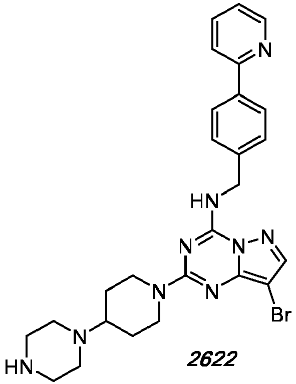

Prepared by TFA deprotection of previous Boc amino compound (0.090 g, 0.139 mmol) as described above in the synthesis procedure of compound 2388, and purified by chromatography on silica gel (gradient 1% to 40% MeOH in DCM). Compound 2622 was obtained as a white foam (0.029 g, 38% yield). MS m/z (ESI+): Calc for C₂₆H₃₀BrN₉ [M+H]+ = 548.19 ; Exp = 548.1. HPLC Method 2, retention time = 3.95 min. ¹H NMR (300 MHz, DMSO) δ 9.18 (bs, 1H), 8.64 (ddd, *J* = 4.8, 1.8, 1.0 Hz, 1H), 8.04 (d, *J* = 8.4 Hz, 2H), 7.97 (s, 1H), 7.92 (dt, *J* = 8.1, 1.2 Hz, 1H), 7.86 (ddd, *J* = 8.0, 7.2, 1.8 Hz, 1H), 7.49 (d, *J* = 8.4 Hz, 2H), 7.33 (ddd, *J* = 7.2, 4.8, 1.3 Hz, 1H), 4.74 - 4.56 (m, 4H), 2.90 - 2.75 (m, 2H), 2.67 - 2.55 (m, 4H), 2.45 - 1.08 (m, 9H).

### 1.7 Synthesis of compound 2569

### Synthesis of Boc protected intermediate compound tert-butyl 4-(((8-bromo-4-((4-(pyridin-2-yl)benzyl)amino)pyrazolo[1,5-a][1,3,5]triazin-2-yl)amino)methyl)piperidine-1-carboxylate

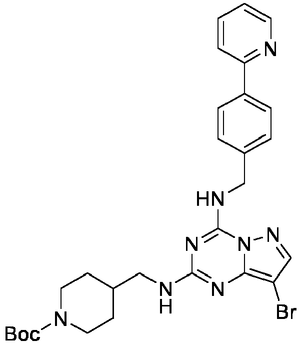

Prepared from [(Piperidin-4-yl)methyl]carbamic acid tert-butyl ester [CAS n°135632-53-0] and the described above sulfoxide 8-bromo-2-(methylsulfinyl)-N-(4-(pyridin-2-yl)benzyl)pyrazolo[1 ,5-*a*][1,3,5]triazin-4-amine (0.088 g, 0.199 mmol) using the same protocol as described for intermediate IV. Boc protected compound was obtained as a colorless resin (0.108 g, 92% yield). MS m/z (ESI+): Calc for CzaHssBrNaOz [M+H]+ = 593.20 ; Exp = 593.2. HPLC Method 2, retention time = 5.82 min. ¹H NMR (300 MHz, DMSO) δ 8.64 (d, *J* = 4.8 Hz, 1H), 8.04 (d, *J* = 8.3 Hz, 2H), 7.97 - 7.81 (m, 3H), 7.46 (d, *J =* 8.1 Hz, 2H), 7.37 - 7.30 (m, 1H), 4.68 (s, 2H), 4.00 - 3.71 (m, 2H), 3.13 (d, *J* = 9.2 Hz, 2H), 2.73 - 2.60 (m, 1H), 1.81 - 1.23 (m, 13H), 1.11 - 0.83 (m, 2H).

### Synthesis of compound 2569

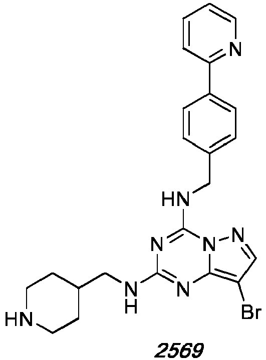

Prepared by TFA deprotection of previous Boc amino compound (0.110 g, 0.185 mmol) as described above in the synthesis procedure of compound 2388. Compound 2569 was obtained as a colorless foam (0.075 g, 82% yield). MS m/z (ESI+): Calc for C₂₃H₂₅BrN₈ [M+H]+ = 493.15 ; Exp = 493.1. HPLC Method 2, retention time = 3.62 min. ¹H NMR (300 MHz, DMSO) δ 8.65 (dt, *J* = 4.9, 1.3 Hz, 1H), 8.04 (d, *J* = 8.4 Hz, 2H), 7.96 (s, 1H), 7.93 (dt, *J* = 8.0, 1.2 Hz, 1H), 7.90 - 7.83 (m, 1H), 7.52 - 7.43 (m, 2H), , 7.34 (ddd, *J* = 7.2, 4.8, 1.3 Hz, 1H), 4.69 (s, 2H), 3.24 - 3.09 (m, 5H), 2.83 - 2.68 - 2.56 (m, 2H), 1.89 - 1.55 (m, 2H), 1.38 - 1.11 (m, 2H).

### 1.8 Synthesis of compound 2560

### Synthesis of Boc protected intermediate compound tert-butyl ((1-(8-bromo-4-((4-(pyridin-2-yl)benzyl)amino)pyrazolo[1,5-a][1,3,5]triazin-2-yl)piperidin-4-yl)methyl)carbamate

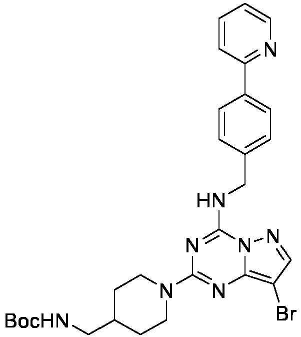

Prepared from [(Piperidin-4-yl)methyl]carbamic acid tert-butyl ester [CAS n°135632-53-0] and the described above sulfoxide 8-bromo-2-(methylsulfinyl)-N-(4-(pyridin-2-yl)benzyl)pyrazolo[1,5-a][1,3,5]triazin-4-amine (0.077 g, 0.174 mmol) using the same protocol as described for intermediate IV. Boc protected compound was obtained as a colorless foam, (0.087 g, 84% yield). MS m/z (ESI+): Calc for C₂₈H₃₃BrN₈O₂ [M+H]+ = 593.20 ; Exp = 593.2. HPLC Method 2, retention time = 6.89 min. ¹H NMR (300 MHz, DMSO) δ 9.14 (t, *J* = 6.2 Hz, 1H), 8.64 (ddd, *J* = 4.8, 1.9, 1.0 Hz, 1H), 8.03 (d, *J* = 8.3 Hz, 2H), 7.96 (s, 1H), 7.93 (dt, *J* = 8.1, 1.2 Hz, 1H), 7.86 (ddd, *J* = 8.0, 7.3, 1.8 Hz, 2H), 7.50 (d, *J* = 8.3 Hz, 2H), 7.33 (ddd, *J* = 7.3, 4.8, 1.3 Hz, 1H), 6.87 (t, *J* = 5.9 Hz, 1H), 4.77 - 4.52 (m, 4H), 2.91 - 2.76 (m, 4H), 1.71 - 1.53 (m, 3H), 1.36 (s, 9H), 1.11 - 0.88 (m, 2H).

### Synthesis of compound 2560

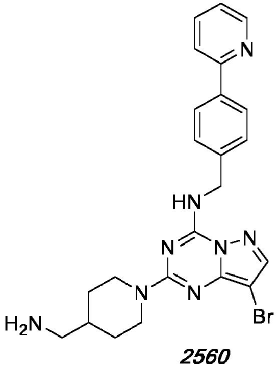

Prepared by TFA deprotection of previous Boc amino compound (0.083 g, 0.140 mmol) as described above in the synthesis procedure of compound 2388. Compound *2560* was obtained as a white foam (0.057 g, 83% yield). MS m/z (ESI+): Calc for C₂₃H₂₅BrN₈ [M+H]+ = 493.15 ; Exp = 493.1. HPLC Method 2, retention time = 4.17 min. ¹H NMR (300 MHz, DMSO) δ 8.64 (ddd, *J* = 4.8, 1.8, 1.0 Hz, 1H), 8.04 (d, *J* = 8.3 Hz, 1H), 7.97 (s, 1H), 7.93 (dt, *J* = 8.1, 1.3 Hz, 1H), 7.86 (ddd, *J* = 8.0, 7.2, 1.8 Hz, 1H), 7.49 (d, *J* = 8.3 Hz, 2H), 7.33 (ddd, *J* = 7.2, 4.8, 1.4 Hz, 1H), 4.73 - 4.57 (m, 4H), 2.92 - 2.76 (m, 2H), 2.66 - 2.54 (m, 2H), 1.79 - 1.62 (m, 2H), 1.62 - 1.47 (m, 1H), 1.09 - 0.88 (m, 2H).

### 1.9 Synthesis of compound 2606

### Synthesis of Boc protected intermediate compound tert-butyl 3-((8-bromo-4-((4-(pyridin-2-yl)benzyl)amino)pyrazolo[1,5-a][1,3,5]triazin-2-yl)amino)piperidine-1 -carboxylate

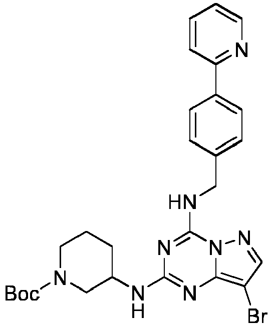

Prepared from 1-Piperidinecarboxylic acid, 3-amino-, 1,1-dimethylethyl ester [CAS n°184637-48-7] and the described above sulfoxide 8-bromo-2-(methylsulfinyl)-N-(4-(pyridin-2-yl)benzyl)pyrazolo[1 ,5-*a*][1,3,5]triazin-4-amine (0.085 g, 0.192 mmol) using the same protocol as described for intermediate IV. Boc protected compound was obtained as a white solid (foam) (0.075 g, 67% yield). MS m/z (ESI+): Calc for C₂₇H₃₁BrN₈O₂ [M+H]+ = 579.18 ; Exp = 579.2. HPLC Method 2, retention time = 6.15 min.

### Synthesis of compound 2606 (racemic)

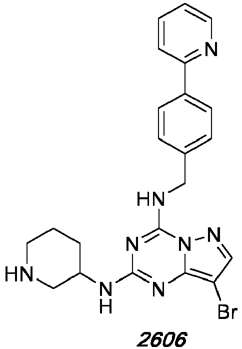

The Boc protected compound was treated with TFA as described above in the synthesis procedure of compound 2388. Compound 2606 (racemic) obtained as a white solid (0.045 g, 74% yield). MS m/z (ESI+): Calc for C₂₂H₂₃BrN₈ [M+H]+ = 479.13; Exp = 479.1. HPLC Method 2, retention time = 3.75 min. ¹H NMR (300 MHz, DMSO) δ 8.64 (ddd, *J* = 4.8, 1.8, 1.0 Hz, 1H), 8.04 (d, *J* = 8.3 Hz, 2H), 7.97 - 7.88 (m, 2H), 7.86 (ddd, *J* = 8.1, 7.2, 1.8 Hz, 1H), 7.56 - 7.42 (m, 2H), 7.33 (ddd, *J* = 7.2, 4.8, 1.3 Hz, 1H), 4.78 - 4.56 (m, 2H), 3.91 - 3.67 (m, 1H), 3.06 - 2.89 (m, 1H), 2.83 - 2.70 (m, 1H), 2.44 - 2.25 (m, 2H), 1.91 - 1.71 (m, 1H), 1.65 - 1.51 (m, 1H), 1.49 - 1.28 (m, 2H).

### Synthesis of compound 2659 (enantiomer S) and 2660 (enantiomer R)

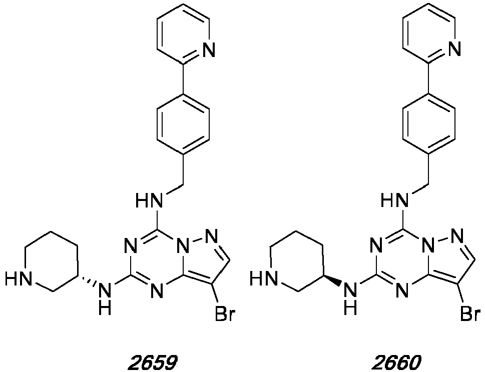

8-bromo-N2-[(3R)-piperidin-3-yl]-N4-{[4-(pyridin-2-yl)phenyl]methyl}pyrazolo[1,5-a][1,3,5]triazine-2,4-diamine: *2660 (enantiomer R) and* 8-bromo-N2-[(3R)-piperidin-3-yl]-N4-{[4-(pyridin-2-yl)phenyl]methyl}pyrazolo[1 ,5-a][1 ,3,5]triazine-2,4-diamine 2659 *(enantiomer S)* were prepared from optically active Boc amine in the same way with 92% and 86% respectively from corresponding (3R)-3-aminopiperidine-1-carboxylate and (3S)-3-aminopiperidine-1-carboxylate.

### 1.10 Synthesis of compound 2538

### Synthesis of Boc protected (2S,4R)-tert-butyl 2-(((8-bromo-4-((4-(pyridin-2-yl)benzyl)amino)pyrazolo[1,5-a] [1,3,5]triazin-2-yl)amino)methyl)-4-hydroxypyrrolidine-1 - carboxylate

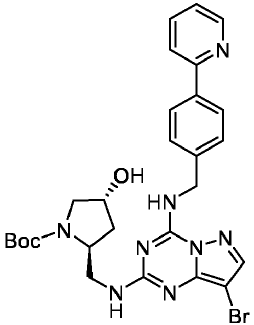

Prepared from (2*R*,4*S*)-1-Pyrrolidinecarboxylic acid, 2-(aminomethyl)-4-hydroxy-, 1,1-dimethylethyl ester [CAS n°1983918-82-6] and the described above sulfoxide 8-bromo-2-(methylsulfinyl)-N-(4-(pyridin-2-yl)benzyl)pyrazolo[1,5-*a*][1,3,5]triazin-4-amine (0.080 g, 0.180 mmol) using the same protocol as described intermediate IV. Boc protected compound was obtained as a white solid (0.086 g, 80% yield). MS m/z (ESI+): Calc for C₂₇H₃₁BrN₈O₃ [M+H]+ = 595.18 ; Exp = 595.2. HPLC Method 2, retention time = 5.16 min. ¹H NMR (300 MHz, DMSO) δ 8.64 (ddd, *J* = 4.9, 1.8, 1.0 Hz, 1H), 8.03 (d, *J* = 8.0 Hz, 2H), 7.95 (s, 1H), 7.92 (d, *J* = 7.9 Hz, 1H), 7.86 (td, *J* = 7.6, 1.8 Hz, 0H), 7.60 - 7.52 (m, 0.5H, exch NH), 7.47 (d, *J* = 7.9 Hz, 2H), 7.33 (ddd, *J* = 7.2, 4.8, 1.4 Hz, 1H), 4.93 - 4.62 (m, 3H, exch OH, CH2), 4.29 - 3.90 (m, 2H), 3.83 - 3.44 (m, 1H), 3.32 - 3.10 (m, 3H), 1.98 - 1.67 (m, 2H), 1.47 - 1.21 (m, 9H).

### Synthesis of compound 2538

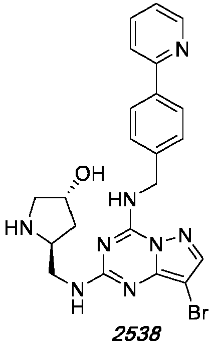

Prepared by TFA deprotection of previous Boc amino compound (0.080 g, 0.134 mmol) as described above in the synthesis procedure of compound 2388. Compound 2538 was obtained as a grey solid (0.066 g, 98% yield). MS m/z (ESI+): Calc for C₂₂H₂₃BrN₈O [M+H]+ = 495.13 ; Exp = 495.1. HPLC Method 2, retention time = 3.75 min. ¹H NMR (300 MHz, DMSO) δ 8.65 (ddd, *J* = 4.8, 1.8, 0.9 Hz, 1H), 8.04 (d, *J* = 8.3 Hz, 2H), 7.95 - 7.91 (m, 2H), 7.86 (ddd, *J* = 8.0, 7.2, 1.8 Hz, 1H), 7.54 - 7.42 (m, 2H), 7.33 (ddd, *J* = 7.3, 4.8, 1.3 Hz, 1H), 4.68 (s, 2H), 4.58 - 4.47 (m, 1H), 4.21 - 4.02 (m, 1H), 3.51 - 3.36 (m, 1H), 3.28 - 3.10 (m, 2H), 2.96 - 2.78 (m, 1H), 1.56 (d, *J* = 56.1 Hz, 4H).

### 1.11 Synthesis of compound 2571

### Synthesis of Boc protected (S)-tert-butyl 2-(((8-bromo-4-((4-(pyridin-2-yl)benzyl)amino)pyrazolo[1,5-a][1,3,5]triazin-2-yl)amino)methyl)pyrrolidine-1-carboxylate

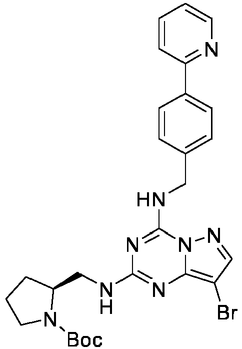

Prepared from (*S*)-*tert*-Butyl 2-(aminomethyl)pyrrolidine-1-carboxylate [CAS n°119020-01-8] and the described above sulfoxide 8-bromo-2-(methylsulfinyl)-N-(4-(pyridin-2-yl)benzyl)pyrazolo[1 ,5-*a*][1,3,5]triazin-4-amine (0.087 g, 0.196 mmol) using the same protocol as described for intermediate IV. Boc protected compound was obtained as a colorless solid (foam) (0.089 g, 78% yield). HRMS m/z (ESI+): Calc for C₂₇H₃₁BrN₈O₂ [M+H]+ = 579.1826 ; Exp = 579.1827. HPLC Method 2, retention time = 6.02 min. ¹H NMR (300 MHz, DMSO) δ 8.64 (ddd, *J* = 4.8, 1.8, 1.0 Hz, 1H), 8.03 (d, *J* = 7.9 Hz, 2H), 7.95 (s, 1H), 7.92 (broad d, *J* = 8.0 Hz, 1H), 7.86 (td, *J* = 7.6, 1.8 Hz, 1H), 7.46 (d, *J* = 8.1 Hz, 2H), 7.33 (ddd, *J* = 7.2, 4.8, 1.3 Hz, 1H), 4.94 - 4.61 (m, 2H), 4.02 - 3.78 (m, 1H), 3.32 (s, 4H), 1.94 - 1.56 (m, 4H), 1.45 - 1.22 (m, 9H).

### Synthesis of compound 2571

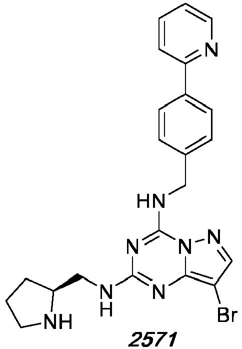

Prepared by TFA deprotection of previous Boc amino compound (0.087 g, 0.150 mmol) as described above in the synthesis procedure of compound 2388. Compound 2571 was obtained as a white foam (0.070 g, 97% yield). MS m/z (ESI+): Calc for C₂₂H₂₃BrN₈ [M+H]+ = 479.1302 ; Exp = 479.1301. HPLC Method 2, retention time = 3.88 min. ¹H NMR (300 MHz, DMSO) δ 9.43 - 8.80 (m, 1H), 8.64 (ddd, *J* = 4.8, 1.8, 1.0 Hz, 1H), 8.04 (d, *J* = 8.4 Hz, 2H), 7.95 (s, 1H), 7.93 (dt, *J* = 8.0, 1.3 Hz, 1H), 7.86 (ddd, *J* = 8.0, 7.2, 1.8 Hz, 1H), 7.47 (broad d, *J* = 7.9 Hz, 2H), 7.33 (ddd, *J* = 7.3, 4.8, 1.3 Hz, 1H), 4.68 (s, 2H), 3.47 - 2.98 (m, 4H), 2.91 - 2.58 (m, 2H), 1.83 - 1.20 (m, 5H).

### 1.12 Synthesis of compound 2585

### Synthesis of Boc protected tert-butyl (4-((8-bromo-4-((4-(pyridin-2-yl)benzyl)amino)pyrazolo[1,5-a][1,3,5]triazin-2-yl)amino)butyl)carbamate

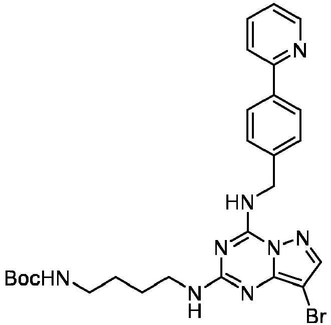

Prepared from *N*-(*tert*-Butoxycarbonyl)-1,4-butanediamine [CAS n°68076-36-8] and the described above sulfoxide 8-bromo-2-(methylsulfinyl)-N-(4-(pyridin-2-yl)benzyl)pyrazolo[1,5-*a*][1,3,5]triazin-4-amine (0.100 g, 0.226 mmol) using the same protocol as described for intermediate IV. Boc protected compound was obtained as a white foam (0.113 g, 88% yield). MS m/z (ESI+): Calc for C₂₆H₃₁BrN₈O₂ [M+H]+ = 567.18 ; Exp = 567.2. HPLC Method 2, retention time = 5.49min. ¹H NMR (300 MHz, DMSO) δ 8.65 (ddd, *J* = 4.8, 1.8, 1.0 Hz, 1H), 8.05 (d, *J* = 8.1 Hz, 2H), 7.97 - 7.91 (m, 2H), 7.90 - 7.83 (m, 1H), 7.55 - 7.41 (m, 2H), 7.34 (ddd, *J* = 7.3, 4.8, 1.3 Hz, 1H), 4.77 - 4.63 (m, 2H), 3.31 - 3.18 (m, 2H), 3.01 - 2.83 (m, 2H), 1.53 - 1.29 (m, 13H).

### Synthesis of compound 2585

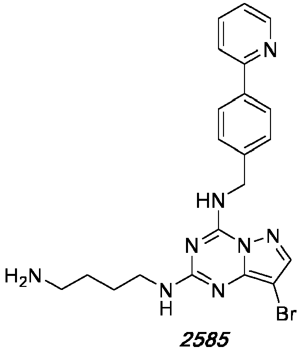

Prepared by TFA deprotection of previous Boc amino compound (0.085 g, 0.143 mmol) as described above in the synthesis procedure of compound 2388. Compound 2585 was obtained as a white foam (0.069 g, 98% yield). MS m/z (ESI+): Calc for C₂₁H₂₃BrN₈ [M+H]+ = 467.12 ; Exp = 467.1. HPLC Method 2, retention time = 3.56 min. ¹H NMR (300 MHz, DMSO) δ 8.65 (ddd, *J* = 4.9, 1.8, 1.0 Hz, 1H), 8.05 (d, *J* = 8.3 Hz, 2H), 7.96 (s, 1H), 7.93 (dt, *J* = 8.1, 1.2 Hz, 1H), 7.87 (ddd, *J* = 8.0, 7.3, 1.8 Hz, 1H), 7.54 - 7.42 (m, 2H), 7.34 (ddd, *J* = 7.2, 4.8, 1.4 Hz, 1H), 4.69 (s, 2H), 3.31 - 3.21 (m, 2H), 2.87 - 2.74 (m, 2H), 1.63 - 1.42 (m, 4H).

### 1.13 Synthesis of compound 2638

### Synthesis of Boc protected tert-butyl (2-((8-bromo-4-((4-(pyridin-2-yl)benzyl)amino)pyrazolo[1,5-a][1,3,5]triazin-2-yl)amino)ethyl)carbamate

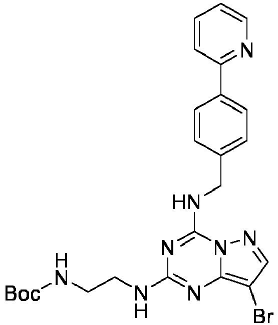

Prepared from 1,1-Dimethylethyl *N*-(2-aminoethyl)carbamate [CAS n° 57260-73-8] and the described above sulfoxide 8-bromo-2-(methylsulfinyl)-N-(4-(pyridin-2-yl)benzyl)pyrazolo[1,5-*a*][1,3,5]triazin-4-amine (0.074 g, 0.167 mmol) using the same protocol as described for intermediate IV. Boc protected compound was obtained as a white solid (foam) (0.075 g, 83% yield). MS m/z (ESI+): Calc for C₂₄H₂₇BrN₈O₂ [M+H]+ = 539.15 ; Exp = 539.1. HPLC Method 2, retention time = 5.15 min. ¹H NMR (300 MHz, DMSO) δ 8.66 (ddd, *J* = 4.8, 1.8, 1.0 Hz, 1H), 8.05 (d, *J* = 8.1 Hz, 2H), 7.99 - 7.91 (m, 2H), 7.87 (td, *J* = 7.6, 1.8 Hz, 1H), 7.60 - 7.43 (m, 2H), 7.34 (ddd, *J* = 7.2, 4.8, 1.3 Hz, 1H), 6.92 - 6.79 (m, 1H), 4.78 - 4.63 (m, 2H), 3.33 - 3.21 (m, 2H), 3.21 - 3.01 (m, 2H), 1.37 (s, 9H).

### Synthesis of compound 2638

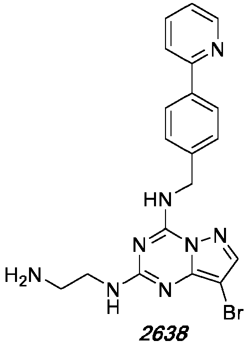

Prepared by TFA deprotection of previous Boc amino compound (0.071 g, 0.132 mmol) as described above in the synthesis procedure of compound 2388 and purified by chromatography on silicagel (gradient 1% to 60% MeOH/DCM). Compound 2638 was obtained as a white solid (0.041 g, 71 % yield). MS m/z (ESI+): Calc for C₁₉H₁₉BrN₈ [M+H]+ = 439.10 ; Exp = 439.1. HPLC Method 2, retention time = 3.52 min. ¹H NMR (300 MHz, DMSO) δ 8.64 (ddd, *J* = 4.8, 1.8, 1.0 Hz, 1H), 8.04 (d, *J* = 8.3 Hz, 2H), 7.97 - 7.90 (m, 2H), 7.86 (ddd, *J* = 8.1, 7.3, 1.8 Hz, 1H), 7.47 (d, *J* = 8.6 Hz, 2H), 7.33 (ddd, *J* = 7.2, 4.8, 1.3 Hz, 1H), 4.68 (s, 2H), 3.32 - 3.20 (m, 2H), 2.75 - 2.56 (m, 2H).

### 1.14 Synthesis of compound 2586

### Synthesis of Boc protected tert-butyl (2-((8-bromo-4-((4-(pyridin-2-yl)benzyl)amino)pyrazolo[1,5-a][1,3,5]triazin-2-yl)amino)ethyl)(methyl)carbamate

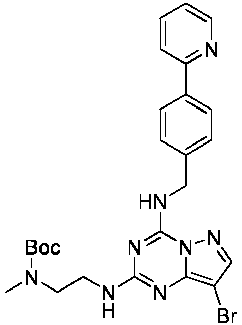

Prepared from 1,1-Dimethylethyl *N*-(2-aminoethyl)-*N*-methylcarbamate [CAS n°121492-06-6] and the described above sulfoxide 8-bromo-2-(methylsulfinyl)-N-(4-(pyridin-2-yl)benzyl)pyrazolo[1,5-a][1,3,5]triazin-4-amine (0.100 g, 0.226 mmol) using the same protocol as described for intermediate IV. Boc protected compound was obtained as a white solid (foam) (0.113 g, 90% yield). MS m/z (ESI+): Calc for C₂₅H₂₉BrN₈O₂ [M+H]+ = 553.17 ; Exp = 553.2. HPLC Method 2, retention time = 5.58 min. ¹H NMR (300 MHz, DMSO) δ 8.68 - 8.63 (m, 1H), 8.04 (d, *J=* 7.9 Hz, 2H), 7.97 (s, 1H), 7.96 - 7.91 (m, 1H), 7.87 (td, *J* = 7.7, 1.8 Hz, 1H), 7.53 - 7.43 (m, 3H), 7.34 (ddd, *J* = 7.3, 4.8, 1.3 Hz, 1H), 4.70 (broad s, 2H), 3.50 - 3.25 (m, 3H), 2.92 - 2.66 (m, 2H), 1.45 - 1.06 (m, 11H).

### Synthesis of compound 2586

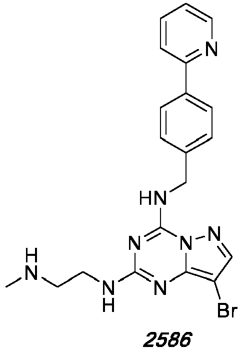

Prepared by TFA deprotection of previous Boc amino compound (0.104 g, 0.188 mmol) as described above in the synthesis procedure of compound 2388. Compound 2586 was obtained as a white solid (0.076 g, 89% yield). MS m/z (ESI+): Calc for C₂₀H₂₁BrN₈ [M+H]+ = 453.11 ; Exp = 453.1. HPLC Method 2, retention time = 3.52 min. ¹H NMR (300 MHz, DMSO) δ 9.30 - 8.81 (m, 1H), 8.65 (ddd, *J* = 4.8, 1.8, 1.0 Hz, 1H), 8.05 (d, *J* = 8.3 Hz, 2H), 7.97 - 7.90 (m, 2H), 7.86 (td, *J* = 7.6, 1.8 Hz, 1H), 7.54 - 7.43 (m, 2H), 7.34 (ddd, *J* = 7.3, 4.8, 1.3 Hz, 1H), 4.69 (s, 2H), 3.39 - 3.33 (m, 2H), 2.71 - 2.55 (m, 2H), 2.38 - 2.15 (m, 3H).

### 1.15 Synthesis of compound 2600

### Synthesis of Boc protected tert-butyl (2-((8-bromo-4-((4-(pyridin-2-yl)benzyl)amino)pyrazolo[1,5-a][1,3,5]triazin-2-yl)(methyl)amino)ethyl)carbamate

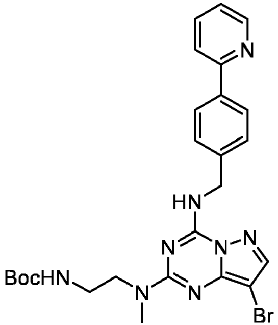

Prepared from 1,1-Dimethylethyl *N*-[2-(methylamino)ethyl]carbamate [CAS n°122734-32-1] and the described above sulfoxide 8-bromo-2-(methylsulfinyl)-N-(4-(pyridin-2-yl)benzyl)pyrazolo[1 ,5-a][1,3,5]triazin-4-amine (0.092 g, 0.208 mmol) using the same protocol as described for intermediate IV. Boc protected compound was obtained as a white solid (foam) (0.098 g, 85% yield). MS m/z (ESI+): Calc for C₂₅H₂₉BrN₈O₂ [M+H]+ = 553.17 ; Exp = 553.2. HPLC Method 2, retention time = 6.23 min. ¹H NMR (300 MHz, DMSO) δ 9.14 (t, *J* = 6.1 Hz, 1H), 8.64 (ddd, *J* = 4.8, 1.8, 1.0 Hz, 1H), 8.03 (d, *J* = 8.3 Hz, 2H), 7.96 (s, 1H), 7.92 (dt, *J* = 8.1, 1.3 Hz, 1H), 7.86 (ddd, *J* = 8.1, 7.2, 1.8 Hz, 1H), 7.52 (d, *J* = 7.9 Hz, 2H), 7.33 (ddd, *J* = 7.2, 4.8, 1.4 Hz, 1H), 6.93 - 6.69 (m, 1H), 4.69 (d, *J* = 6.1 Hz, 2H), 3.60 (t, *J* = 6.4 Hz, 2H), 3.18 - 3.02 (m, 5H), 1.33 (s, 9H).

### Synthesis of compound 2600

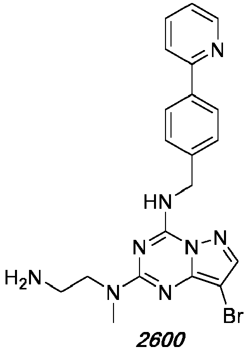

Prepared by TFA deprotection of previous Boc amino compound (0.092 g, 0.166 mmol) as described above in the synthesis procedure of compound 2388. Compound 2600 was obtained as a white foam (0.072 g, 95% yield). MS m/z (ESI+): Calc for C₂₀H₂₁BrN₈ [M+H]+ = 453.11 ; Exp = 453.1. HPLC Method 2, retention time = 3.99 min. ¹H NMR (300 MHz, DMSO) δ 8.64 (ddd, *J* = 4.8, 1.8, 1.0 Hz, 1H), 8.04 (d, *J* = 8.3 Hz, 2H), 7.95 (s, 1H), 7.92 (dt, *J* = 8.1, 1.2 Hz, 1H), 7.86 (ddd, *J* = 8.0, 7.2, 1.8 Hz, 1H), 7.51 (d, *J* = 8.4 Hz, 2H), 7.33 (ddd, *J* = 7.3, 4.8, 1.3 Hz, 1H), 4.67 (s, 2H), 3.54 (t, *J* = 6.7 Hz, 2H), 3.09 (broad s, 3H), 2.82 - 2.58 (m, 2H).

### 1.16 Synthesis of compound 2605

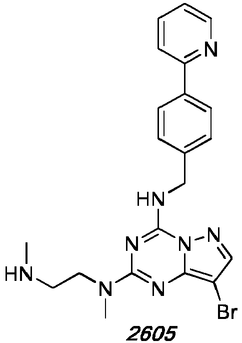

Prepared from sulfoxide compound III (0.082 g, 0.185 mmol) and N,N'-dimethylamino ethylenediamine as described above for compound 2384. Compound 2605 was obtained as a white foam (0.050 g, 58% yield). MS m/z (ESI+): Calc for C₂₁H₂₃BrN₈ [M+H]+ = 467.13 ; Exp = 467.1. HPLC Method 2, retention time = 4.09 min. ¹H NMR (300 MHz, DMSO) δ z8.64 (ddd, *J* = 4.8, 1.9, 1.0 Hz, 1H), 8.04 (d, *J* = 8.3 Hz, 2H), 7.96 (s, 1H), 7.92 (dt, *J* = 8.1, 1.2 Hz, 1H), 7.86 (ddd, *J* = 8.1, 7.2, 1.8 Hz, 1H), 7.49 (broad d, *J* = 8.0 Hz, 2H), 7.33 (ddd, *J* = 7.2, 4.8, 1.4 Hz, 1H), 4.67 (broad s, 2H), 3.63 (t, *J=* 6.5 Hz, 2H), 3.08 (broad s, 3H), 2.70 - 2.54 (m, 2H), 2.34 - 2.09 (m, 3H).

### 1.17 Synthesis of compound 2601

### Synthesis of Boc protected tert-butyl 4-(8-bromo-4-((4-(pyridin-2-yl)benzyl)amino)pyrazolo[1,5-a][1,3,5]triazin-2-yl)piperazine-1-carboxylate

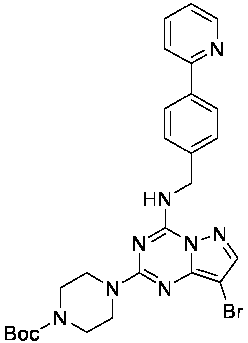

Prepared from Boc piperazine [CAS n°57260-71-6] and the described above sulfoxide 8-bromo-2-(methylsulfinyl)-N-(4-(pyridin-2-yl)benzyl)pyrazolo[1,5-*a*][1,3,5]triazin-4-amine (0.091 g, 0.205 mmol) using the same protocol as described for intermediate IV. Boc protected compound was obtained as a white solid (foam) (0.098 g, 84% yield). MS m/z (ESI+): Calc for C₂₆H₂₉BrN₈O₂ [M+H]+ = 565.17 ; Exp = 565.2. HPLC Method 2, retention time = 7.08 min. ¹H NMR (300 MHz, DMSO) δ 9.24 (t, *J* = 6.1 Hz, 1H), 8.64 (ddd, *J* = 4.8, 1.8, 1.0 Hz, 1H), 8.04 (d, *J* = 8.4 Hz, 2H), 8.00 (s, 1H), 7.93 (dt, *J* = 8.1, 1.2 Hz, 1H), 7.86 (ddd, *J* = 8.0, 7.2, 1.8 Hz, 1H), 7.51 (d, *J* = 8.3 Hz, 2H), 7.33 (ddd, *J* = 7.2, 4.8, 1.3 Hz, 1H), 4.68 (d, *J* = 5.9 Hz, 2H), 3.76 - 3.69 (m, 4H), 3.42 - 3.32 (m, 4H), 1.40 (s, 9H).

### Synthesis of compound 2601

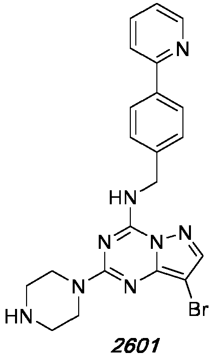

Prepared by TFA deprotection of previous Boc amino compound (0.092 g, 0.163 mmol) as described above in the synthesis procedure of compound 2388. Compound 2601 was obtained as a white solid (0.073 g, 96% yield). MS m/z (ESI+): Calc for C₂₁H₂₁BrN₈ [M+H]+ = 465.12 ; Exp = 465.1. HPLC Method 2, retention time = 3.99 min. ¹H NMR (300 MHz, DMSO) δ 9.18 - 9.12 (m, 1H), 8.64 (ddd, *J* = 4.8, 1.8, 1.0 Hz, 1H), 8.04 (d, *J* = 8.3 Hz, 2H), 7.97 (s, 1H), 7.93 (dt, *J* = 8.0, 1.2 Hz, 1H), 7.86 (ddd, *J* = 8.0, 7.2, 1.8 Hz, 1H), 7.49 (d, *J* = 8.4 Hz, 2H), 7.33 (ddd, *J* = 7.2, 4.8, 1.3 Hz, 1H), 4.67 (d, *J* = 4.9 Hz, 2H), 3.69 - 3.63 (m, 4H), 2.70 - 2.64 (m, 4H).

### 1.18 Synthesis of compound 2655

### Synthesis of Boc protected tert-butyl N-[(3S)-1-[8-bromo-4-({[4-(pyridin-2-yl)phenyl]methyl}amino)pyrazolo[1,5-a][1,3,5]triazin-2-yl]pyrrolidin-3-yl]carbamate

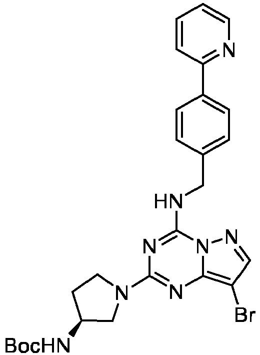

Prepared from tert-butyl N-[(3S)-pyrrolidin-3-yl]carbamate [CAS n° 122536-76-9] and the described above sulfoxide 8-bromo-2-(methylsulfinyl)-N-(4-(pyridin-2-yl)benzyl)pyrazolo[1,5-*a*][1,3,5]triazin-4-amine (0.069 g, 0.156 mmol) using the same protocol as described for intermediate IV. Boc protected compound was obtained as a white solid (foam) (0.088 g, quant. yield). MS m/z (ESI+): Calc for C₂₆H₂₉BrN₈O₂ [M+H]+ = 565.17 ; Exp = 565.3. HPLC Method 2, retention time = 6.00 min.

### Synthesis of compound 2655

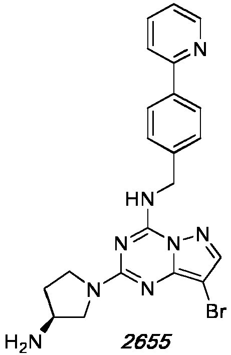

The Boc protected compound (0.086 g, 0.152 mmol) was treated with TFA as described above in the synthesis procedure of compound 2388. Compound 2655 was obtained as a white solid (0.070 g, 99% yield). MS m/z (ESI+): Calc for C₂₁H₂₁BrN₈ [M+H]+ = 465.11; Exp = 465.2. HPLC Method 2, retention time = 3.73 min. ¹H NMR (300 MHz, DMSO) δ 8.64 (ddd, *J* = 4.8, 1.8, 1.0 Hz, 1H), 8.04 (d, *J* = 8.2 Hz, 2H), 7.93 (m, 2H), 7.86 (td, *J* = 7.6, 1.8 Hz, 1H), 7.51 (d, *J* = 8.1 Hz, 2H), 7.33 (ddd, *J* = 7.3, 4.8, 1.3 Hz, 1H), 4.67 (s, 2H), 3.74 - 3.37 (m, 4H), 3.26 - 3.11 (m, 1H), 2.08 - 1.55 (m, 2H).

### 1.19 Synthesis of compounds 2656 (R enantiomer) and 2657 (S enantiomer)

### Synthesis of Boc protected tert-butyl (3R)-3-{[8-bromo-4-({[4-(pyridin-2-yl)phenyl]methyl}amino)pyrazolo[1,5-a][1,3,5]triazin-yl]amino}pyrrolidine-1 -carboxylate

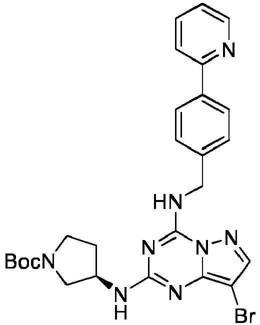

Prepared from tert-butyl (3R)-3-aminopyrrolidine-1-carboxylate [CAS n°147081-49-0] and the described above sulfoxide 8-bromo-2-(methylsulfinyl)-N-(4-(pyridin-2-yl)benzyl)pyrazolo[1 ,5-*a*][1,3,5]triazin-4-amine (0.065 g, 0.147 mmol) using the same protocol as described for intermediate IV. Boc protected compound was obtained as a white solid (foam) (0.083 g, quant. yield). MS m/z (ESI+): Calc for C₂₆H₂₉BrN₈O₂ [M+H]+ = 565.17 ; Exp = 565.2. HPLC Method 2, retention time = 6.00 min.

### Synthesis of compound 2656

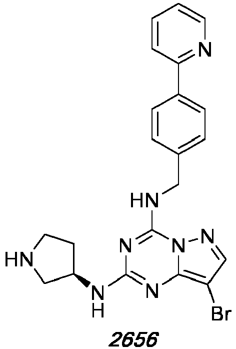

The Boc protected compound (0.080 g, 0.141 mmol) was treated with TFA as described above in the synthesis procedure of compound 2388. Compound 2656 (R enantiomer) was obtained as a white solid (0.051g, 77% yield). MS m/z (ESI+): Calc for C₂₁H₂₁BrN₈ [M+H]+ = 465.11; Exp = 465.2. HPLC Method 2, retention time = 3.67 min. ¹H NMR (300 MHz, DMSO) δ 8.64 (ddd, *J* = 4.8, 1.8, 1.0 Hz, 1H), 8.04 (d, *J* = 8.3 Hz, 2H), 7.97 - 7.90 (m, 2H), 7.86 (ddd, *J* = 8.0, 7.2, 1.8 Hz, 1H), 7.52 - 7.43 (m, 2H), 7.33 (ddd, *J =* 7.2, 4.8, 1.3 Hz, 1H), 4.69 (s, 2H), 4.37 - 4.19 (m, 1H), 3.00 - 2.57 (m, 4H), 2.02 - 1.82 (m, 1H), 1.68 - 1.47 (m, 1H).

### Synthesis of compound 2657

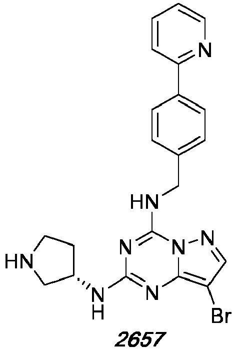

Compound 2657 (S enantiomer) was prepared as described above for compound 2656 with 79% overall yield from tert-butyl (3S)-3-aminopyrrolidine-1-carboxylate [CAS n°147081-44-5].

### 1.20 Synthesis of compound 2658

### Synthesis of Boc protected tert-butyl N-(3-{[8-bromo-4-({[4-(pyridin-2-yl)phenyl]methyl}amino)pyrazolo[1,5-a][1,3,5]triazin-2-yl]amino}propyl)carbamate

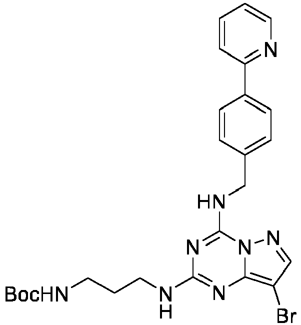

Prepared from tert-butyl N-(3-aminopropyl)carbamate [CAS n° 75178-96-0] and the described above sulfoxide 8-bromo-2-(methylsulfinyl)-N-(4-(pyridin-2-yl)benzyl)pyrazolo[1,5-*a*][1,3,5]triazin-4-amine (0.061 g, 0.138 mmol) using the same protocol as described for intermediate IV. Boc protected compound was obtained as a white solid (foam) (0.067 g, 88% yield). MS m/z (ESI+): Calc for C₂₅H₂₉BrN₈O₂ [M+H]+ = 553.17 ; Exp = 553.2. HPLC Method 2, retention time = 6.5.37 min.

### Synthesis of compound 2658

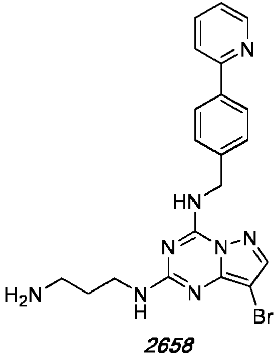

The Boc protected compound (0.065, 0.149 mmol) was treated with TFA as described above in the synthesis procedure of compound 2388. Compound 2658 was obtained as a white solid (0.053 g, 79% yield). MS m/z (ESI+): Calc for C₂₀H₂₁BrN₈ [M+H]+ = 453.11; Exp = 453.1. HPLC Method 2, retention time = 3.42 min. ¹H NMR (300 MHz, DMSO) δ 8.64 (ddd, *J* = 4.8, 1.8, 0.9 Hz, 1H), 8.04 (d, *J* = 8.3 Hz, 2H), 7.97 - 7.89 (m, 2H), 7.86 (td, *J* = 7.6, 1.8 Hz, 1H), 7.52 - 7.42 (m, 2H), 7.33 (ddd, *J* = 7.3, 4.8, 1.3 Hz, 1H), 4.68 (s, 2H), 3.45 - 3.16 (m, 4H), 1.58 (m, 2H).

### 1.21 Synthesis of compound 2668

### Synthesis of Boc protected tert-butyl N-[(1S,2S)-2-{[8-bromo-4-({[4-(pyridin-2-yl)phenyl]methyl}amino)pyrazolo[1,5-a][1,3,5]triazin-2-yl]amino}cyclopentyl]carbamate

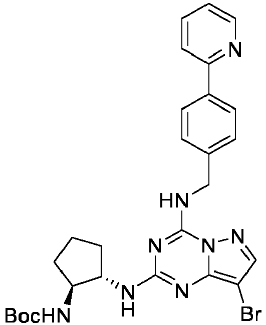

Prepared from tert-butyl N-[(1S,2S)-2-aminocyclopentyl]carbamate [CAS n° 586961-34-4] and the described above sulfoxide 8-bromo-2-(methylsulfinyl)-N-(4-(pyridin-2-yl)benzyl)pyrazolo[1 ,5-*a*][1 ,3,5]triazin-4-amine (0.070 g, 0.158 mmol) using the same protocol as described for intermediate IV. Boc protected compound was obtained as a white solid (foam) (0.079 g, 86% yield). MS m/z (ESI+): Calc for C₂₇H₃₁BrN₈O₂ [M+H]+ = 579.18 ; Exp = 579.3. HPLC Method 2, retention time = 6.02 min.

### Synthesis of compound 2668

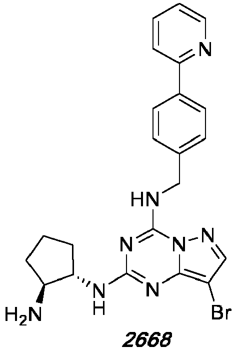

The Boc protected compound (0.076 g, 0.131 mmol) was treated with TFA as described above in the synthesis procedure of compound 2388. Compound 2668 was obtained as a white solid (0.048 g, 76% yield). MS m/z (ESI+): Calc for C₂₂H₂₃BrN₈ [M+H]+ = 479.13; Exp = 479.1. HPLC Method 2, retention time = 3.75 min ¹H NMR (300 MHz, DMSO) δ 8.64 (ddd, *J* = 4.8, 1.8, 0.9 Hz, 1H), 8.04 (d, *J* = 8.3 Hz, 2H), 7.96 - 7.89 (m, 2H), 7.86 (ddd, *J* = 8.0, 7.3, 1.8 Hz, 1H), 7.55 - 7.38 (m, 2H), 7.33 (ddd, *J* = 7.2, 4.8, 1.3 Hz, 1H), 4.69 (s, 2H), 4.39 (s, 1H), 3.42), 2.15 - 1.12 (m, 6H).

### 1.22 Synthesis of compound 2669

### Synthesis of Boc protected tert-butyl N-[(1S,3S)-3-{[8-bromo-4-({[4-(pyridin-2-yl)phenyl]methyl}amino)pyrazolo[1,5-a][1,3,5]triazin-2-yl]amino}cyclopentyl]carbamate

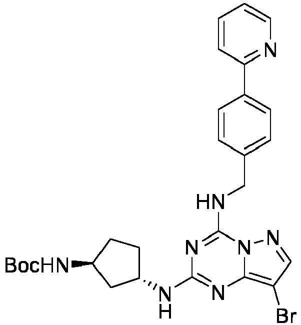

Prepared from tert-butyl N-[(1S,3S)-3-aminocyclopentyl]carbamate [CAS n° 645400-44-8] and the described above sulfoxide 8-bromo-2-(methylsulfinyl)-N-(4-(pyridin-2-yl)benzyl)pyrazolo[1 ,5-*a*][1,3,5]triazin-4-amine (0.071 g, 0.160 mmol) using the same protocol as described for intermediate IV. Boc protected compound was obtained as a white solid (foam) (0.075 g, 81% yield). MS m/z (ESI+): Calc for C₂₇H₃₁BrN₈O₂ [M+H]+ = 579.18 ; Exp = 579.3. HPLC Method 2, retention time = 5.70 min.

### Synthesis of compound 2669

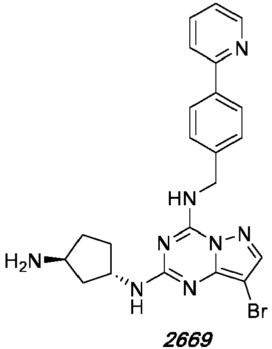

The Boc protected compound (0.0715 g, 0.123 mmol) was treated with TFA as described above in the synthesis procedure of compound 2388. Compound 2669 was obtained as a white solid (0.045 g, 79% yield). MS m/z (ESI+): Calc for C₂₂H₂₃BrN₈ [M+H]+ = 479.13; Exp = 479.2. HPLC Method 2, retention time = 3.61 min. ¹H NMR (300 MHz, DMSO) δ 8.64 (ddd, *J* = 4.8, 1.8, 0.9 Hz, 1H), 8.04 (d, *J* = 8.3 Hz, 2H), 7.96 - 7.89 (m, 2H), 7.86 (ddd, *J* = 8.0, 7.3, 1.8 Hz, 1H), 7.55 - 7.38 (m, 2H), 7.33 (ddd, *J* = 7.2, 4.8, 1.3 Hz, 1H), 4.69 (s, 2H), 4.39 (s, 1H), 3.42 - 3.31 (m, 1H), 2.15 - 1.12 (m, 6H).

### 1.23 Synthesis of compound 2670

### Synthesis of Boc protected tert-butyl N-[(1S,3R)-3-{[8-bromo-4-({[4-(pyridin-2-yl)phenyl]methyl}amino)pyrazolo[1,5-a][1,3,5]triazin-2-yl]amino}cyclohexyl]carbamate

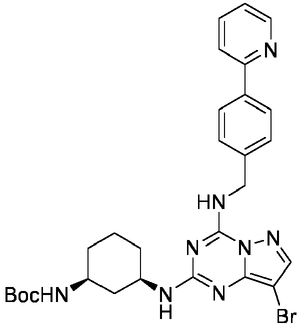

Prepared from tert-butyl N-[(1S,3R)-3-aminocyclohexyl]carbamate [CAS n° 1298101-47-9] and the described above sulfoxide 8-bromo-2-(methylsulfinyl)-N-(4-(pyridin-2-yl)benzyl)pyrazolo[1 ,5-*a*][1,3,5]triazin-4-amine (0.068 g, 0.153 mmol) using the same protocol as described for intermediate IV. Boc protected compound was obtained as a white solid (foam) (0.080 g, 88% yield). MS m/z (ESI+): Calc for C₂₈H₃₃BrN₈O₂ [M+H]+ = 593.20 ; Exp = 593.3. HPLC Method 2, retention time = 5.92 min.

### Synthesis of compound 2670

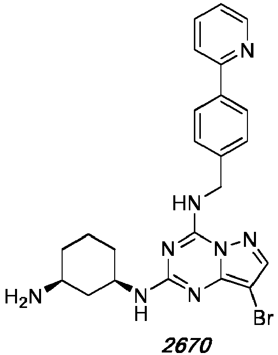

The Boc protected compound (0.0765 g, 0.129 mmol) was treated with TFA as described above in the synthesis procedure of compound 2388. Compound 2670 was obtained as a white solid (0.038 g, 60% yield). MS m/z (ESI+): Calc for C₂₃H₂₅BrN₈ [M+H]+ = 493.15; Exp = 493.2. HPLC Method 2, retention time = 3.81 min. ¹H NMR (300 MHz, DMSO) δ 8.64 (dt, *J* = 4.5, 1.5 Hz, 1H), 8.04 (d, *J* = 8.3 Hz, 2H), 7.96 - 7.89 (m, 2H), 7.88 - 7.80 (m, 1H), 7.53 - 7.43 (m, 2H), 7.33 (ddd, *J* = 7.3, 4.8, 1.3 Hz, 1H), 4.76 - 4.57 (m, 2H), 3.87 - 3.60 (m, 1H), 2.69 - 2.55 (m, 1H), 2.02 - 1.56 (m, 4H), 1.35 - 0.78 (m, 4H).

### 1.24 Synthesis of compound 2671

### Synthesis of Boc protected intermediate compound tert-butyl N-[(1S,2S)-2-{[8-bromo-4-({[4-(pyridin-2-yl)phenyl]methyl}amino)pyrazolo[1,5-a][1,3,5]triazin-2-yl]amino)cyclohexyl]carbamate

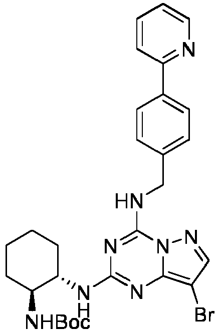

Prepared from tert-butyl N-[(1S,2S)-2-aminocyclohexyl]carbamate [CAS n° 180683-64-1] and the described above sulfoxide 8-bromo-2-(methylsulfinyl)-N-(4-(pyridin-2-yl)benzyl)pyrazolo[1,5-*a*][1,3,5]triazin-4-amine (0.071 g, 0.160 mmol) using the same protocol as described for intermediate IV. Boc protected compound was obtained as a white solid (foam) (0.091 g, 96% yield). MS m/z (ESI+): Calc for CzaHssBrNaOz [M+H]+ = 593.20 ; Exp = 593.3. HPLC Method 2, retention time = 6.22 min.

### Synthesis of compound 2671

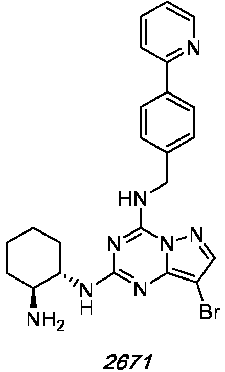

The Boc protected compound (0.089 g, 0.150 mmol) was treated with TFA as described above in the synthesis procedure of compound 2388. Compound 2671 was obtained as a white solid (0.064 g, 86% yield). MS m/z (ESI+): Calc for C₂₃H₂₅BrN₈ [M+H]+ = 493.15; Exp = 493.2. HPLC Method 2, retention time = 4.17 min¹H NMR (300 MHz, DMSO) δ 8.64 (ddd, *J* = 4.8, 1.8, 0.9 Hz, 1H), 8.04 (d, *J =* 8.0 Hz, 2H), 7.98 - 7.90 (m, 2H), 7.89 - 7.82 (m, 1H), 7.52 - 7.46 (m, 2H), 7.33 (ddd, *J* = 7.2, 4.8, 1.3 Hz, 1H), 4.82 - 4.51 (m, 2H), 3.68 - 3.34 (m, 2H), 2.69 - 2.31 (m, 1H), 2.02 - 1.50 (m, 2H), 1.34 - 0.93 (m, 5H).

### 1.25 Synthesis of compound 2672

### Synthesis of Boc protected tert-butyl N-[(1S,2R)-2-{[8-bromo-4-({[4-(pyridin-2-yl)phenyl]methyl}amino)pyrazolo[1,5-a][1,3,5]triazin-2-yl]amino}cyclohexyl]carbamate

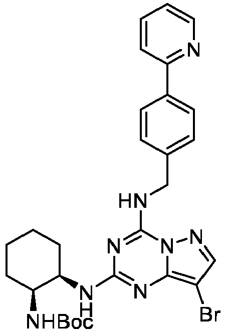

Prepared from tert-butyl N-[(1S,2R)-2-aminocyclohexyl]carbamate [CAS n° 365996-30-1] and the described above sulfoxide 8-bromo-2-(methylsulfinyl)-N-(4-(pyridin-2-yl)benzyl)pyrazolo[1,5-*a*][1,3,5]triazin-4-amine (0.070 g, 0.158 mmol) using the same protocol as described for intermediate IV. Boc protected compound was obtained as a white solid (foam) (0.091 g, 97% yield). MS m/z (ESI+): Calc for CzaHssBrNaOz [M+H]+ = 593.20 ; Exp = 593.3. HPLC Method 2, retention time = 6.08 min.

### Synthesis of compound 2672

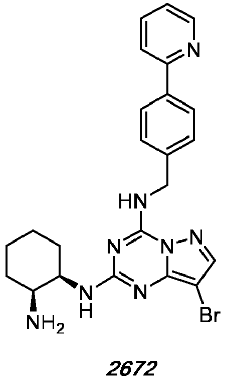

The Boc protected compound (0.088 g, 0.148 mmol) was treated with TFA as described above in the synthesis procedure of compound 2388. Compound 2672 was obtained as a white solid (0.064 g, 73% yield). MS m/z (ESI+): Calc for C₂₈H₃₃BrN₈ [M+H]+ = 493.15; Exp = 493.2. HPLC Method 2, retention time = 4.21 min. ¹H NMR (300 MHz, DMSO) δ 8.65 (ddd, *J* = 4.8, 1.8, 1.0 Hz, 1H), 8.05 (d, *J* = 8.3 Hz, 2H), 7.99 (s, 1H), 7.93 (dt, *J* = 8.1, 1.2 Hz, 1H), 7.86 (ddd, *J* = 8.0, 7.2, 1.8 Hz, 1H), 7.48 (d, *J* = 8.0 Hz, 2H), 7.34 (ddd, *J* = 7.2, 4.8, 1.3 Hz, 1H), 4.72 (m, 2H), 4.17 - 3.93 (m, 1H), 1.83 - 1.22 (m, 9H).

### 1.26 Synthesis of compound 2636

### Synthesis of Boc protected tert-butyl N-{[trans4-{[8-bromo-4-({[4-(pyridin-2-yl)phenyl]methyl}amino)pyrazolo[1,5-a][1,3,5]triazin-2-yl]amino}cyclohexyl]methyl}carbamate

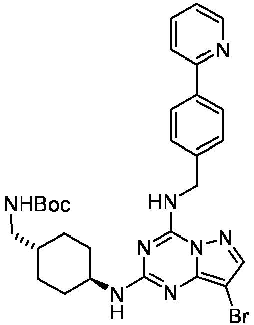

Prepared from *tert*-butyl *N*-[(trans-4-aminocyclohexyl)methyl]carbamate [CAS n° 192323-07-2] and the described above sulfoxide 8-bromo-2-(methylsulfinyl)-N-(4-(pyridin-2-yl)benzyl)pyrazolo[1 ,5-*a*][1,3,5]triazin-4-amine (0.080 g, 0.18 mmol) using the same protocol as described for intermediate IV. Boc protected compound was obtained as a white solid (foam) (0.083 g, 76% yield). MS m/z (ESI+): Calc for C₂₉H₃₅BrN₈O₂ [M+H]+ = 607.21 ; Exp = 607.2. HPLC Method 2, retention time = 5.96 min.

### Synthesis of compound 2636

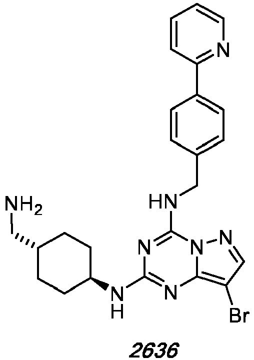

The Boc protected compound (0.081 g, 0.133 mmol) was treated with TFA as described above in the synthesis procedure of compound 2388. Compound 2636 was obtained as a white solid (0.041 g, 61 % yield). MS m/z (ESI+): Calc for C₂₄H₂₇BrN₈ [M+H]+ = 507.16; Exp = 507.2. HPLC Method 2, retention time = 3.81 min. ¹H NMR (300 MHz, DMSO) δ 8.62 (ddd, *J* = 4.8, 1.8, 1.0 Hz, 1H), 8.01 (d, *J* = 7.9 Hz, 2H), 7.94 - 7.89 (m, 2H), 7.85 (ddd, *J* = 8.0, 7.1, 1.8 Hz, 1H), 7.52 - 7.41 (m, 2H), 7.33 (ddd, *J* = 7.2, 4.8, 1.4 Hz, 1H), 4.74 - 4.56 (m, 2H), 3.77 - 3.56 (m, 1H), 2.36 (d, *J* = 6.3 Hz, 2H), 1.97 - 1.64 (m, 4H), 1.32 - 0.80 (m, 5H).

### 1.27 Synthesis of compound 2635

### Synthesis of Boc protected tert-butyl N-[trans-4-({[8-bromo-4-({[4-(pyridin-2-yl)phenyl]methyl}amino)pyrazolo[1,5-a][1,3,5]triazin-2-yl]amino}methyl)cyclohexyl]carbamate

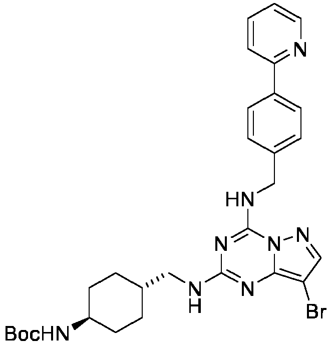

Prepared from tert-butyl N-[*trans*-4-(aminomethyl)cyclohexyl]carbamate [CAS n° 177583-27-6] and the described above sulfoxide 8-bromo-2-(methylsulfinyl)-N-(4-(pyridin-2-yl)benzyl)pyrazolo[1 ,5-a][1 ,3,5]triazin-4-amine (0.081 g, 0.183 mmol) using the same protocol as described for intermediate IV. Boc protected compound was obtained as a white solid (foam) (0.085 g, 77% yield). MS m/z (ESI+): Calc for C₂₉H₃₅BrN₈O₂ [M+H]+ = 607.21 ; Exp = 607.2. HPLC Method 2, retention time = 5.97 min.

### Synthesis of compound 2635

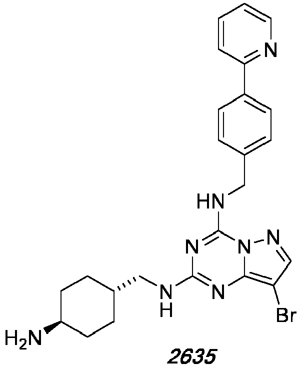

The Boc protected compound (0.0825 g, 0.136 mmol) was treated with TFA as described above in the synthesis procedure of compound 2388. Compound D27 was obtained as a white solid (0.020 g, 29% yield). MS m/z (ESI+): Calc for C₂₈H₃₃BrN₈ [M+H]+ = 507.16; Exp = 507.2. HPLC Method 2, retention time = 3.75 min. ¹H NMR (300 MHz, DMSO) δ 8.66 (ddd, *J* = 4.8, 1.8, 1.0 Hz, 1H), 8.05 (d, *J* = 8.4 Hz, 2H), 7.97 - 7.91 (m, 2H), 7.87 (ddd, *J* = 8.0, 7.2, 1.8 Hz, 1H), 7.52 - 7.42 (m, 2H), 7.37 - 7.31 (m, 1H), 4.69 (d, *J* = 7.2 Hz, 2H), 3.16 -3.01 (m, 2H), 1.83 -0.67 (m, 10H).

### 1.28 Synthesis of compound 2590

The iododerivative 2590 was prepared as described above in the synthesis of compounds 2384 and 2388)

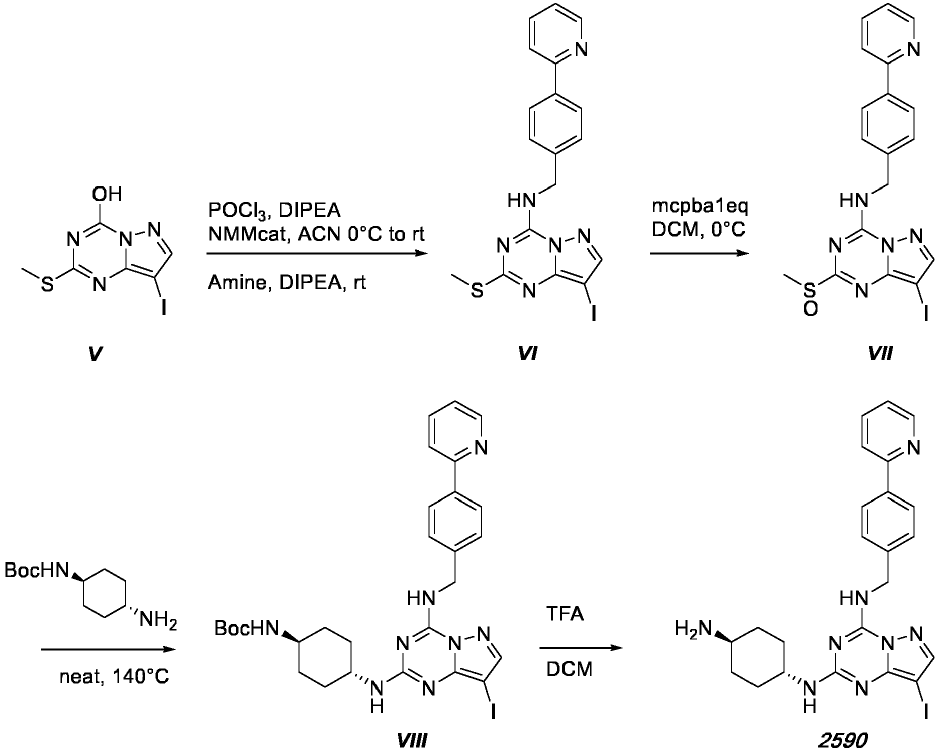

### Synthesis of compound V

Compound V (8-lodo-2-(methylthio)pyrazolo[1,5-*a*]-1,3,5-triazin-4(3*H*)-one [CAS n°147916-85-6]) was prepared by standard iodination protocol of 2-(Methylthio)pyrazolo[1,5-*a*]-1,3,5-triazin-4(3*H*)-one [CAS n°54346-18-8], as described above for bromoderivative compound I (N-iodosuccinimide was used instead of N-bromosuccinimide).

### Synthesis compound VI

Prepared from 8-lodo-2-(methylthio)pyrazolo[1,5-*a*]-1,3,5-triazin-4(3*H*)-one [CAS n°147916-85-6] (compound V) (0.200 g, 0.649 mmol) as described above for compound II. Compound *VI* (0.196 g, 64% yield) was obtained as a white powder. MS m/z (ESI+): Calc for C₁₈H₁₅IN₆S [M+H]+ = 475.02 ; Exp = 475.0. HPLC Method 2, retention time = 6.08 min. ¹H NMR (300 MHz, DMSO) δ 9.61 (t, *J* = 6.3 Hz, 1H), 8.65 (ddd, *J* = 4.8, 1.8, 1.0 Hz, 1H), 8.18 (s, 1H), 8.05 (d, *J=* 8.4 Hz, 2H), 7.93 (dt, *J* = 8.0, 1.2 Hz, 1H), 7.86 (td, *J* = 7.7, 1.8 Hz, 1H), 7.48 (d, *J* = 8.4 Hz, 2H), 7.34 (ddd, *J* = 7.3, 4.8, 1.3 Hz, 1H), 4.74 (d, *J* = 6.2 Hz, 2H), 2.5 (s, 3H, under the solvent peak).

### Synthesis of compound VII

Prepared as described above for compound III by mcpba oxidation of previous 8-lodo-2-(methylthio)-N-(4-(pyridin-2-yl)benzyl)pyrazolo[1,5-*a*][1,3,5]triazin-4-amine (compound VI) (0.193 g, 0.407 mmol). Compound *VII* (0.199 g, quant. yield) was obtained as a white powder). MS m/z (ESI+): Calc for C1₈H₁₅IN₆OS [M+H]+ = 491.01 ; Exp = 491.0. HPLC Method 2, retention time = 4.00 min. ¹H NMR (300 MHz, DMSO) δ 10.04 (s, 1H), 8.64 (ddd, *J* = 4.8, 1.8, 1.0 Hz, 1H), 8.37 (s, 1H), 8.04 (d, *J* = 8.4 Hz, 2H), 7.93 (dt, *J* = 8.1, 1.2 Hz, 1H), 7.86 (ddd, *J* = 8.0, 7.3, 1.8 Hz, 1H), 7.51 (d, *J* = 8.4 Hz, 2H), 7.33 (ddd, *J* = 7.3, 4.8, 1.3 Hz, 1H), 4.79 (s, 2H), 2.83 (s, 3H).

### Compound VIII

Prepared from N-Boc-trans-1,4-Cyclohexanediamine [CAS n° 195314-59-1] and the described above sulfoxide 8-lodo-2-(methylsulfinyl)-N-(4-(pyridin-2-yl)benzyl)pyrazolo[1,5-*a*][1,3,5]triazin-4-amine (compound VII) (0.065 g, 0.133 mmol) using the same protocol as previously described for intermediate IV. Compound VIII was obtained as a white solid (0.054 g, 64% yield). MS m/z (ESI+): Calc for C₂₈H₃₃IN₈O₂ [M+H]+ = 641.18 ; Exp = 641.2. HPLC Method 2, retention time = 5.93 min. ¹H NMR (300 MHz, DMSO) δ 9.16, 8.87 (2 broad s, 1H, exch NH), 8.64 (ddd, *J* = 4.8, 1.8, 1.0 Hz, 1H), 8.04 (d, *J* = 7.9 Hz, 2H), 7.95 - 7.81 (m, 3H), 7.52 - 7.41 (m, 2H), 7.33 (ddd, *J* = 7.2, 4.8, 1.3 Hz, 1H), 7.25 (broad d, *J* = 7.8 Hz, 0.5 H, exch NH), 7.08 (broad d, *J* = 8.1 Hz, 0.5 H, exch NH), 6.73 (s, 1H), 4.74 - 4.55 (m, 2H), 3.76 - 3.52 (m, 1H), 3.25 -3.06 (m, 1H), 1.76 (s, 4H), 1.38 (s, 9H), 1.35 - 1.10 (m, 4H).

### Synthesis of compound 2590

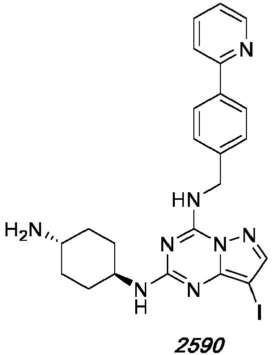

Prepared by TFA deprotection of previous Boc amino compound VIII (0.051 g, 0.080 mmol) as described above for compound 2388. Compound 2590 was obtained as a grey solid (0.028 g, 65% yield). MS m/z (ESI+): Calc for C₂₃H₂₅IN₈ [M+H]+ = 541.13 ; Exp = 541.1. HPLC Method 2, retention time = 3.77 min¹H NMR (300 MHz, DMSO) δ 8.64 (ddd, *J* = 4.8, 1.8, 1.0 Hz, 1H), 8.03 (d, *J* = 8.2 Hz, 2H), 7.95 - 7.89 (m, 1H), 7.89 - 7.82 (m, 2H), 7.54 - 7.42 (m, 2H), 7.33 (ddd, *J* = 7.2, 4.8, 1.4 Hz, 1H), 7.23 (d, *J* = 7.9 Hz, 0.5H exch NH), 7.04 (d, *J* = 8.2 Hz, 0.5H, exch NH), 4.74 - 4.58 (m, 2H), 3.78 - 3.55 (m, 1H), 2.50 - 2.40 (m, 1H), 1.92 - 1.03 (m, 8H).

### 1.29 Synthesis of compound 2629

The chloroderivative 2629 was prepared as described above in the synthesis of compounds 2384 and 2388)

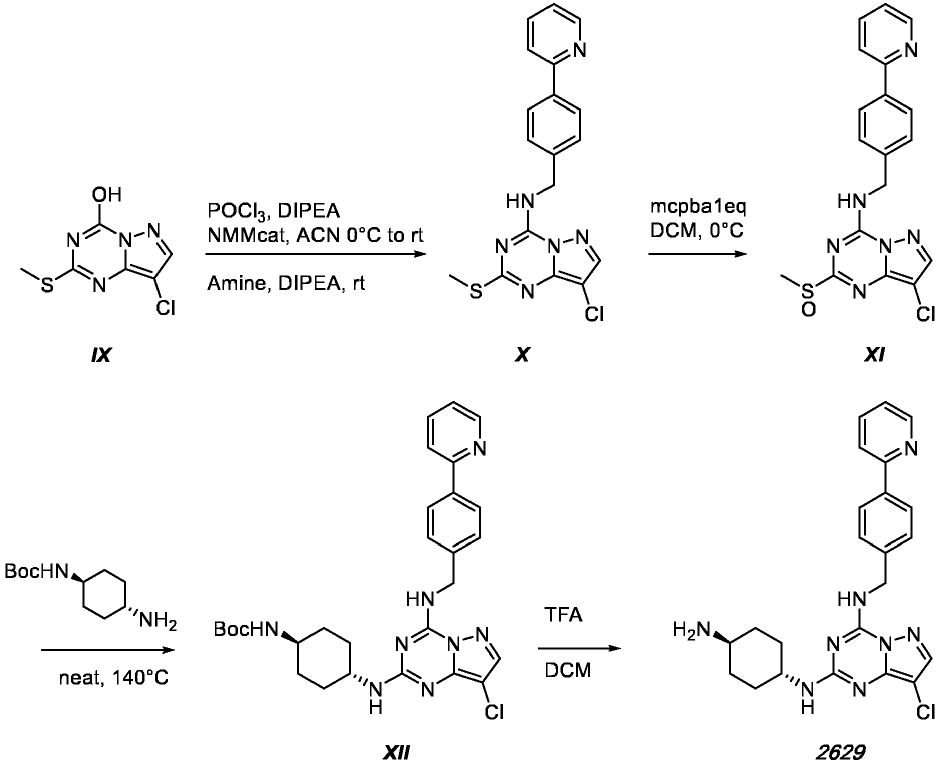

### Synthesis compound IX

Compound IX (8-Chloro-2-(methylthio)pyrazolo[1 ,5-*a*]-1,3,5-triazin-4(3*H*)-one [CAS n°1843260-73-0]) was prepared by standard chlorination protocol of 2-(Methylthio)pyrazolo[1 ,5-*a*]-1,3,5-triazin-4(3*H*)-one [CAS n°54346-18-8] using N-chlorosuccinimide instead of N-bromosuccinimide.

### Synthesis of compound X

Prepared from 8-chloro-2-(methylthio)pyrazolo[1,5-*a*]-1,3,5-triazin-4(3*H*)-one [CAS n°1843260-73-0] (compound IX) (0.250 g, 1.154 mmol) as described above for compound II. Compound X (0.407 g, 92% yield) was obtained as a beige powder. MS m/z (ESI+): Calc for C₁₈H₁₅ClN₆S [M+H]+ = 383.08 ; Exp = 383.1. HPLC Method 2, retention time = 5.68 min. ¹H NMR (300 MHz, DMSO) δ 9.68 (t, *J* = 6.3 Hz, 1H), 8.65 (ddd, *J* = 4.8, 1.8, 0.9 Hz, 1H), 8.26 (s, 1H), 8.05 (d, *J* = 8.3 Hz, 2H), 7.94 (dt, *J* = 8.0, 1.2 Hz, 1H), 7.87 (ddd, *J* = 8.0, 7.3, 1.8 Hz, 1H), 7.48 (d, *J* = 8.4 Hz, 2H), 7.34 (ddd, *J* = 7.3, 4.8, 1.3 Hz, 1H), 4.74 (d, *J* = 6.3 Hz, 2H), 2.49 (s, 3H).

### Synthesis of compound XI

Prepared as described above for compound II by mcpba oxidation of previous 8-chloro-2-(methylthio)-N-(4-(pyridin-2-yl)benzyl)pyrazolo[1,5-*a*][1,3,5]triazin-4-amine (compound X) (0.353 g, 0.922 mmol). Compound XI (quant. yield) was obtained as a white powder). MS m/z (ESI+): Calc for C₁₈H₁₅ClN₆OS [M+H]+ = 399.08 ; Exp = 399.1. HPLC Method 2, retention time = 3.79 min. ¹H NMR (300 MHz, DMSO) δ 10.11 (broad s, 1H), 8.64 (ddd, *J* = 4.8, 1.8, 0.9 Hz, 1H), 8.35 (s, 1H), 8.04 (d, *J* = 8.3 Hz, 2H), 7.96 - 7.91 (m, 1H), 7.86 (ddd, J = 8.0, 7.3, 1.8 Hz, 1H), 7.51 (d, *J* = 8.4 Hz, 2H), 7.33 (ddd, *J* = 7.3, 4.8, 1.3 Hz, 1H), 4.77 (s, 2H), 2.82 (s, 3H).

### Synthesis of compound XII

Prepared from N-Boc-trans-1,4-Cyclohexanediamine [CAS n° 195314-59-1] and the described above sulfoxide 8-chloro-2-(methylsulfinyl)-N-(4-(pyridin-2-yl)benzyl)pyrazolo[1,5-a][1,3,5]triazin-4-amine (compound XI) (0.100 g, 0.251 mmol) using the same protocol as described above for intermediate IV. Compound XII was obtained as a white solid (0.107 g, 78% yield). MS m/z (ESI+): Calc for C₂₈H₃₃ClN₈O₂ [M+H]+ = 549.25 ; Exp = 549.2. HPLC Method 2, retention time = 5.76 min. ¹H NMR (300 MHz, DMSO) δ 8.66 (ddd, *J* = 4.8, 1.8, 1.0 Hz, 1H), 8.09 - 8.02 (m, 2H), 7.98 - 7.91 (m, 2H), 7.87 (td, *J* = 7.6, 1.8 Hz, 1H), 7.54 - 7.42 (m, 2H), 7.34 (ddd, *J* = 7.2, 4.8, 1.4 Hz, 1H), 6.81 - 6.70 (m, 1H), 4.77 - 4.58 (m, 2H), 3.77 - 3.52 (m, 1H), 3.25 - 3.07 (m, 1H), 1.96 - 1.64 (m, 4H), 1.39 (s, 9H), 1.35 - 1.07 (m, 4H).

### Synthesis of compound 2629

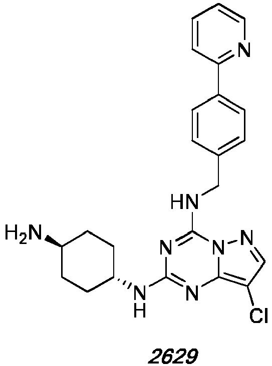

Prepared by TFA deprotection of previous Boc amino compound XII (0.103 g, 0.188 mmol) as described for compound IV. Compound 2629 was obtained as a grey solid (0.082 g, 97% yield). MS m/z (ESI+): Calc for C₂₀H₂₁BrN₈ [M+H]+ = 449.20 ; Exp = 449.2. HPLC Method 2, retention time = 3.59 min. ¹H NMR (300 MHz, DMSO) δ 8.65 (ddd, *J* = 4.8, 1.8, 1.0 Hz, 1H), 8.04 (d, *J* = 8.2 Hz, 2H), 7.96 - 7.90 (m, 2H), 7.86 (ddd, *J* = 8.0, 7.2, 1.8 Hz, 1H), 7.54 - 7.43 (m, 2H), 7.33 (ddd, *J* = 7.2, 4.8, 1.3 Hz, 1H), 4.71 - 4.61 (m, 2H), 3.78 - 3.54 (m, 1H), 2.50 - 2.38 (m, 1H), 1.93 - 0.99 (m, 8H).

### 1.30 Synthesis of compound 2420

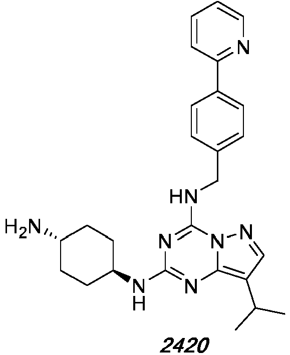

Compound 2420 [CAS n° 2254007-13-9], N2-((1r,4r)-4-aminocyclohexyl)-8-isopropyl-N4-(4-(pyridin-2-yl)benzyl)pyrazolo[1,5-a][1,3,5]triazine-2,4-diamine was obtained as described by G. Compain et al, Eur. J. Med. Chem, 2018, 208, 158, 1-6 (doi.org/10.1016/j.ejmech.2018.08.100).

### 1.31 Synthesis of compound 2428

### Synthesis of compound XIII

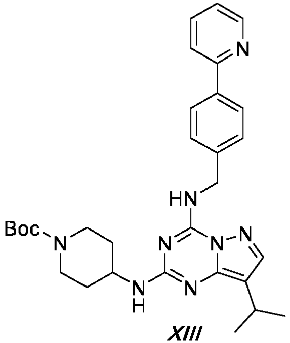

Prepared from tert-Butyl 4-aminopiperidine-1-carboxylate [CAS 87120-72-7] and the known sulfoxide 8-(1-Methylethyl)-2-(methylsulfinyl)-*N*-[[4-(2-pyridinyl)phenyl]methyl]pyrazolo[1,5-*a*]-1,3,5-triazin-4-amine [CAS n°1244955-43-8]; see G. Compain et al, Eur. J. Med. Chem, 2018, 208, 158, 1-6 (0.074 g, 0.136 mmol) using the same protocol as described above for intermediate IV. Compound XIII was obtained as a white solid. HPLC Method 2, retention time = 5.96 min. ¹H NMR (300 MHz, DMSO) δ 8.35 - 8.28 (m, 1H), 7.78 (d, *J* = 7.9 Hz, 2H), 7.73 (s, 1H), 7.64 - 7.54 (m, 1H), 7.49 - 7.42 (m, 2H), 7.41 - 7.36 (m, 2H), 4.67 (s, 2H), 4.00 - 3.71 (m, 3H), 3.04 - 2.69 (m, 3H), 1.96 - 1.55 (m, 2H), 1.50 - 1.11 (m, 17H).

### Synthesis of compound 2428

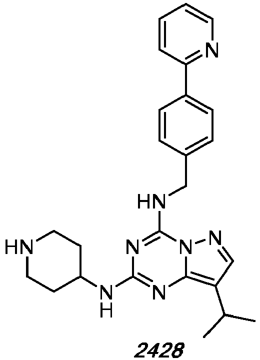

Prepared by TFA deprotection of previous Boc amino compound XIII, white solid. MS m/z (ESI+): Calc for C₂₅H₃₀N₈ [M+H]+ = 443.27 ; Exp = 443.3. HPLC Method 2, retention time = 4.08 min. ¹H NMR (300 MHz, DMSO) δ 8.65 (ddd, *J* = 4.8, 1.8, 1.0 Hz, 1H), 8.04 (d, *J* = 8.0 Hz, 2H), 7.93 (dt, *J* = 8.1, 1.2 Hz, 1H), 7.86 (td, *J* = 7.7, 1.8 Hz, 1H), 7.71 (s, 1H), 7.47 (d, *J* = 7.9 Hz, 2H), 7.34 (ddd, *J* = 7.2, 4.8, 1.4 Hz, 1H), 4.66 (broad s, 2H), 3.78 - 3.60 (m, 1H), 2.99 - 2.81 (m, 3H), 2.50 - 2.41 (m, 2H), 1.92 - 1.59 (m, 2H), 1.41 - 1.10 (m, 8H).

### 1.32 Synthesis of compound 2478

Prepared from known 2-(Methylthio)-4-oxo-3,4-dihydropyrazolo[1,5*-*a][1,3,5]triazine-8-carbonitrile (see G. Compain et al, Eur. J. Med. Chem, 2018, 208, 158, 1-6 [CAS n°1273577-70-0]).

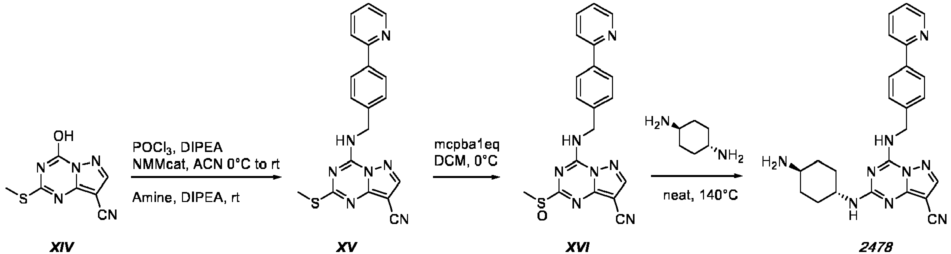

### Synthesis of compound XIV

Prepared from 2-(Methylthio)-4-oxo-3,4-dihydropyrazolo[1,5*-*a][1,3,5]triazine-8-carbonitrile [CAS n°1273577-70-0] (compound XIV) (0.195 g, 0.941 mmol) as described above for compound II. Compound XV (0.308 g, 88% yield) was obtained as a foam. MS m/z (ESI+): Calc for C₁₉H₁₅N₇S [M+H]+ = 374.12 ; Exp = 374.1. HPLC Method 2, retention time = 5.00 min. ¹H NMR (300 MHz, DMSO) δ 9.93 (t, *J* = 6.0 Hz, 1H), 8.65 (d, *J* = 4.0 Hz, 1H), 8.60 (s, 1H), 8.05 (d, *J* = 7.9 Hz, 2H), 7.94 (d, *J* = 7.6 Hz, 1H), 7.87 (t, *J* = 7.7 Hz, 1H), 7.49 (s, 2H), 7.39 - 7.30 (m, 1H), 4.76 (d, *J* = 6.1 Hz, 2H), 2.50 (CH₃S under the solvent signal, , 3H).

### Synthesis of compound XVI

Prepared as described above for compound III by mcpba oxidation of previous methylsulfinyl compound XV (0.300 g, 0.803 mmol). Compound XI (quant. yield) was obtained as a white foam). MS m/z (ESI+): Calc for C₁₉H₁₅N₇OS [M+H]+ = 390.11 ; Exp = 390.1. HPLC Method 2, retention time = 3.39 min.

### Synthesis of compound 2478

Prepared from previous sulfoxide compound XVI (0.101 g, 0.259 mmol) as described above for compound 2384. Compound 2478 was obtained as foam (0.059 g, 52% yield). MS m/z (ESI+): Calc for C₂₄H₂₅N₉ [M+H]+ = 440.23 ; Exp = 440.2. HPLC Method 2, retention time = 3.63 min. ¹H NMR (300 MHz, DMSO) δ 8.65 (broad d, *J* = 4.2 Hz, 1H), 8.29 (d, *J* = 6.4 Hz, 1H), 8.04 (d, *J* = 7.7 Hz, 2H), 7.96 - 7.83 (m, 2H), 7.53 - 7.43 (m, 2H), 7.36 - 7.30 (m, 1H), 4.71 - 4.61 (m, 2H), 3.81 - 3.53 (m, 1H), 2.50 under solvent peak (m, 1H), 1.88 - 1.64 (m, 4H), 1.36 - 0.99 (m, 4H).

### 1.33 Synthesis of compound 2533

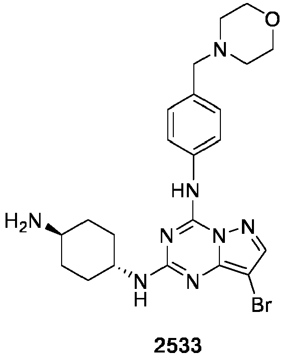

Prepared following the procedures described above for compound 2384, using 4-Morpholin-4-ylmethyl-phenylamine (CAS# 51013-67-3) instead of 4-(Pyridin-2-yl)phenyl)methanamine. Compound 2533 was obtained as a white foam (0.056 g, 93% yield). MS m/z (ESI+): Calc for C₂₂H₂₉BrN₈O [M+H]+ = 501.17 ; Exp = 501.2. HPLC Method 2, retention time = 3.75 min. ¹H NMR (300 MHz, DMSO) δ 8.01 (s, 1H), 7.87 - 7.76 (m, 2H), 7.32 - 7.23 (m, 2H), 3.57 (t, J = 4.6 Hz, 5H), 3.45 (m, 2H), 3.37 - 3.29 (m, 1H), 2.40 - 2.30 (m, 4H), 1.95 - 1.71 (m, 4H), 1.40 - 1.01 (m, 4H).

### 1.34 Synthesis of compound 2554

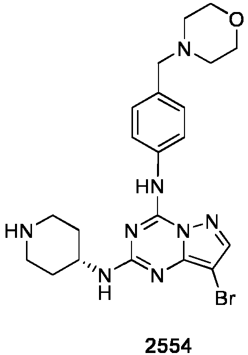

Prepared following the procedures described above for compound 2388, using 4-Morpholin-4-ylmethyl-phenylamine (CAS# 51013-67-3) instead of 4-(Pyridin-2-yl)phenyl)methanamine. Compound 2554 was obtained as a beige solid. MS m/z (ESI+): Calc for C₂₁H₂₇BrN₈O [M+H]+ = 487.16 ; Exp = 487.1. HPLC Method 2, retention time = 3.77 min. ¹H NMR (300 MHz, DMSO) δ 8.02 (s, 1H), 7.87 - 7.78 (m, 2H), 7.32 - 7.24 (m, 2H), 3.91 - 3.60 (m, 1H), 3.59 - 3.54 (m, 4H), 3.48 - 3.42 (m, 2H), 3.01 - 2.91 (m, 2H), 2.39 - 2.30 (m, 4H), 1.86 - 1.76 (m, 2H), 1.45 - 1.11 (m, 4H).

### Example 2: General cell culture practices and animal experiments

Human cancer cell lines used in the present project were purchased from either the American Type Culture Collection (ATCC), The Leibniz Institute DSMZ German Collection of Microorganisms and Cell Cultures GmbH (DSMZ) or the European Collection of Authenticated Cell Cultures (ECACC). For use in experiments, cells were cultivated following good and established cell culture practices, following instructions from the original ATCC/DSMZ/ECACC supplier for cell culture media & supplement, cryopreservation, and subculturing procedures. Cells were incubated under a 5% CO₂ atmosphere at 37°C for no longer than 2 months.

All animal procedures were performed in accordance with the European Union directive 86/609/EEC. Experiments were performed under individual permit and in accredited animal care facilities.

### Example 3: In vitro cytotoxicity assay of 2-substituted 8-halogeno-N4-(4-(pyridin-2-yl) benzyl) pyrazolo[1,5-a][1,3,5] triazine-2,4-diamine family of compounds

In vitro cytotoxicity of compounds of the disclosure was assessed on several cancerous human cell lines. Cells were plated in 96-well plates at an appropriate density depending on the cell line (between 1000 and 10 000 cells/well in 100µL of appropriate culture media) and incubated at 37°C for 24 hours. Serial dilutions of the tested compound previously dissolved in culture media (50µL) were added, and incubation was carried at 37°C out for 6 days. MTT (5mg/mL in PBS, 20*µ*L, Sigma-Aldrich) was added into the wells, and incubation was continued for 1 to 4 hours at 37°C. Culture media was then carefully removed, and well content was homogeneously dissolved with acidified isopropanol. Absorbance values were measured on a microplate reader using a wavelength of 570 nm (with a reference wavelength of 690 nm). The IC₅₀ concentration values compared to untreated control cells were determined using inhibition dose response curve fitting (GraphPad Prism 9). Exatecan mesylate (cat#HY-13631A) and CR8 (CAS# 294646-77-8; cat#HY-18340) were purchased from MedChemExpress (China).

Table 1 below shows the observed in vitro cytotoxicity IC₅₀ values of the compounds of the disclosure in a panel of human cancer cell lines. Low nanomolar efficacies were observed with compounds of the invention. Compared to the known CycK molecular glue degrader compound CR8 (see: Stabicki, M., Kozicka, Z., Petzold, G. et al. The CDK inhibitor CR8 acts as a molecular glue degrader that depletes cyclin K. Nature 585, 293-297, 2020), compounds of the invention are 2-100 fold more potent.

**Table 1: summary of in vitro cytotoxicity IC₅₀ values of compounds of the disclosure (in nanomolar)**

| | **Cell line** | | | | | | | | | | | | | |
|---|---|---|---|---|---|---|---|---|---|---|---|---|---|---|
| **Compound** | **BT-474** | **NCI-N87** | **MDA-MB-453** | **SKBR-3** | **MDA-MB-231** | **JIMT-1** | **MCF-7** | **HT-29** | **BXPC3** | **OE-19** | **A549** | **KB-1** | **HCT-116** | **Caco-2** |
| **2384** | 0.94 | 0.76 | 0.53 | 0.73 | 0.38 | 0.25 | 3.42 | 0.22 | 1.46 | 2.24 | 0.56 | 0.38 | 1.19 | 6.64 |
| **2388** | 1.64 | 1.40 | 1.27 | 1.68 | 0.76 | 0.64 | 5.40 | 0.23 | 1.97 | 3.77 | 0.65 | | 1.66 | 4.46 |
| **2420** | 3.41 | 4.93 | 2.73 | 3.25 | 0.70 | 0.61 | 7.78 | 1.74 | | 6.91 | 1.99 | | 4.40 | 56.15 |
| **2428** | 3.73 | 4.50 | 3.11 | 1.94 | 2.31 | 0.92 | 9.12 | 0.83 | | 7.84 | 2.22 | | 5.31 | 24.20 |
| **2478** | 1.72 | 6.88 | 1.34 | 17.59 | 1.64 | 1.18 | | | | | | | | |
| **2533** | >100 | >100 | >100 | >100 | >100 | >100 | | | | | | | | |
| **2538** | 6.54 | 21.59 | 8.12 | 39.15 | 8.75 | 5.13 | | | | | | | | |
| **2554** | >100 | >100 | >100 | >100 | >100 | >100 | | | | | | | | |
| **2559** | 2.63 | 1.32 | 1.52 | 3.73 | 1.35 | 0.41 | 12.85 | 0.21 | 1.63 | | | | | |
| **2560** | 5.92 | 3.29 | 1.61 | 4.33 | 1.92 | 0.60 | | | | | | | | |
| **2568** | 1.75 | 2.22 | 1.67 | 4.36 | 0.98 | 0.62 | 11.57 | 0.46 | 4.86 | | | 4.95 | | |
| **2569** | 15.22 | 19.70 | 12.81 | 62.61 | 11.24 | 6.70 | | | | | | | | |
| **2571** | 2.68 | 4.27 | 3.55 | 7.75 | 2.22 | 1.53 | 79.63 | 1.12 | 5.43 | | | | | |
| **2584** | 9.72 | 9.44 | 12.33 | 25.91 | 7.17 | 7.61 | | | | | | | | |
| **2585** | 1.77 | 15.41 | 26.75 | 38.89 | 24.72 | 7.86 | | | | | | | | |
| **2586** | 1.73 | 3.14 | 2.47 | 6.59 | 1.44 | 1.13 | 33.90 | 0.45 | 2.44 | | | | | |
| **2590** | 1.33 | 2.42 | 1.76 | 2.55 | 0.74 | 0.29 | | | | | | | | |
| **2600** | 1.18 | 1.32 | 1.17 | 1.69 | 0.29 | 0.19 | 12.19 | 0.28 | 1.16 | | | 1.48 | | |
| **2601** | 1.86 | 2.70 | 1.92 | 3.61 | 0.86 | 0.45 | 15.36 | 0.13 | 1.72 | | | | | |
| **2605** | 2.76 | 4.50 | 3.66 | 7.46 | 1.54 | 1.22 | 3.70 | 0.33 | 2.91 | | | | | |
| **2606** | 0.92 | 0.77 | 0.64 | 1.23 | 0.46 | 0.88 | 6.88 | 0.13 | 1.16 | | | | | |
| **2622** | 13.47 | 3.00 | 21.14 | 2.24 | 12.72 | 4.84 | >100 | 8.25 | 36.13 | | | | | |
| **2629** | 1.75 | 0.94 | 0.77 | 0.27 | 0.66 | 0.11 | 3.91 | 0.36 | 1.17 | | | | | |
| **2635** | 23.12 | 15.88 | 17.94 | 0.83 | 12.87 | 3.52 | >100 | 8.93 | 39.70 | | | | | |
| **2636** | 3.68 | 2.46 | 1.97 | 0.17 | 1.46 | 0.12 | 33.12 | 1.28 | 4.68 | | | 8.10 | | |
| **2659** | 0.72 | 0.34 | 0.40 | 0.72 | 0.26 | | | | | | | | | |
| **2660** | 1.25 | 0.49 | 0.68 | 0.11 | 0.43 | | | | | | | | | |
| **2638** | 0.78 | 0.42 | 0.88 | 0.82 | 0.30 | | | | | | | | | |
| **2658** | 4.57 | 6.43 | 7.84 | 0.77 | 1.98 | | | | | | | | | |
| **2655** | 1.57 | 1.95 | 0.97 | 0.14 | 0.54 | | | | | | | 1.66 | | |
| **2656** | 2.52 | 1.42 | 1.13 | 0.17 | 0.65 | | | | | | | | | |
| **2657** | 1.71 | 0.43 | 0.77 | 0.13 | 0.56 | | | | | | | | | |
| **2668** | 2.16 | 0.84 | 1.78 | 0.20 | 0.82 | | | | | | | 2.99 | | |
| **2669** | 1.75 | 0.39 | 0.59 | 0.67 | 0.44 | | | | | | | | | |
| **2670** | 5.77 | 2.53 | 9.19 | 0.72 | 2.67 | | | | | | | | | |
| **2671** | 3.34 | 1.47 | 2.22 | 0.42 | 1.74 | | | | | | | 7.60 | | |
| **2672** | 2.12 | 0.55 | 0.76 | 0.24 | 0.46 | | | | | | | | | |
| **CR8*** | 18.4 | 62.2 | 40.3 | 30.6 | 10.4 | 25.2 | | | | | | | | |

| | | | | | | | | | | | | | | |
|---|---|---|---|---|---|---|---|---|---|---|---|---|---|---|
| *CR8 is a known molecular glue degrader of CycK (see: S 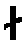 abicki, M., Kozicka, Z., Petzold, G. et al. The CDK inhibitor CR8 acts as a molecular glue degrader that depletes cyclin K. Nature 585, 293-297, 2020), but with limited efficacy (high nM IC₅₀ potency range) | | | | | | | | | | | | | | |

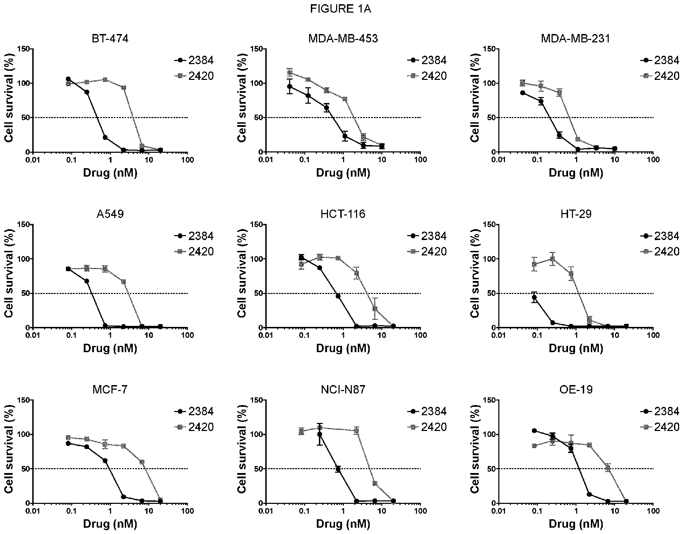

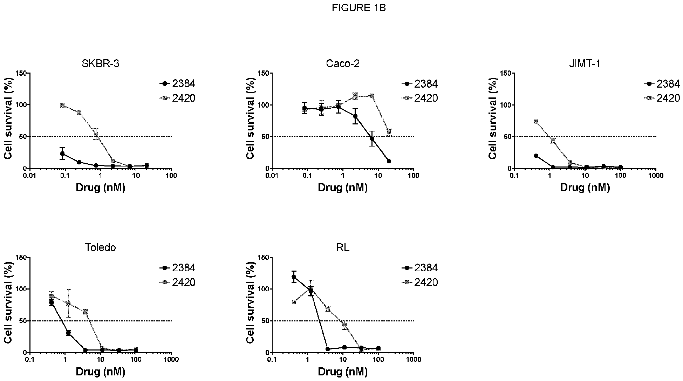

Figures 1A and 1B show side-by-side comparison of halogenated compound 2384 to its isopropyl counterpart 2420. The halogenated compound 2384 is systematically more potent compared to its isopropyl counterpart. This shows that inclusion of a halogen atom in this position increases compound efficacy.

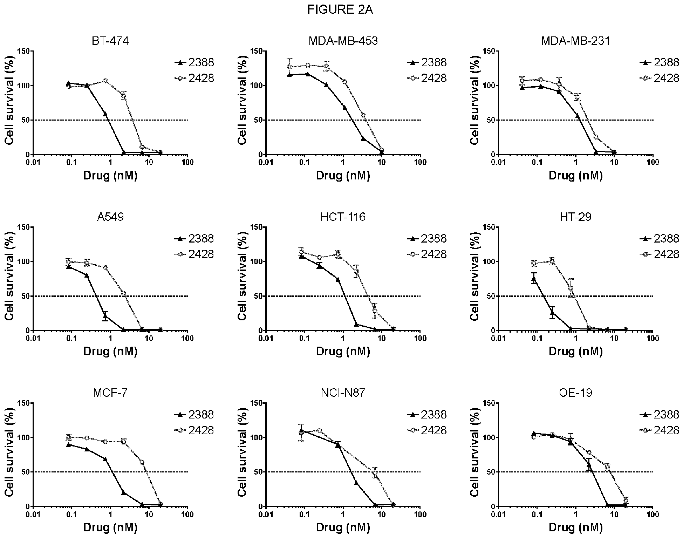

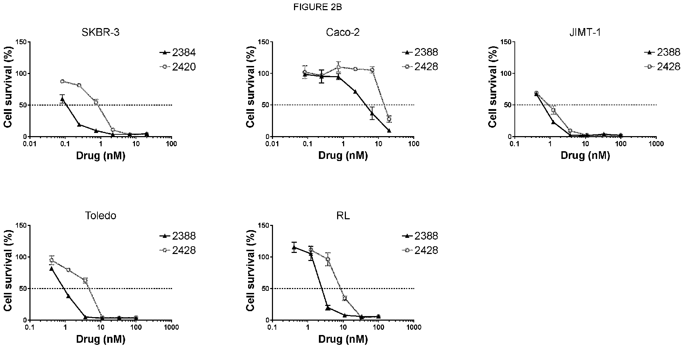

Figures 2A and 2B show side-by-side comparison of halogenated compound 2388 of the disclosure to its isopropyl counterpart 2428. The halogenated compound 2384 is systematically more potent compared to its isopropyl counterpart. This shows that inclusion of a halogen atom in this position increases compound efficacy.

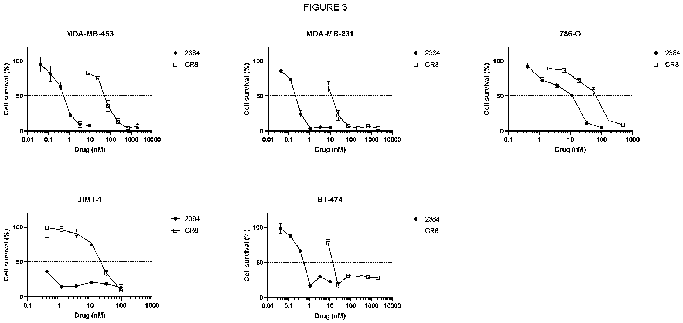

Figure 3 shows side-by-side comparison of compound 2384 of the disclosure to the known CycK molecular glue degrader compound CR8 (see: S 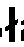 abicki, M., Kozicka, Z., Petzold, G. et al. The CDK inhibitor CR8 acts as a molecular glue degrader that depletes cyclin K. Nature 585, 293-297, 2020). The halogenated compound 2384 shows drastically improved low nanomolar potencies, whereas CR8 shows potencies in the high nanomolar range.

This example shows that the series of halogenated compounds of the invention are more potent than their isopropyl counterpart and than the known molecular glue degrader CR8.

### Example 4: In vivo mice tolerability assessment of 2-substituted 8-halogeno-N4-(4-(pyridin-2-yl) benzyl) pyrazolo[1,5-a][1,3,5] triazine-2,4-diamine family of compounds

Mouse tolerability experiments were performed with selected compounds of the disclosure. Female CD-1 mice (n=5 mice per group) were treated with a single intravenous 1mg/kg dose of compound. Vehicle was PBS pH 6.0. Mice were carefully monitored for mortality, external clinical findings, weight loss, behavior and apparent signs of toxicity over the course of 21 days.

Table 2 below shows the average weight change over time and animal deaths for each individual compound. Some compounds of the disclosure were comparatively better tolerated than others, potentially supporting an improved therapeutic index.

**Table 2: average weight change (% initial weight) over time and animal deaths of in vivo mice tolerability study**

| **Compound** | | **Study Day** | | | | | | | | | | | | | | | | | | | | |
|---|---|---|---|---|---|---|---|---|---|---|---|---|---|---|---|---|---|---|---|---|---|---|
| | | **1** | **2** | **3** | **4** | **5** | **6** | **7** | **8** | **9** | **10** | **11** | **12** | **13** | **14** | **15** | **16** | **17** | **18** | **19** | **20** | **21** |
| **2384** | **Mean** | 0.0 | **-2.5** | **-7.1** | **-14.6** | **-15.2** | **-14.9** | **-12.4** | **-9.2** | **-7.3** | **-1.9** | **-0.7** | **4.1** | **1.9** | **-1.8** | **-3.4** | **-4.1** | **-1.6** | **-0.5** | **0.7** | **2.6** | **2.0** |
| | SD | *0.0* | *2.3* | *6.3* | *9.2* | *9.4* | *11.8* | *13.1* | *11.5* | *11.2* | *12.6* | *17.2* | *15.4* | *6.6* | *10.0* | *9.6* | *8.5* | *9.0* | *8.4* | *8.1* | *7.7* | *9.5* |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2388** | **Mean** | **0.0** | **-7.1** | **-15.5** | **-21.9** | **-29.7** | **-33.8** | | | | | | | | | | | | | | | |
| | SD | *0.0* | *2.5* | *3.8* | *6.7* | *5.2* | *4.0* | | | | | | | | | | | | | | | |
| | N | 5 | 5 | 5 | 5 | 3 | 3 | | | | | | | | | | | | | | | |
| **2559** | **Mean** | 0.0 | 11.4 | 20.2 | 29.0 | 33.9 | 36.9 | 39.4 | | | | | | | | | | | | | | |
| | SD | *0.0* | *0.8* | *0.9* | *1.8* | *4.6* | *3.9* | *0.2* | | | | | | | | | | | | | | |
| | N | 5 | 5 | 5 | 5 | 4 | 4 | 2 | | | | | | | | | | | | | | |
| **2560** | **Mean** | **0.0** | **-2.1** | **1.4** | **3.2** | **4.2** | **4.4** | **4.1** | **6.7** | **7.9** | **8.3** | **7.9** | **9.7** | **10.0** | **10.5** | **7.7** | **9.4** | **10.8** | **12.5** | **12.3** | **12.2** | **13.6** |
| | SD | *0.0* | *3.1* | *2.6* | *1.6* | *2.3* | *2.1* | *1*.*5* | *1.8* | *2.4* | *4.2* | *5.6* | *6.2* | *6.2* | *4.0* | *2.4* | *3.6* | *3.8* | *3.1* | *3.0* | *3.1* | *3.8* |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2568** | Mean | 0.0 | 0.7 | -0.2 | 1.9 | 2.9 | 2.4 | 2.7 | 4.8 | 6.9 | 5.1 | 4.5 | 6.0 | 8.3 | 11.2 | 12.2 | 12.4 | 11.5 | 13.6 | 13.2 | 11.3 | 11.4 |
| | SD | *0.0* | 1.2 | 4.3 | *3.7* | *3.5* | *3.3* | *3.0* | *3.0* | *4.4* | *3.4* | *3.1* | *3.5* | *4.2* | *3.5* | *4.7* | *7.3* | *8.1* | 6.9 | *4.9* | *3.6* | *3.5* |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2571** | **Mean** | **0.0** | **1.7** | **1.6** | **2.4** | **4.5** | **3.8** | **3.0** | **5.9** | **9.1** | **9.1** | **10.6** | **12.5** | **13.0** | **13.2** | **14.1** | **15.2** | **15.9** | **13.6** | **13.7** | **15.2** | **16.0** |
| | SD | *0.0* | *2.6* | *3.2* | *3.5* | *2.4* | *1.9* | *3.0* | *4.8* | *3.1* | *3.0* | *2.8* | *2.2* | *2.3* | *2.8* | *3.4* | *3.4* | *2.7* | *1.6* | *0.9* | *1.8* | *2.2* |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2585** | **Mean** | **0.0** | **1.3** | **1.5** | **1.7** | **2.3** | **4.2** | **3.3** | **4.2** | **4.5** | **7.8** | **7.7** | **8.0** | **7.9** | **8.0** | **7.9** | **9.4** | **9.3** | **9.8** | **10.5** | **11.4** | **12.0** |
| | SD | *0.0* | *2.5* | *3.2* | *2.2* | *1.9* | *2.2* | *1.6* | *3.9* | 3.8 | *3.2* | *2.4* | *3.3* | *3.6* | *2.3* | *2.5* | *2.9* | *1.8* | *3.2* | 2.5 | *3.3* | *2.4* |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2586** | **Mean** | **0.0** | **-2.1** | **1.4** | **3.2** | **4.2** | **4.4** | **4.1** | **6.7** | **7.9** | **8.3** | **7.9** | **9.7** | **10.0** | **10.5** | **7.7** | **9.4** | **10.8** | **12.5** | **12.3** | **12.2** | **13.6** |
| | SD | *0.0* | *3.1* | 2.6 | *1.6* | *2.3* | 2.1 | 1.5 | *1.8* | *2.4* | 4.2 | 5.6 | *6.2* | *6.2* | *4.0* | *2.4* | *3.6* | 3.8 | *3.1* | *3.0* | *3.1* | 3.8 |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2600** | **Mean** | **0.0** | **0.7** | **-0.2** | **1.9** | **2.9** | **2.4** | **2.7** | **4.8** | **6.9** | **5.1** | **4.5** | **6.0** | **8.3** | **11.2** | **12.2** | **12.4** | **11.5** | **13.6** | **13.2** | **11.3** | **11.4** |
| | SD | *0.0* | *1.2* | *4.3* | 3.7 | *3.5* | *3.3* | *3.0* | *3.0* | *4.4* | *3.4* | *3.1* | *3.5* | *4.2* | *3.5* | *4.7* | *7.3* | *8.1* | 6.9 | *4.9* | *3.6* | *3.5* |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2605** | **Mean** | **0.0** | **1.7** | **1.6** | **2.4** | **4.5** | **3.8** | **3.0** | **5.9** | **9.1** | **9.1** | **10.6** | **12.5** | **13.0** | **13.2** | **14.1** | **15.2** | **15.9** | **13.6** | **13.7** | **15.2** | **16.0** |
| | SD | *0.0* | 2.6 | *3.2* | *3.5* | *2.4* | *1.9* | *3.0* | *4.8* | *3.1* | *3.0* | *2.8* | *2.2* | *2.3* | *2.8* | *3.4* | *3.4* | *2.7* | *1.6* | *0*.*9* | *1.8* | *2.2* |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2636** | **Mean** | **0.0** | **1.3** | **1.5** | **1.7** | **2.3** | **4.2** | **3.3** | **4.2** | **4.5** | **7.8** | **7.7** | **8.0** | **7.9** | **8.0** | **7.9** | **9.4** | **9.3** | **9.8** | **10.5** | **11.4** | **12.0** |
| | SD | *0.0* | 2.5 | *3.2* | *2.2* | *1.9* | *2.2* | *1.6* | *3.9* | 3.8 | *3.2* | *2.4* | *3.3* | *3.6* | *2.3* | *2.5* | *2.9* | *1.8* | *3.2* | 2.5 | *3.3* | *2.4* |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2655** | **Mean** | **0.0** | **-9.3** | **17.4** | **25.2** | **28.3** | **30.6** | **36.4** | | | | | | | | | | | | | | |
| | SD | *0.0* | *1.9* | *2.6* | 2.9 | *5.7* | *5.3* | *7.3* | | | | | | | | | | | | | | |
| | N | 5 | 5 | 5 | 5 | 4 | 4 | 3 | | | | | | | | | | | | | | |
| **2656** | **Mean** | **0.0** | 1.7 | **1.6** | **2.4** | **4.5** | **3.8** | **3.0** | **5.9** | **9.1** | **9.1** | **10.6** | **12.5** | **13.0** | **13.2** | **14.1** | **15.2** | **15.9** | **13.6** | **13.7** | **15.2** | **16.0** |
| | SD | *0.0* | 2.6 | *3.2* | *3.5* | *2.4* | *1.9* | *3.0* | *4.8* | *3.1* | *3.0* | *2.8* | *2.2* | *2.3* | *2.8* | *3.4* | *3.4* | *2.7* | *1.6* | *0*.*9* | *1.8* | *2.2* |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2657** | Mean | 0.0 | 1.3 | 1.5 | 1.7 | 2.3 | 4.2 | 3.3 | 4.2 | 4.5 | 7.8 | 7.7 | 8.0 | 7.9 | 8.0 | 7.9 | 9.4 | 9.3 | 9.8 | 10.5 | 11.4 | 12.0 |
| | SD | *0.0* | *2.5* | *3.2* | *2.2* | *1.9* | *2.2* | *1.6* | *3.9* | 3.8 | *3.2* | *2.4* | *3.3* | *3.6* | *2.3* | *2.5* | *2.9* | *1.8* | *3.2* | *2.5* | *3.3* | *2.4* |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2659** | **Mean** | **0.0** | **-9.0** | **-17.7** | **-26.6** | **-28.1** | **-32.0** | **-37.2** | **-39.6** | | | | | | | | | | | | | |
| | SD | *0.0* | *1.0* | *1.8* | *2.8* | *4.7* | *4.0* | 3.8 | 2.5 | | | | | | | | | | | | | |
| | N | 5 | 5 | 5 | 5 | 5 | 5 | 4 | 2 | | | | | | | | | | | | | |
| **2660** | **Mean** | **0.0** | **-9.3** | **-17.4** | **-25.2** | **-28.3** | **-30.6** | **-36.4** | | | | | | | | | | | | | | |
| | SD | *0.0* | *1.9* | *2.6* | *2.9* | *5.7* | *5.3* | *7.3* | | | | | | | | | | | | | | |
| | N | 5 | 5 | 5 | 5 | 4 | 4 | 3 | | | | | | | | | | | | | | |
| **2668** | **Mean** | **0.0** | **-0.9** | **0.3** | **2.3** | **2.4** | **1.1** | **0.1** | **1.5** | **2.8** | **3.8** | **2.8** | **3.7** | **6.5** | **6.2** | **5.2** | **4.2** | **6.0** | **8.5** | **8.0** | **8.6** | **10.4** |
| | SD | *0.0* | *3.2* | *2.9* | *1.9* | 2.6 | *2.8* | *2.8* | *3.3* | *3.3* | *5.1* | *4.7* | *5.5* | *5.4* | *5.0* | *3.6* | *4.9* | *3.5* | 3.8 | *3.0* | *3.6* | *2.3* |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2669** | **Mean** | **0.0** | **-1.5** | **-0.8** | **-0.9** | **1.9** | **1.6** | **3.8** | **3.0** | **3.9** | **3.1** | **4.3** | **7.7** | **8.0** | **6.5** | **7.4** | **7.9** | **11.7** | **7.9** | **7.4** | **9.7** | **13.5** |
| | SD | *0.0* | *1.9* | *5.0* | *4.8* | *3.2* | *2.0* | *2.5* | *3.2* | *1.8* | *1*.*1* | *2.0* | *3.7* | *3.4* | *1.6* | *2.3* | *2.5* | *4.2* | *2.4* | *2.2* | *1.8* | 4.6 |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |
| **2670** | **Mean** | **0.0** | **-9.0** | **-17.7** | **-26.6** | **-28.1** | **-32.0** | **-37.2** | **-39.6** | | | | | | | | | | | | | |
| | SD | *0.0* | 1.0 | *1.8* | *2.8* | 4.7 | *4.0* | *3.8* | *2.5* | | | | | | | | | | | | | |
| | N | 5 | 5 | 5 | 5 | 5 | 5 | 4 | 2 | | | | | | | | | | | | | |
| **2671** | **Mean** | **0.0** | **-9.3** | **-17.4** | **-25.2** | **-28.3** | **-30.6** | **-36.4** | | | | | | | | | | | | | | |
| | SD | *0.0* | *1.9* | *2.6* | *2.9* | *5.7* | *5.3* | *7.3* | | | | | | | | | | | | | | |
| | N | 5 | 5 | 5 | 5 | 4 | 4 | 3 | | | | | | | | | | | | | | |
| **2672** | **Mean** | **0.0** | **-0.9** | **0.3** | **2.3** | **2.4** | **1.1** | **0.1** | **1.5** | 2.8 | 3.8 | 2.8 | 3.7 | 6.5 | 6.2 | 5.2 | 4.2 | 6.0 | 8.5 | 8.0 | 8.6 | 10.4 |
| | SD | *0.0* | *3.2* | *2.9* | *1.9* | *2.6* | *2.8* | *2.8* | *3.3* | *3.3* | *5.1* | *4.7* | *5.5* | *5.4* | *5.0* | *3.6* | *4.9* | *3.5* | 3.8 | *3.0* | *3.6* | *2.3* |
| | N | 5 | 5 | 5 | 5 | 5 | 5 | 5 | 5 | 5 | 5 | 5 | 5 | 5 | 5 | 5 | 5 | 5 | 5 | 5 | 5 | 5 |

### Example 5: kinase screening assays

Compound 2384 according to the disclosure was subjected to kinase screening assays (performed at Reaction Biology, Germany). The screening assay was performed at a final compound concentration of 10 µM. A radiometric protein kinase assay (33PanQinase^{®} ActivityAssay) was used for measuring the kinase activity of 340 protein kinases. All kinase assays were performed in 96-well FlashPlates^{™} from Perkin Elmer (Boston, MA, USA) in a 50 µL reaction volume. The reaction cocktail was pipetted in 4 steps in the following order: (1) 10 µL of non-radioactive ATP solution (in H₂O); (2) 25 µL of assay buffer/ [γ- 33P]-ATP mixture; (3) 5 µL of test sample in 10% DMSO and (4) 10 µL of enzyme/substrate mixture The assay for all protein kinases contained 70 mM HEPES-NaOH pH 7.5, 3 mM MgCl2, 3 mM MnCl2, 3 µM Na-orthovanadate, 1.2 mM DTT, 50 µg/ml PEG₂₀₀₀₀, ATP (variable amounts, corresponding to the apparent ATP-Km of the respective kinase), [γ- 33P]-ATP (approx. 8 × 10⁵ cpm per well), protein kinase, and substrate. All PKC assays (except the PKC-mu and the PKC-nu assay) additionally contained 1 mM CaCl2, 4 mM EDTA, 5 µg/ml Phosphatidylserine and 1 µg/ml 1,2-Dioleyl-glycerol. The CAMK1D, CAMK2A, CAMK2B, CAMK2D, CAMK2G, CAMK4, CAMKK1, CAMKK2, DAPK2, EEF2K, MYLK, MYLK2 and MYLK3 assays additionally contained 1 µg/ml Calmodulin and 0.5 mM CaCl2. The PRKG1 and PRKG2 assays additionally contained 1 µM cGMP. The DNA-PK assay additionally contained 2.5 µg/ml DNA. The protein kinase reaction cocktails were incubated at 30°C for 60 minutes. The reaction was stopped with 50 µl of 2 % (v/v) H₃PO₄, plates were aspirated and washed two times with 200 µL 0.9 % (w/v) NaCl. Incorporation of 33Pi (counting of "cpm") was determined with a microplate scintillation counter (Microbeta, Wallac). All protein kinase assays were performed with a BeckmanCoulter Biomek 2000/SL robotic system. All protein kinases provided by RBE were expressed in Sf9 insect cells or in E.coli as recombinant GST-fusion proteins or His-tagged proteins, either as full-length or enzymatically active fragments. All kinases were produced from human cDNAs and purified by either GSH-affinity chromatography or immobilized metal. Affinity tags were removed from a number of kinases during purification. The purity of the protein kinases was examined by SDS-PAGE/Coomassie staining, the identity was checked by mass spectroscopy. Kinases from external vendors were expressed, purified and quality-controlled. Staurosporine was used as positive control.

An IC₅₀ profiling (titration from 0.1 nM to 10 000 nM) on top 25 protein kinases was also performed on the 2384 compound, following the same procedure. Staurosporine was used as positive control.

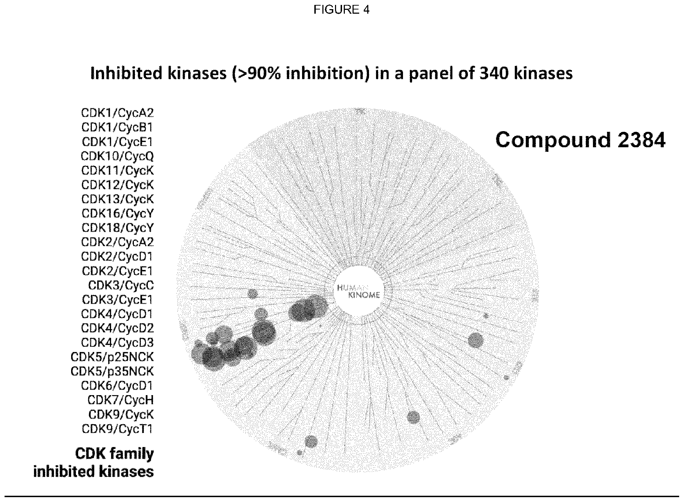

Figure 4 shows the result of the kinase screening assay of the 2384 compound according to the disclosure. Kinases inhibited at more than 90% are presented in the map as circles whose size is representative of percentage of inhibition. Inhibited CDKs at more than 90% are named in the lefthand side of the figure. Most inhibited kinases (more than 90 % inhibition) were found to belong to the CMGC kinase family, and particularly to the cyclin-dependent kinase (CDK) family, as pictured in the kinase map. Specifically, 23 out of the 30 most inhibited kinases were CDK/Cyclin complexes.

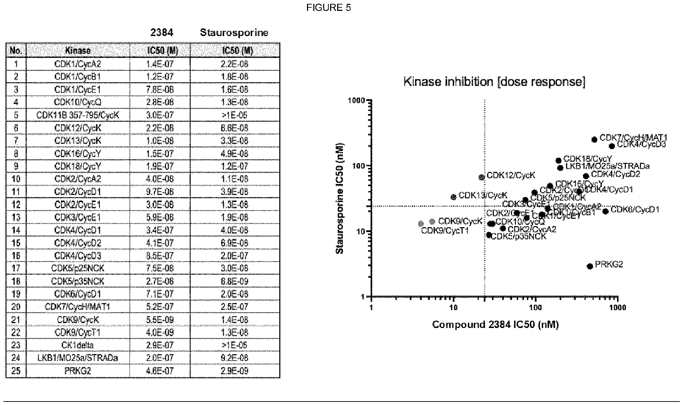

Figure 5 shows the dose-response inhibition on top 25 selected kinases. This allowed to identify CDK9/CycT1, CDK9/CycK, CDK13/CycK and CDK12/CycK as the main targets of compound 2384, with IC₅₀'s below 25 nM (bottom left quadrant).

### Example 6: in vitro cytotoxicity assays in presence of the NEDDylation inhibitor pevonedistat

In vitro cytotoxicity of compounds of the disclosure was assessed in breast cancer cell line BT-474, in presence or absence of the non-cytotoxic cullin NEDDylation inhibitor pevonedistat. NEDDylation is the process by which the ubiquitin-like protein NEDD8 drives degradation of the CycK protein by a process which is analogous to ubiquitination.

8000 cells were plated in 96-well plates and incubated at 37°C for 24 hours. Serial dilutions of the tested compound previously dissolved in culture media were added, and incubation was carried at 37°C out for 5 days in the presence or absence of 400nM of pevonedistat. MTT (5mg/mL in PBS, 20*µ*L, Sigma-Aldrich) was added into the wells, and incubation was continued for 1 to 4 hours at 37°C. Culture media was then carefully removed, and well content was homogeneously dissolved with acidified isopropanol. Absorbance values were measured on a microplate reader using a wavelength of 570 nm (with a reference wavelength of 690 nm). The IC₅₀ concentration values compared to untreated control cells were determined using inhibition dose response curve fitting (GraphPad Prism 9). Pevonedistat (cat#HY-70062) was purchased from MedChemExpress (China).

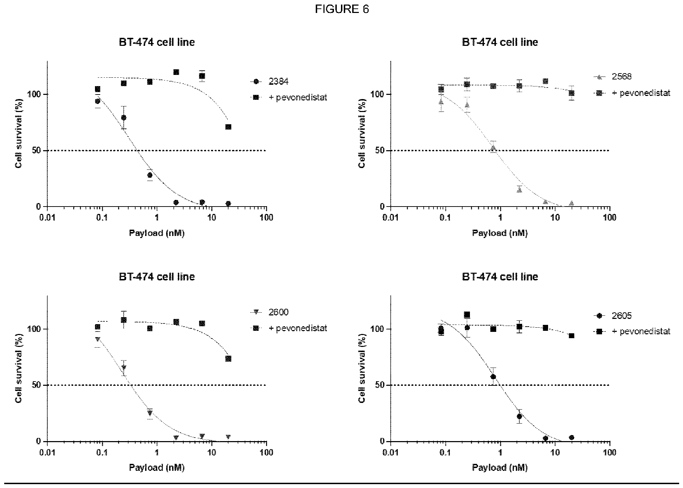

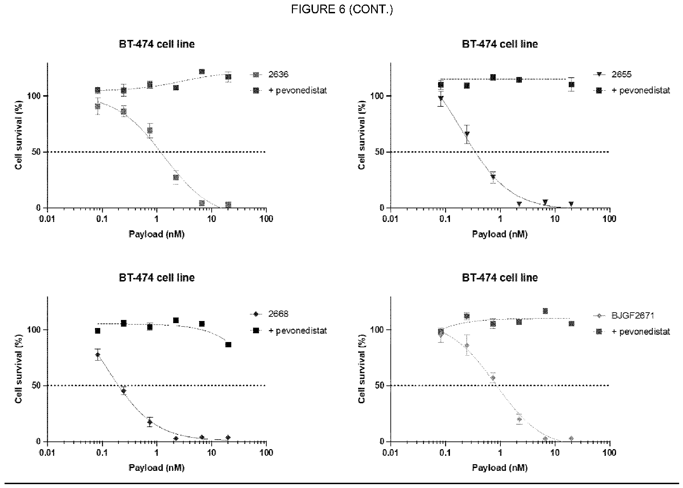

Figure 6 shows that the cell-killing property of compounds of the invention is strongly inhibited by pevonedistat, thereby confirming that main mechanism of action of cytotoxicity is molecular glue degradation of CycK.

### Example 7: in vitro mechanistic studies of compound 2384 of the invention

### Protein quantification by Western Blot

1×10⁶ JIMT-1 cells were plated in 3mL of DMEM/FBS 90:10 (v/v) in 6-well plates and incubated overnight. Cells were treated with the compound 2384 of the invention with or without the proteasome inhibitor MG-132 (MedChemExpress, Cat HY-13259) for 2h. Cells were harvested, and protein were extracted using complete RIPA buffer (RIPA buffer, 1 mM DTT, 1M NaF, 100 mM sodium orthovanadate, phosphatase inhibitor buffer and protease inhibitors) for 1 hour on ice. Lysate were centrifuged 15min at 12 000g at 4°C. and proteins were mixed with 4x Laemmli Sample Buffer (Bio-Rad), heated at 95 °C for 5 mins and separated by SDS PAGE (PROTEAN TGX Stain-Free Gels, Bio-Rad). Proteins were then transferred onto PVDF membrane (iBlot^{®}2 PVDF stacks, Invitrogen) using the iBlot2 device (Invitrogen) and blocked with TBS buffer (Intercept^{®} Blocking Buffer, LI-COR Biosciences). Primary antibodies were incubated overnight at 4°C and secondary antibodies (IRDye Infrared Dyes from LI-COR Biosciences) 1 hour at room temperature. Used primary antibody were: anti-CycK (cat#A301-939A, Bethyl Laboratories, dil. 1:1000) and anti-β-actin (cat#A5441, Sigma-Aldrich, dil. 1:5000). Membranes were scanned using a c500 Imaging System (Azure Biosystems) and densitometric quantification were performed using imaged software. Protein levels were normalized against β-actin.

### RT-qPCR

1×10⁶ JIMT-1 cells were plated in 3mL of DMEM/FBS 90:10 (v/v) in 6-well plates and incubated overnight. Cells were treated with the compound 2384 of the invention, with CR8 (a known molecular glue degrader that depletes CycK) or with the proteasome inhibitor MG-132 (MedChemExpress, Cat HY-13259) for 2h. RNA was extracted using the QIAamp RNeasy Mini Kit (cat#74106, Qiagen) according to the manufacturer's protocol. Reverse transcription was performed using SuperScripr^{™} IV reverse transcriptase (cat#LT02241, Invitrogen). Quantitative PCR was performed using a No ROX Sybr Master Mix in the LightCycler 480 Real-Time PCR system (Roche Life Science).

Cyclin K forward (CACTATGATACCCTGGCAACTGG) and reverse (CAGAAAGAGGCAACAGGCTCCT) primers were purchased from Eurogentec. Gene expression was normalized using ribosomal 28S as the housekeeping gene.

### Cell cycle arrest assessment

1×10⁶ JIMT-1 cells were plated in 3mL of DMEM/FBS 90:10 (v/v) in 6-well plates and incubated overnight. Cells were treated with the compound 2384 of the invention or with CR8 for 24h. Cells were then harvested and washed three times with PBS. Cells were suspended into 300µL of propidium iodide working solution (0.05mg/mL) and incubated for 45 minutes at 4°C in the dark. Analysis was performed using a BD Fortessa flow cytometer controlled by BD FACSDiva software (BD Biosciences) and data were analyzed using FlowJo software (BD Bioscience).

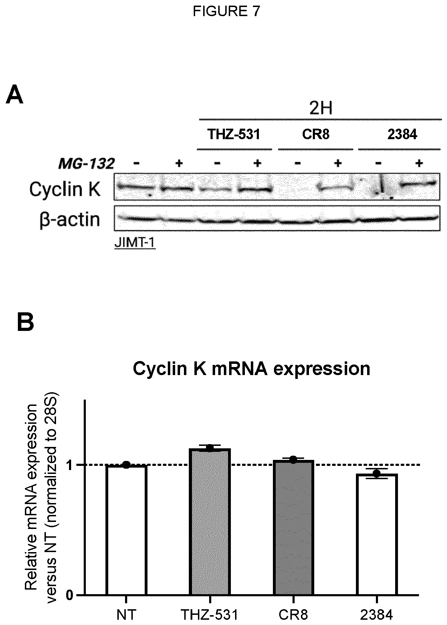

Figure 7A and 7B below shows that exposure of cells to compound 2384 of the invention or known comparator CycK molecular glue degrader (1µM final concentration) led to decreased CycK protein content (Figure 7A) but not at the CycK mRNA level (Figure 7B). THZ-531, a CDK12 inhibitor, had no impact on CycK protein content at this 1µM concentration (Figure 7A).

Combination with the proteasome inhibitor MG-132 restored the content in cycK protein (Figure 7A).

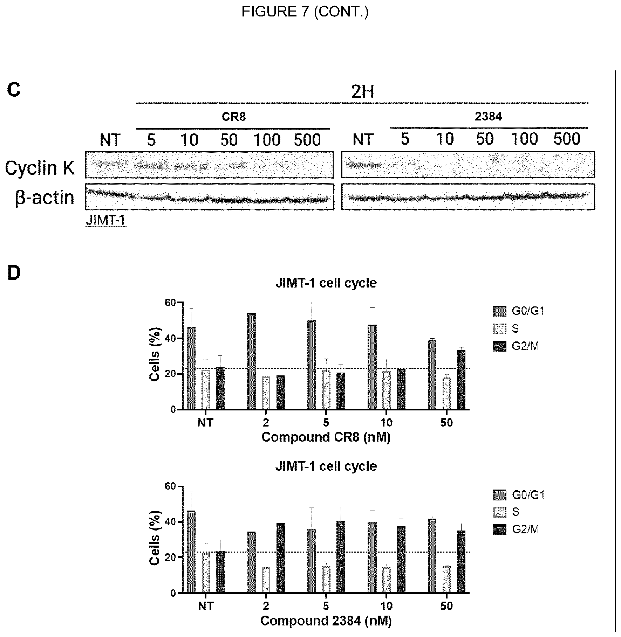

CycK degradation was also assessed using decreasing doses of either compounds 2384 of the invention or CR8 comparator (Figure 7C). 2h treatment of JIMT-1 cells with 10nM compound 2384 induced complete CycK degradation whereas no CycK was observed using CR8 at this dose. Complete CycK degradation by CR8 required concentrations between 100 and 500nM, showing that the 2384 minimum effective dose for CycK degradation is at approximately 10-times lower than that of CR8, thus explaining, its superior in vitro cytotoxicity.

Cell cycle analysis of cells using propidium iodide (PI) demonstrated an arrest in the G2/M phase upon compounds 2384 treatment for 24h at low and high doses (2, 5, 10, and 50nM), while CR8 treatment at 2, 5, and 10 nM had no impact on cell cycle (Figure 7D). Treatment with CR8 at 50 nM was required to induce an arrest in G2/M phase. This observation again contributes to explain the superior cytotoxicity of compound 2384 compared to that of CR8.

These examples above show that the compounds of the disclosure act as molecular glue degraders of CykK.

### Example 7: passive permeability assessment (PAMPA assay)

Parallel artificial membrane permeability assay (PAMPA) studies were conducted using the PAMPA Explorer kit (pION Inc., USA) and the double sink protocol (Avdeef et al., 2005). Stock solutions of compounds of the invention were prepared in dimethyl sulfoxide (DMSO) to a final concentration of 10 mM. Each stock solution was diluted to 50 µM in pH 7.4 Prisma HT buffer (pION) in the presence of 10% MeOH (v/v). 200 µL were added to each well of the donor plate in quintuplicate. The polyvinylidene fluoride (PVDF, 0.45 µM) filter membrane on the acceptor plate was coated with 5 µL of the gastrointestinal tract lipid formulation (GIT-0, pION) and to each well of the acceptor plate, 200 µL of acceptor sink buffer (ASB, pION), supplemented with 10% (v/v) MeOH, was added. The acceptor filter plate was carefully placed on top of the donor plate to form a "sandwich." The sandwich was incubated at 25°C for 5 h, at 300 rpm. UV-vis spectra of the solutions in the blank, reference, acceptor, and donor plates were measured using a Tecan Infinite 200 PRO M Nano Plus microplate reader. For each compound, apparent permeability values Pₐₚₚ (10⁻⁶ cm/s) were calculated using the PAMPA Explorer software v.3.6 (pION). Ketoprofen was used as negative impermeable control and verapamil was used as positive permeable control.

Table 3 below shows the apparent permeability values Pₐₚₚ (10⁻⁶ cm/s) of tested compounds of the invention. Compounds of the invention exhibited a continuum of passive permeability potential, ranging from none (compound 2585) to moderate (compound 2636) to high (compound 2671). A high apparent permeability values Pₐₚₚ is correlated to a higher passive permeation through the gastrointestinal tract, the blood-brain barrier, the skin and to a higher intratumoral diffusion of the drug (bystander effect).

**Table 3: apparent permeability values Pₐₚₚ (10⁻⁶ cm/s) of compounds of the invention**

| **Compound** | **Pₐₚₚ (10⁻⁶ cm/s)** | **Standard error** |
|---|---|---|
| **2384** | 84 | 5 |
| **2560** | 135 | 18 |
| **2568** | 88 | 16 |
| **2571** | 149 | 13 |
| **2585** | <0.3 | N/A |
| **2600** | 90 | 4 |
| **2605** | 112 | 6 |
| **2636** | 67 | 4 |
| **2655** | 125 | 5 |
| **2668** | 164 | 7 |
| **2670** | 132 | 6 |
| **2671** | 176 | 2 |
| **Verapamil** | 141 | 9 |
| **Ketoprofen** | 0.4 | 0.1 |

### Example 8 : MDR1/BCRP substrate

MDR1 and BCRP efflux substrate assays were conducted in MDCK-MDR1 and MDCK-BCRP in vitro models (Preadyport^{™}, Solvo Biotech) at Symeres (Nederland). Compound of the invention (donor well concentration of 5µM) were incubated in cell monolayer 24-transwell Preadyport system, using Preadyport recommended pH 7.4 assay buffer (donor and receiver wells). After 60 min of incubation (no shaking), samples are collected and suspended into triple volume of 66% cold acetonitrile. Samples are stored at -20°C until thawed, centrifuged and analyzed by LC-MS. Quinidine was used a positive control for the MDR1 assay, and prazosin was used as a positive control for the BCRP assay. Exatecan was used as a control, known not to be an MDR1 substrate. Residual DMSO content from compounds stock solutions was below 1% (v/v). The assay was performed in duplicate. The quantification of test compounds in donor or receptor compartment is conducted using relative LC/MS peak areas (samples of initial incubation solutions are used as a single point calibrator), without any spiked standard samples. The data is used for estimating apparent permeability (Pₐₚₚ) and calculation of efflux ratio.

Table 4 below shows that compounds of the invention exhibited different levels of MDR1 efflux pump susceptibility. MDR1 is protein of the tumor cell membrane that pumps the anticancer drugs out of cancer cells, thereby rendering the cell drug-resistant. The Pₐₚₚ (10⁻⁶ cm/s) B (acceptor = basolateral) to A (donor = apical) reflects the susceptibility of a compounds to be pumped out of a tumor and as such a low Pₐₚₚ B→A value is advantageous in the context of oncology. The efflux ratio B/A reflects the overall ability of a compounds to enter MDR1-expressing tumor cells (equilibrium between ability to passively enter the cell A→B and susceptibility to be pumped out of a tumor B→A).

Table 5 below shows that compounds of the invention exhibited different levels of BCRP efflux pump susceptibility. BCRP, like MDR1, is a protein of the tumor cell membrane that pumps the anticancer drugs out of cancer cells, thereby rendering the cell drug-resistant. This multidrug-resistant protein is particularly expressed in breast and lung cancers. The efflux ratio B/A reflects the overall ability of a compounds to enter BCRP-expressing tumor cells (equilibrium between ability to passively enter the cell A→B and susceptibility to be pumped out of a tumor B→A).

This example shows that compounds of the invention exhibited different levels of MDR1 and BCRP efflux potential. As an example, compounds 2655, 2600 and 2605 show low level of MDR1 and BCRP efflux potential and as such could be especially well suited to tackle multidrug-resistant tumor types (treat patients that have acquired resistance to first/second lines of chemotherapy).

**Table 4: apparent MDR1 permeability values Pₐₚₚ (10⁻⁶ cm/s) and efflux ratio of compounds of the invention**

| **Compound** | Pₐₚₚ **(10⁻⁶ cm/s) A (donor) to B (acceptor)** | **Pₐₚₚ (10⁻⁶ cm/s) B (acceptor) to A (donor)** | **Efflux ratio B/A** |
|---|---|---|---|
| **2655** | 5.13 | 26.9 | 5.25 |
| **Exatecan** | 2.28 | 16.7 | 7.31 |
| **2605** | 4.05 | 54.5 | 13.44 |
| **2600** | 2.05 | 59.3 | 28.86 |
| **2668** | 1.36 | 56.4 | 41.60 |
| **2671** | 0.99 | 43.2 | 43.68 |
| **2670** | < 0.50* | 23.5 | N/A |
| **2560** | < 0.70* | 26.6 | N/A |
| **2571** | 0.59 | 64.2 | 108.24 |
| **2636** | < 12.9* | 30.8 | N/A |
| **2585** | < 3.4* | 34.0 | N/A |
| **2384** | < 0.25* | 36.9 | N/A |
| **2568** | < 0.54* | 40.5 | N/A |

| | | | |
|---|---|---|---|
| *"based on LC-MS quantification method lower limit of quantification (LLOQ)* | | | |

**Table 5: apparent BCRP permeability values Pₐₚₚ (10⁻⁶ cm/s) and efflux ratio of compounds of the invention**

| **Compound** | **Pₐₚₚ (10⁻⁶ cm/s) A (donor) to B (acceptor)** | **Pₐₚₚ (10⁻⁶ cm/s) B (acceptor) to A (donor)** | **Efflux ratio B/A** |
|---|---|---|---|
| **2605** | 30.9 | 40.5 | 1.31 |
| **2600** | 22.4 | 47.1 | 2.10 |
| **2655** | 11.1 | 38.7 | 3.47 |
| **2671** | 10.1 | 46.0 | 4.54 |
| **2560** | 7.53 | 37.2 | 4.94 |
| **2670** | 5.02 | 29.8 | 5.93 |
| **2636** | 3.00 | 18.6 | 6.21 |
| **2668** | 10.2 | 71.0 | 6.99 |
| **2384** | 5.49 | 50.7 | 9.24 |
| **Exatecan** | 1.43 | 17.6 | 12.3 |
| **2571** | 7.09 | 91.4 | 12.9 |
| **2568** | 2.52 | 59.5 | 23.6 |
| **2585** | 1.75 | 49.0 | 27.9 |

## Claims

1. A compound of formula (I): 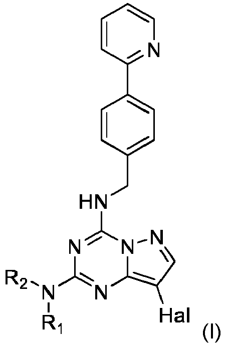 wherein
Hal is a halogen atom, preferably Br;
R1 is selected from the group consisting of H and C₁-C₆ alkyl, said alkyl being optionally substituted;
R2 is selected from the group consisting of C₁-C₂₀ alkyl, C₁-C₂₀ heteroalkyl, aryl having 6 to 10 ring atoms, C₃-C₈ cycloalkyl, heterocycloalkyl having 3 to 10 ring atoms, heteroaryl having 5 to 10 ring atoms, and any combination thereof, said alkyl, heteroalkyl, aryl, cycloalkyl, heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, and -NR'R"; R', and R" being independently selected from H and C₁-C₆ alkyl;
or, R1 and R2, together with the N atom to which they are bonded, form a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), -(C₃-C₈ cycloalkyl)-(C₁-C₁₂ heteroalkyl), (heterocycloalkyl having 3 to 10 ring atoms)-(C₁-C₁₂ heteroalkyl), (heteroaryl having 5 to 10 ring atoms)-(C₁-C₁₂ heteroalkyl), (aryl having 6 to 10 ring atoms)-(C₁-C₁₂ heteroalkyl), and -NR'R"; R', and R" being independently selected from H and C₁-C₆ alkyl;
and pharmaceutically acceptable salts and solvates thereof.

2. A compound of formula (I) according to claim 1, wherein R2 is selected from the group consisting of C₁-C₁₂ heteroalkyl, aryl having 6 to 10 ring atoms, C₃-C₈ cycloalkyl, heterocycloalkyl having 3 to 10 ring atoms, -(C₁-C₁₂ alkyl)-(C₃-C₈ cycloalkyl), -(C₁-C₁₂ alkyl)-(heterocycloalkyl having 3 to 10 ring atoms), said alkyl, heteroalkyl, aryl, cycloalkyl, and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and - NR'R"; R', and R" being independently selected from H and C₁-C₆ alkyl.

3. A compound of formula (I) according to claim 1, wherein R1 and R2, together with the N atom to which they are bonded, form a heterocycloalkyl having 3 to 10 ring atoms and comprising at least 2 N atoms, said heterocycloalkyl being optionally substituted, by one or more substituent preferably selected from C₁-C₁₂ heteroalkyl, heterocyclyl having 5 to 10 ring atoms, and -NR'R"; R', and R" being independently selected from H and C₁-C₆ alkyl.

4. A compound of formula (I) according to any of claim 1 to 2, wherein
Hal is Br,
R1 is H,
R2 is a C₃-C₈ cycloalkyl substituted by -NR'R"; R', and R" being independently selected from H and C₁-C₆ alkyl.

5. A compound of formula (I) according to claim 1, wherein the compound is of formula (II): 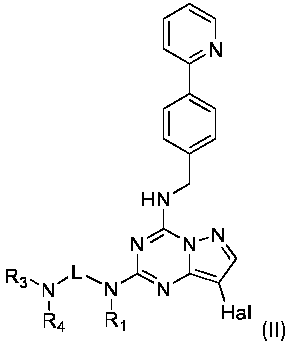 wherein
L is a linker selected from a bond, C₁-C₂₀ alkylene, C₁-C₂₀ heteroalkylene, arylene having 6 to 10 ring atoms, C₃-C₈ cycloalkylene, heterocycloalkylene having 3 to 10 ring atoms, heteroarylene having 5 to 10 ring atoms, and any combination thereof, said alkylene, heteroalkylene, arylene, cycloalkylene, heteroarylene and heterocycloalkylene being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl,
R3 is selected from the group consisting of H and C₁-C₆ alkyl, and
R4 is selected from the group consisting of H and C₁-C₆ alkyl.

6. A compound of formula (I) according to claim 1, wherein the compound is of formula (III) 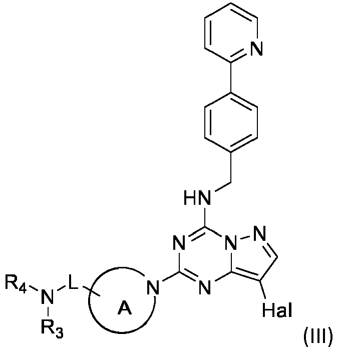 wherein
Ring A is a heterocycloalkyl having 3 to 10 ring atoms, or a heteroaryl having 5 to 10 ring atoms, said heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl;
L is a linker selected from a bond, C₁-C₁₂ alkylene, C₁-C₁₂ heteroalkylene, arylene having 6 to 10 ring atoms, C₃-C₈ cycloalkylene, heterocycloalkylene having 3 to 10 ring atoms, heteroarylene having 5 to 10 ring atoms, and any combination thereof, said alkylene, heteroalkylene, arylene, cycloalkylene, heteroarylene and heterocycloalkylene being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl;
R3 is selected from the group consisting of H and C₁-C₆ alkyl, and
R4 is selected from the group consisting of H and C₁-C₆ alkyl.

7. A compound of formula (I) according to claim 1, wherein the compound is of formula (IV) 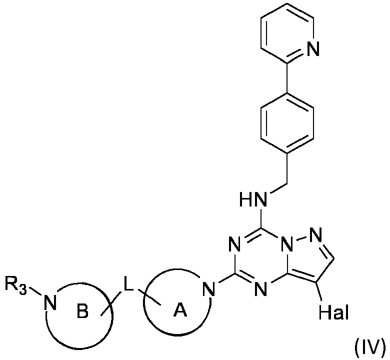 wherein
Ring A is a heterocycloalkyl having 3 to 10 ring atoms, or a heteroaryl having 5 to 10 ring atoms, said heteroaryl and heterocycloalkyl being optionally substituted, preferably by one or more substituent selected from the group consisting of -OH, =O, C₁-C₁₂ alkyl, C₁₋ C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl
Ring B is a heterocycloalkyl having 3 to 10 ring atoms, or a heteroaryl having 5 to 10 ring atoms, said heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl
L is a linker selected from a bond, C₁-C₁₂ alkylene, C₁-C₁₂ heteroalkylene, arylene having 6 to 10 ring atoms, C₃-C₈ cycloalkylene, heterocycloalkylene having 3 to 10 ring atoms, heteroarylene having 5 to 10 ring atoms, and any combination thereof, said alkylene, heteroalkylene, arylene, cycloalkylene, heteroarylene and heterocycloalkylene being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl; and
R3 is selected from the group consisting of H and C₁-C₆ alkyl.

8. A compound of formula (I) according to claim 1, wherein the compound is of formula (V) 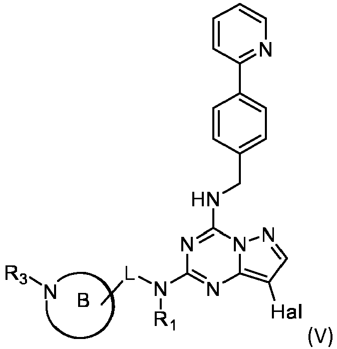 wherein
Ring B is a heterocycloalkyl having 3 to 10 ring atoms, or a heteroaryl having 5 to 10 ring atoms, said heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl;
L is a linker selected from a bond, C₁-C₁₂ alkylene, C₁-C₁₂ heteroalkylene, arylene having 6 to 10 ring atoms, C₃-C₈ cycloalkylene, heterocycloalkylene having 3 to 10 ring atoms, heteroarylene having 5 to 10 ring atoms, and any combination thereof, said alkylene, heteroalkylene, arylene, cycloalkylene, heteroarylene and heterocycloalkylene being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl; and
R3 is selected from the group consisting of H and C₁-C₆ alkyl.

9. Compound according to claim 1, wherein the compound is selected from

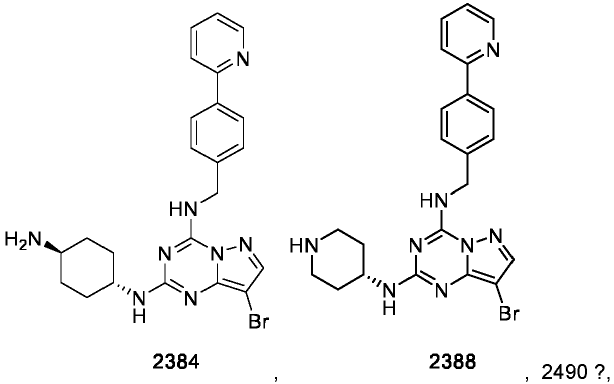

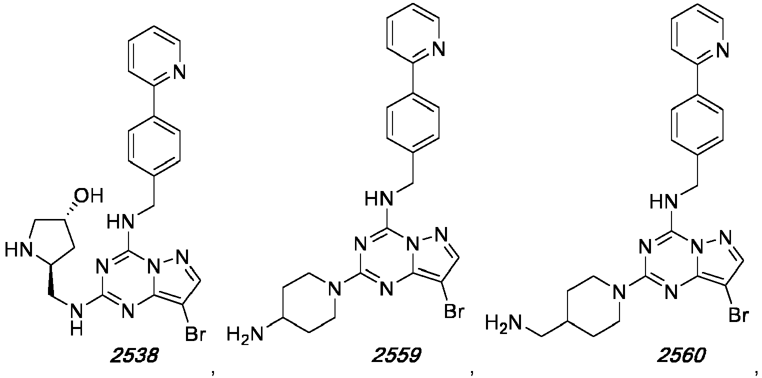

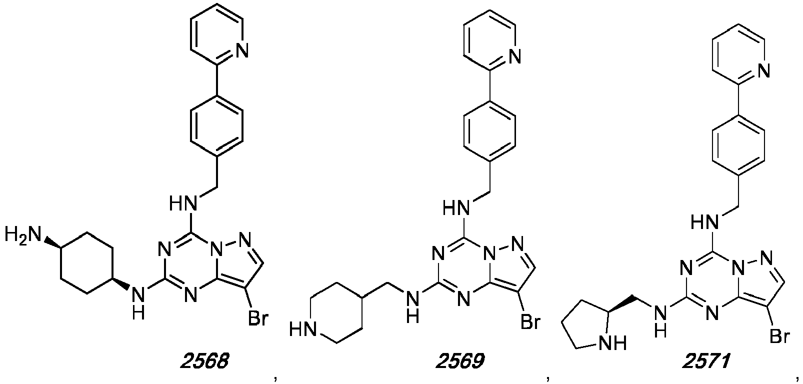

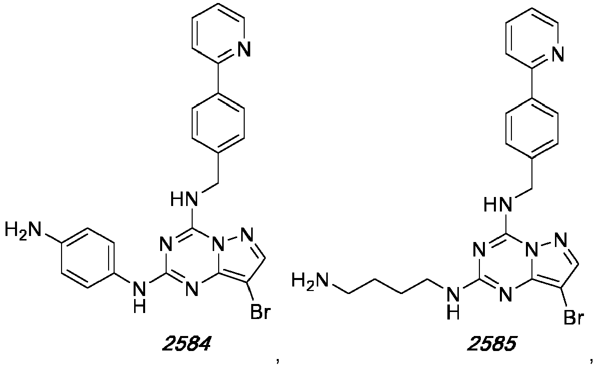

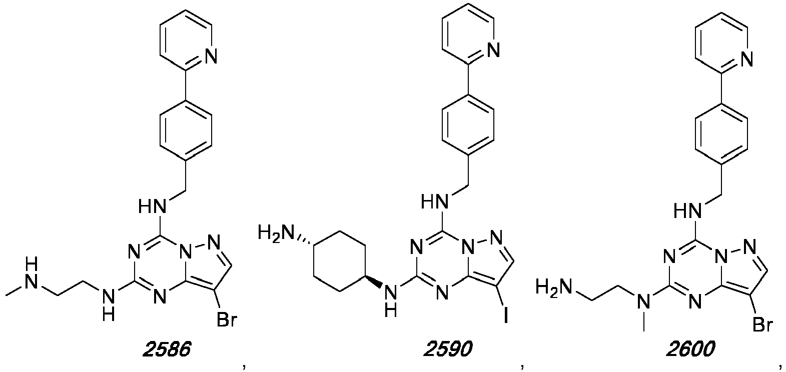

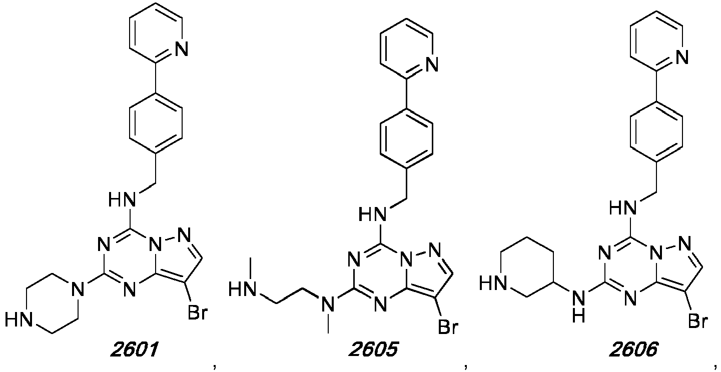

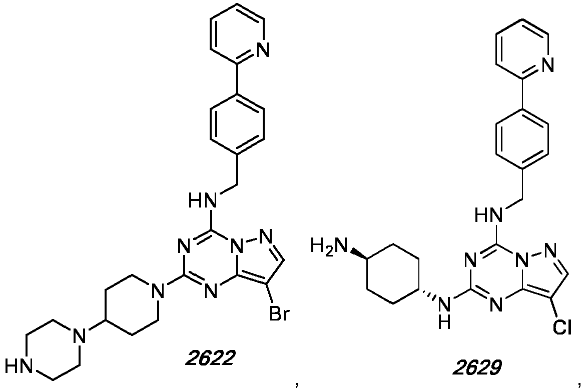

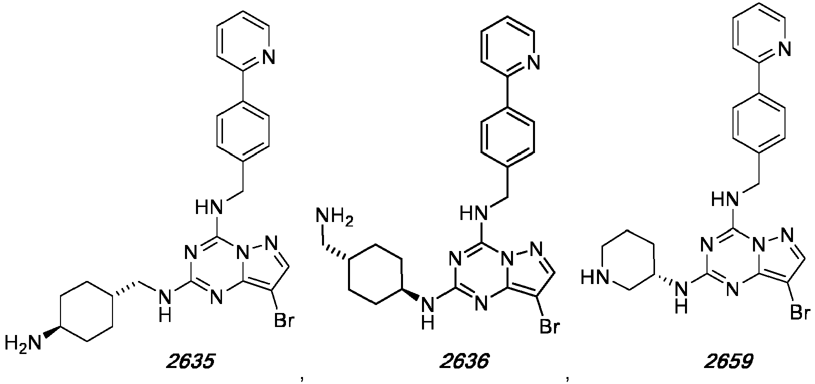

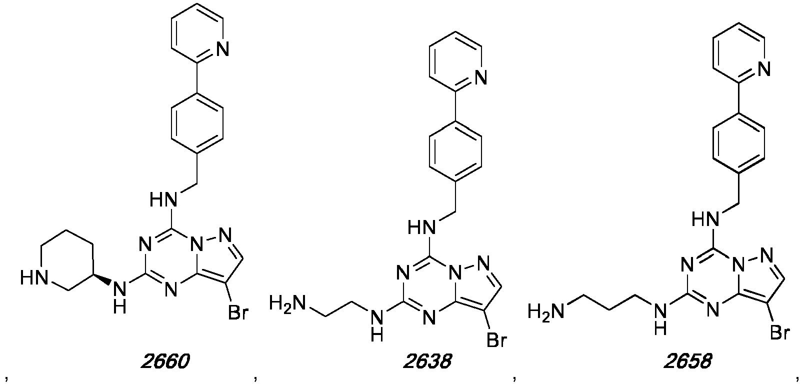

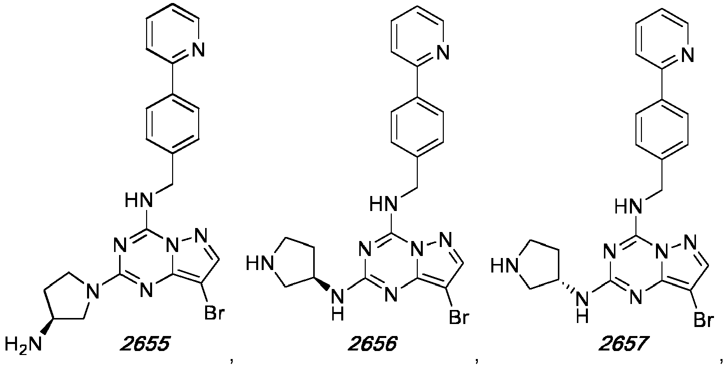

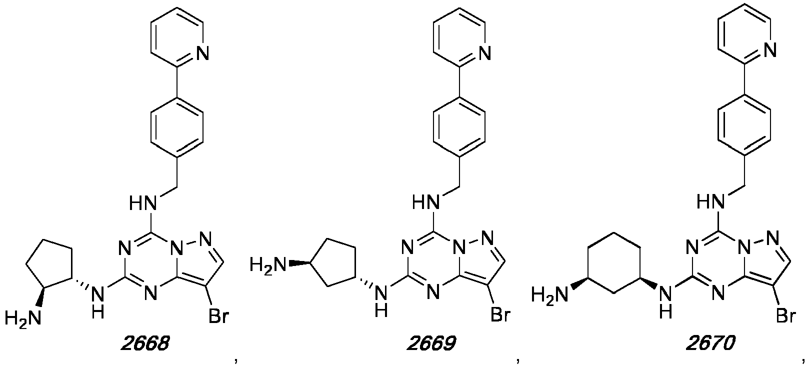

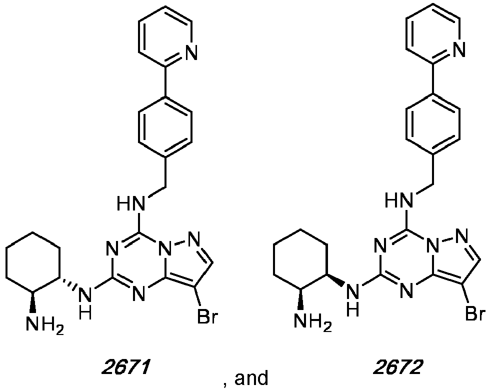

10. Pharmaceutical composition comprising a compound according to any of claims 1 to 9 and a pharmaceutically acceptable carrier.

11. Compound according to any of claims 1 to 9, or the pharmaceutical composition according to claim 10, for use as a drug.

12. Compound according to any of claims 1 to 9, or the pharmaceutical composition according to claim 10, for use in the treatment of cancer or an inflammatory disease.
